# EUROPEAN PATENT SPECIFICATION

(11) **EP 1 147 181 B1**
(45) Date of publication and mention of the grant of the patent: **12.05.2004**
(21) Application number: 00917613.2
(22) Date of filing: 04.02.2000
(51) Int. Cl.: C12N 7/01, C12N 15/54, C12N 9/12, C12Q 1/70, C12N 5/10, C12N 15/85, A61K 35/76, A61P 35/00, A61K 31/7088

(54) **REPLICATIVE VIRUS DRIVEN BY THE PROMOTER FOR TELOMERASE REVERSE TRANSCRIPTASE FOR USE IN TREATING CANCER**
REPLIKATIVER VIRUS GESTEUERT VON DEM PROMOTOR DER REVERSEN TELOMERASE TRANSKRIPTASE ZUR BEHANDLUNG VON KREBS
VIRUS REPLICATIF CONTROLE PAR LE PROMOTEUR DE LA TRANSCIPTASE INVERSE TELOMEREUSE POUR LE TRAITEMENT DU CANCER

(30) Priority: 04.02.1999 US 244438
(43) Date of publication of application: 24.10.2001
(73) Proprietor: Geron Corporation, Menlo Park, CA 94025 (US)
(72) Inventor: MORIN, Gregg, B., Davis - 95616 / CA (US); LICHTSTEINER, Serge, Encinitas - 92024/CA (US); VASSEROT, Alain, Berkeley - 94708 - CA (US); ADAMS, Robert, Redwood City - 94062 / CA (US); CARDOZA, Lisa, M., San Mateo - 94401/CA (US); LEBKOWSKI, Jane, S., Portola Valley - 94028 / CA (US)
(74) Representative: Bassil, Nicholas Charles
(86) International application number: PCT/US2000/003104
(87) International publication number: WO 2000/046355

(56) References cited:
- WO-A-99/33998
- GB-A- 2 317 891
- US-A- 5 728 379
- TAKAKURA ET AL: "Cloning of human telomerase catalytic subunit (hTERT) gene promoter and identification of proximal core promoter sequences essential for transcriptional activation in immortalized and cancer cells" CANCER RESEARCH,US,AMERICAN ASSOCIATION FOR CANCER RESEARCH, BALTIMORE, MD, vol. 59, no. 3, 1 February 1999 (1999-02-01), pages 551-557, XP002105209 ISSN: 0008-5472
- CONG ET AL: "The human telomerase catalytic subunit hTERT: organization of the gene and characterization of the promoter" HUMAN MOLECULAR GENETICS,GB,OXFORD UNIVERSITY PRESS, SURREY, vol. 8, no. 1, 1999, pages 137-142, XP002105208 ISSN: 0964-6906

## Description

### PRIORITY CLAIM

This application claims the priority basis of U.S. Patent Application 09/244,438.

### FIELD OF THE INVENTION

The invention is related generally to the fields of genetic regulatory elements that control protein transcription in eukariotic cells, and recombinant viral constructs useful for the treatment of disease, including cancer. More specifically, the invention describes promoters based on regulatory elements for telomerase reverse transcriptase, transcriptional control sequences, and the use of these features in the design of oncolytic viruses.

### BACKGROUND OF THE INVENTION

It has long been recognized that complete replication of the ends of eukaryotic chromosomes requires specialized cell components (Watson (1972) Nature New Biol. 239:197; Olovnikov (1973) J. Theor. Biol. 41:181). Replication of a linear DNA strand by conventional DNA polymerases requires an RNA primer, and can proceed only 5' to 3'. When the RNA primer bound at the extreme 5' ends of eukaryotic chromosomal DNA strands is removed, a gap is introduced, leading to a progressive shortening of daughter strands with each round of replication. This shortening of telomeres, the protein-DNA structures physically located on the ends of chromosomes, is thought to account for the phenomenon of cellular senescence or aging of normal human somatic cells in vitro and in vivo (Goldstein (1990) Science 249:1129; Martin (1979) Lab. Invest. 23:86; Goldstein (1969) Proc. Natl. Acad. Sci. USA 64:155; Schneider (1976) Proc. Natl. Acad. Sci. USA, 73:3584; Harley (1990) Nature 345:458-460; Hastie (1990) Nature 346:866-868; Counter (1992) EMBOJ. 11:1921-1929; Bodnar (1998) Science 279:349-52).

The length and integrity of telomeres is thus related to entry of a cell into a senescent stage. Moreover, the ability of a cell to maintain (or increase) telomere length may allow a cell to escape senescence.

The maintenance of telomeres is a function of a specific DNA polymerase known as telomerase reverse transcriptase (TERT). Telomerase is a ribonucleoprotein (RNP) that uses a portion of its RNA moiety as a template for telomere repeat DNA synthesis (Morin (1997) Eur. J. Cancer 33:750). Consistent with the relationship of telomeres and TERT to the proliferative capacity of a cell, telomerase activity can be detected in highly replicative cell types such as stem cells. It is also active in an extraordinarily diverse set of tumor tissues, but is active in normal somatic cell cultures or normal tissues adjacent to a tumor (U.S. Patent Nos. 5,629,154; 5,489,508; 5,648,215; and 5,639,613; Morin (1989) Cell 59:521; Shay (1997) Eur. J. Cancer 33:787; Kim (1994) Science 266:2011). Moreover, a correlation between the level of telomerase activity in a tumor and the likely clinical outcome of the patient has been reported (U.S. Patent No. 5,639,613; Langford (1997) Hum. Pathol. 28:416).

Telomerase activity has also been detected in human germ cells, proliferating stem or progenitor cells, and activated lymphocytes. In somatic stem or progenitor cells, and in activated lymphocytes, telomerase activity is typically either very low or only transiently expressed (Chiu (1996) Stem Cells 14:239; Bodnar (1996) Exp. Cell Res. 228:58; Taylor (1996) J. Invest. Dermatol. 106:759).

United Kingdom patents GB 2317891 B and GB 2321642 B, and Intemational Patent Publication WO 98/14593 (Geron Corporation) disclose the gene sequence for the human telomerase catalytic subunit (hTERT). Takakura et al. (Cancer Res. 59:551, 1999) report the cloning of the hTERT gene promoter and identification of proximal core promoter sequencers essential for transcriptioanl activation in immortalized and cancer cells. International Patent Publication WO 99/33998 (Bayer AG), filed after the priority date of this appliation, report regulatory DNA sequences of the human catalytic telomerase sub-unit gene, and its use for diagnosis and therapy. Cong et al. (Hu. MOlec. Genetics 8:137, 1999), also filed after the priority date of this applicatoin, report the organization of the hTERT gene and characterization of the promoter. U.S. Patent 5,728,379 (Georgetown University) report the tumor- or cell-specific replication of herpes simplex virus.

The preceding summary is intended to introduce the field of the present invention to the reader. The cited references in this application are not to be construed as admitted prior art.

### SUMMARY OF THE INVENTION

This disclosure explains that telomerase reverse transcriptase (TERT) is an ideal target for treating human diseases relating to cellular proliferation and senescence, such as cancer. The cis-acting transcriptional control elements of the this invention enable identification of trans-acting transcription control factors. The discovery and characterization of a promoter specific for TERT expressing cells has provided an opportunity to develop important new disease therapies.

One embodiment of the invention is an oncolytic virus having a genome in which a telomerase reverse transcriplase (TERT) promoter is operably linked to a genetic element essential for replication or assembly of the virus. The promoter drives transcription of the genetic element in cells expressing telomerase reverse transcriptase (TERT), thereby causing replication of the virus. Replication of the virus in a cell in turn leads to lysis of the cell.

Another embodiment is a polynucleotide component suitable for assembling an oncolytic virus of this invention, in which a promoter for TERT is operatively linked to a genetic element essential for replication or assembly of a virus.

Exemplary replication-competent viruses of this invention include replication-conditional adenovirus and herpesvirus. Exemplary genetic elements linked to the promoter are adenovirus E4, E1a, E1b or E2 genes and herpes simplex virus ICP6 or ICP4 genes. Exemplary TERT promoter sequences are contained in phage λGϕ5, deposited with the ATCC under Accession No. 98505. The sequence may contain about 100 or 500 consecutive nucleotides of SEQ. ID NO:1 or 2, o a sequence that hybridizes to the complement thereof and having the characteristic of preferentially promoting transcription in cells expressing TERT. Exemplary portions of the TERT gene sequence are described later in the disclosure. Optionally, the oncolytic virus may also contain an encoding region whose expression is toxic to the cell, or which renders the cell more susceptible to toxic effects of a drug for example, the gene may encode thymidine kinase, rendering the cell susceptible to the drug is ganciclovir.

Another embodiment is a method for selecting a virus having characteristics of an oncolytic virus of the invention. A virus construct is provided in which a TERT promoter is operably linked to a genetic element required for replication or assembly of the virus, which is used to infect a cell expressing TERT and a cell not expressing TERT. A virus is then selected if it preferentially kills the cell expressing TERT. Another embodiment is a method for producing an oncolytic virus of the invention, comprising operably linking a TERT promoter sequence to a genetic element to form a linked gene; transfecting the linked gene into a telomerase expressing cell; and then propagating virus obtained from the cell.

Another embodiment of the invention is a method for killing a cell expressing telomerase reverse transcriptase, comprising contacting the cell with the oncolytic virus of the invention. The cell may be a cancer cell. Another embodiment is a medicament comprising the oncolytic virus of the invention for use in medicine, or for treatment of cancer. Illustrative malignancies include lung cancer, pancreatic cancer, medulloblastoma, cervical carcinoma, and fibrosarcoma.

A further understanding of the nature and advantages of the invention will be appreciated from the disclosure that follows.

### BRIEF DESCRIPTION OF THE DRAWINGS

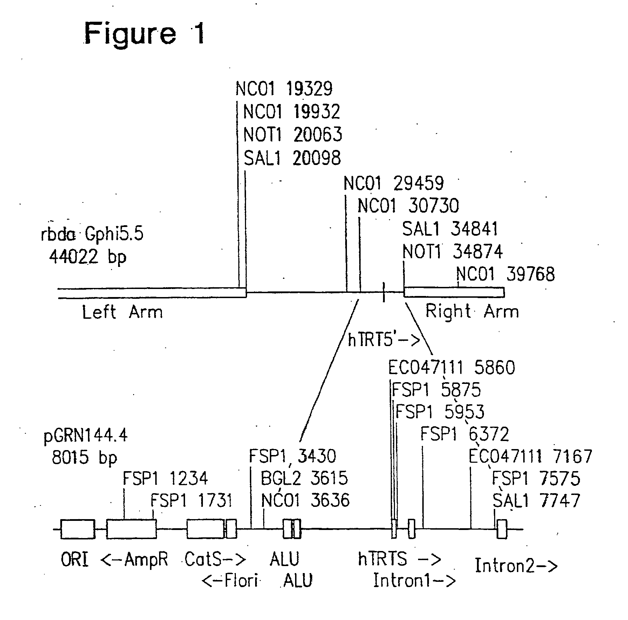

**Figure 1** is a restriction map of lambda phage clone λGϕ5, used for obtaining the sequence about 15 kbases upstream from the translation initiation site. This region includes the hTERT promoter.

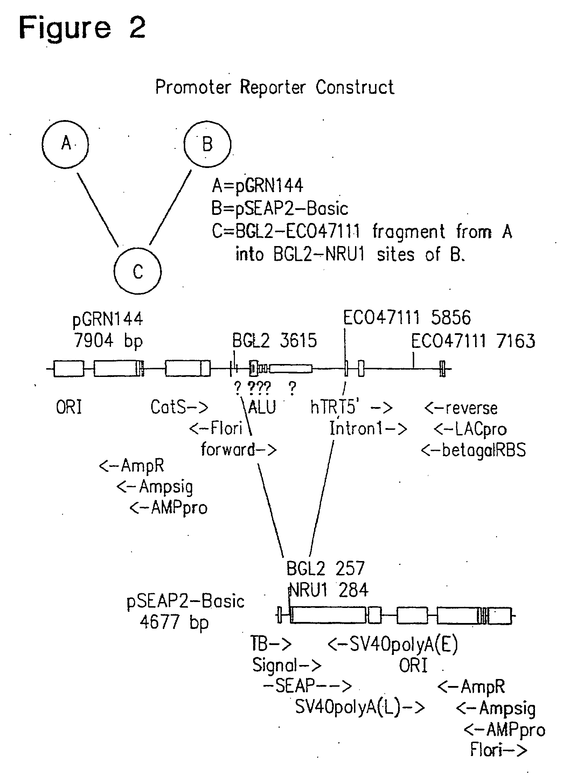

**Figure 2** is a map showing features of an hTERT promoter-reporter plasmid, Reporter plasmids have been used to demonstrate that the promoter specifically promotes transcription in cells expressing TERT, including cancer cells.

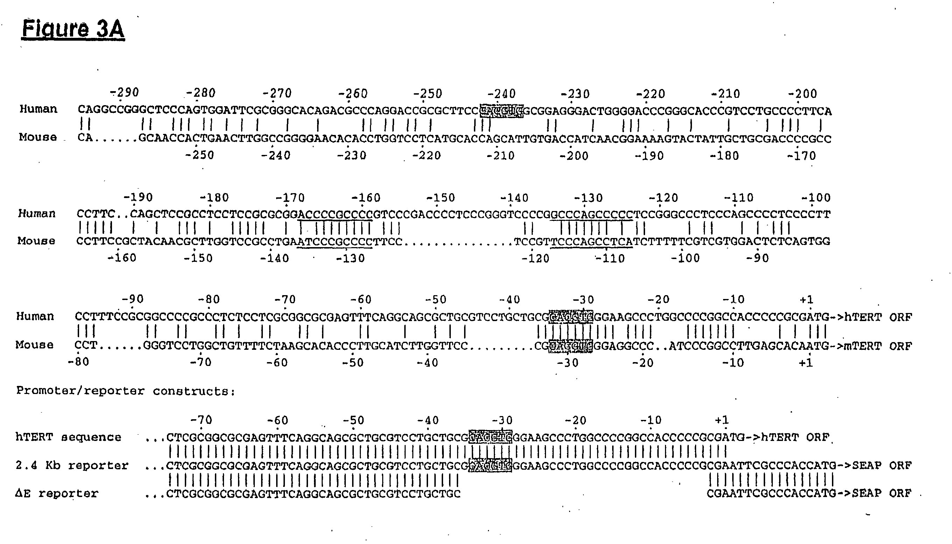

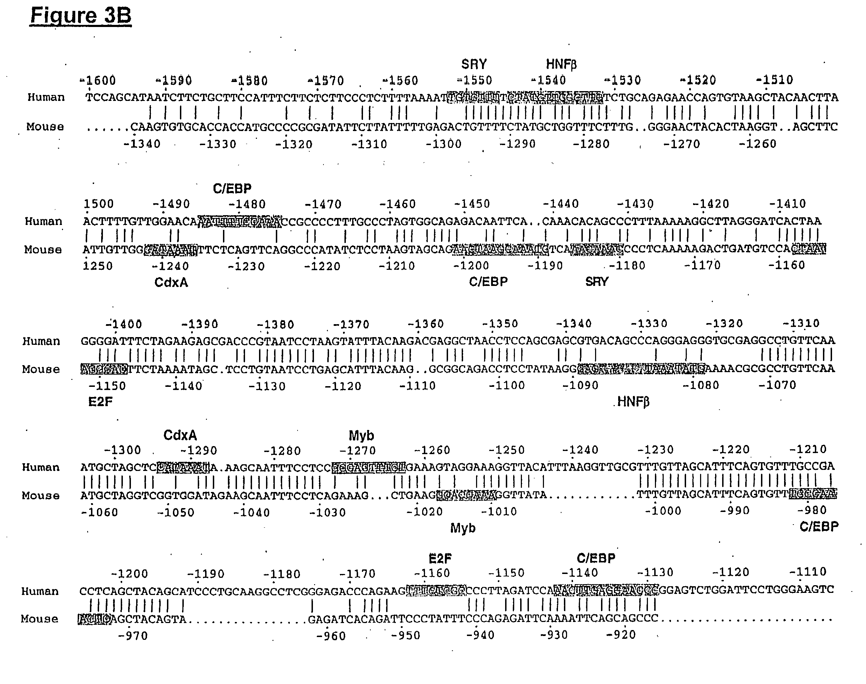

**Figure 3** is a sequence alignment, comparing regions of the hTERT promoter (SEQ. ID NO:1) with that of mTERT (SEQ. ID NO:2). Regions of homology were used to identify regulatory elements. **Figure 3(A)** shows the position of conserved cis-acting transcriptional regulatory motifs, including the E-box (the Myc/Max binding site, indicated by shading) and the SP1 sites (underlined). The lower panel illustrates the proximal sequences of the 2.5 kb hTERT and E-box reporter constructs, including the region deleted in the E-box reporter construct, as described in Example 8. Figure 3(B) shows the identification of other regulatory elements. The numbering shown is calculated from the translation initiation site.

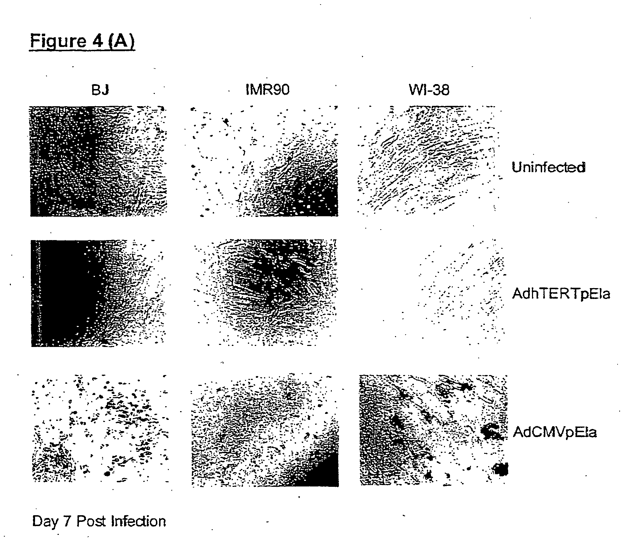

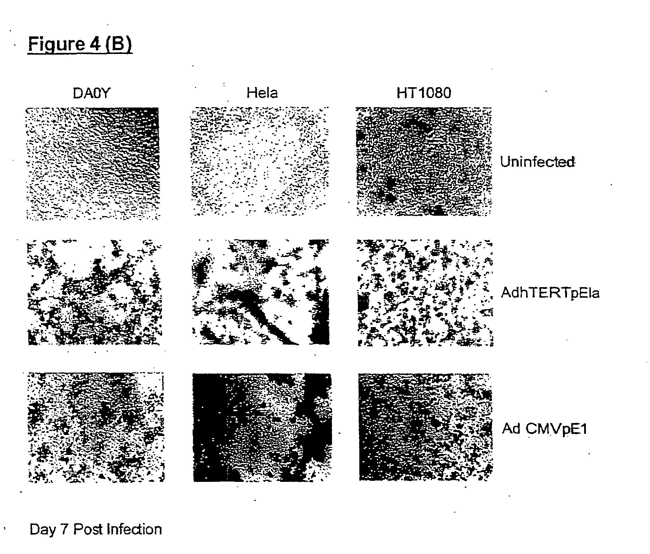

**Figure 4** is a half tone reproduction of cell lines photographed 7 days after infection with oncolytic virus. Top row: uninfected cells (negative control). Middle row: cells infected with oncolytic adenovirus, in which replication gene E1a is operably linked to the hTERT promoter. Bottom row: cells infected with adenovirus in which E1a is operably linked to the CMV promoter (positive control).

The cells tested were as follows: Figure 4(A): BJ (foreskin fibroblast); IMR-90 (lung fibroblast); WI-38 (lung fibroblast); cells of non-malignant origin. Figure 4(B): A549 (lung carcinoma) AsPC-1 and BxPC-3: (adenocarcinoma, pancreas). Figure 4(C): DAOY (medulloblastoma); HeLa (cervical carcinoma); HT1080 (fibrosarcoma). The results show that the hTERT-regulated oncolytic virus specifically lyses cancer cells, in preference to cell lines that don't express telomerase reverse transcriptase at a substantial level. This is in contrast to oncolytic virus regulated by a constitutive promoter like CMV promoter, which lyses cells non-specifically.

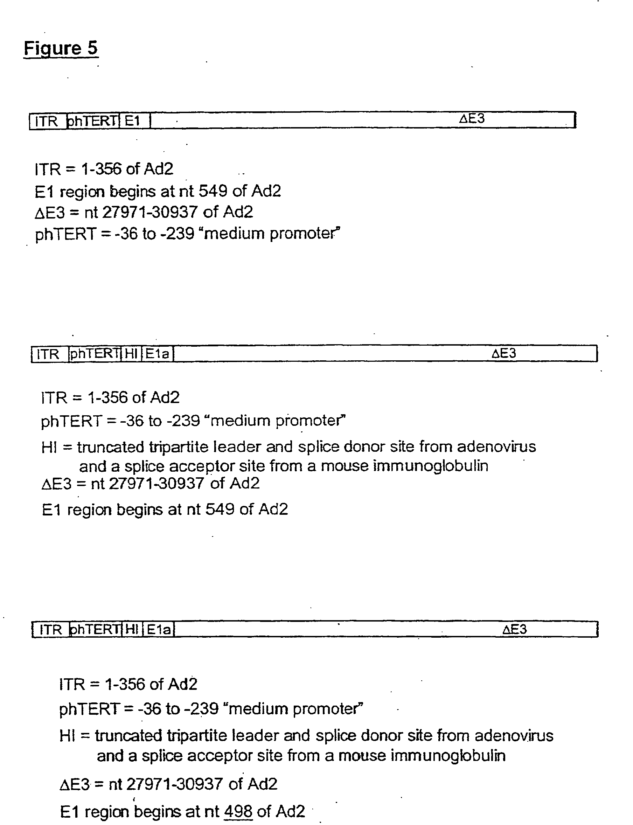

**Figure 5** is a series of maps showing construction of oncolytic adenovirus, made conditionally replicative by placing the E1a replication under control of an hTERT promoter. The first construct comprises the Inverted Terminal Repeat (ITR) from the adenovirus (Ad2); followed by the hTERT medium-length promoter (pGRN176) operably linked to the adenovirus E1a region; followed by the rest of the adenovirus deleted for the E3 region (ΔE3). This construct was used in the virus infection experiments shown in Figure 4. The second conditionally replicative adenovirus construct shown in the Figure comprises an additional sequence in between the hTERT promoter and the E1a region. The HI sequence is an artificial intron engineered from adenovirus and immunoglobulin intron splice sequences. The third adenovirus construct is similar, except that the E1a region used is longer at the 5' end by 51 nucleotides.

### DETAILED DESCRIPTION

The invention provides novel isolated polynucleotides comprising cis-acting transcriptional control sequences of telomerase reverse transcriptase genes. The polynucleotides of the invention include those based on or derived from genomic sequences of untranscribed, transcribed and intron regions of TERT genes, including the human and mouse homolog. Cis-acting TERT transcriptional control sequences include those that regulate and modulate timing and rates of transcription of the TERT gene. The TERT promoter sequences of the invention include cis-acting elements such as promoters, enhancers, repressors, and polynucleotide sequences that can bind factors that influence transcription.

### Isolating and characterizing human TERT promoter sequences

As described in Example 1, the hTERT promoter (SEQ ID NO:1) was obtained by sequencing an insert from a lambda phage isolated from a human genomic library. This lambda clone is designated λGϕ5 and has been deposited at the ATCC, under Accession No. 98505. Lambda Gθ5 contains a 15.3 kilobase pair (kbp) insert including approximately 13,500 bases upstream from the hTERT coding sequence. These hTERT promoter sequences were further subcloned into plasmids. A Not1 fragment (SEQ ID NO:1) from λGϕ5 containing the hTERT promoter sequences was subcloned in opposite orientations into the NotI site of pUC derived plasmids (designated pGRN142 and pGRN143, respectively, and pGRN142 was sequenced.

In SEQ ID NO:1, the hTERT genomic insert begins at residue 44 and ends at residue 15375. The start of the cDNA from which it was derived begins at residue 13490. The hTERT ATG translation initiation codon starts at residue 13545. Untranscribed hTERT promoter sequences lie downstream of residue 44 and upstream of the encoding region, and may also reside in the first Intron. In immortal cells, a reporter gene driven by a sequence upstream of the TERT coding sequence drove expression as efficiently as the positive control (containing an SV40 early promoter and enhancer). Certain TERT promoter sequences of the invention also include intron sequences.

### Identification of cis-acting transcriptional regulatory sequences in the human and mouse TERT promoter

To identify cis-acting transcriptional regulatory sequences in human TERT and mouse TERT sequences 5' to their respective TERT coding sequence, the human and mouse promoter sequences were analyzed for sequence identity. Alignment of the first 300 bases upstream of the human and mouse coding sequences indicated a number of conserved regions, and putative cis-acting transcriptional regulatory sequences were identified (Figure 3(A)).

In particular, located at residues -34 to -29 upstream of the human TERT translation start site (ATG, A at 13545 of SEQ ID NO: 1) and at residues -32 to -27 upstream of the mouse TERT translation start site (ATG) are highly conserved motifs. They correspond to a cis-acting motif known to interact with c-Myc, the so-called "E-box" or "Myc/Max binding site." Specifically, they are highly conserved with respect to the core nucleotides that comprise the E-box, nucleotides flanking the E-box and position of the E-box relative to the translation start site. A second E-box was identified at residues -242 to -237 upstream of the human TERT translation start site. This second E-box was not conserved in the mouse promoter. These observations support the finding that the conserved Myc binding site, by interacting with c-Myc as a trans-acting transcriptional regulatory factor, plays a major role in TERT promoter regulation and telomerase expression.

Sequence alignment identified additional conserved cis-acting transcriptional regulatory elements in the TERT gene promoter. For example, two SP1 binding sites, located at residue -168 to -159 and residue - 133 to -121 relative to the TERT translation start site were identified, which are highly conserved between the mouse and human TERT promoters. Binding sites (cis-acting sequences) for a number of other transcription factors, including the sex determining region Y gene product (SRY), hepatic nuclear factors 3-β and 5, TFIID-MBP, E2F and c-Myb were also found within this region of both the mouse and human promoters.

Further analysis of the human and mouse TERT promoter sequences indicated other regions of sequence conservation. In particular, a region with a high degree of sequence identity between human and mouse promoter was found between residue -1106 and residue -1602 upstream of the human TERT translation start site and residue -916 and residue -1340 upstream of the mouse TERT translation start site (Figure 3(B)). Thus, the invention provides cis-acting sequences specific for the modulation of TERT transcription. In a preferred embodiment, the methods of the invention use these human and mouse TERT-specific transcriptional regulatory motifs to identify and isolate TERT-specific, and other, trans-acting transcriptional regulatory factors.

The invention also provides the reagents and methods for screening and isolating trans-acting TERT transcriptional regulatory factors. Alternative embodiments include novel in vitro and cell-based in vivo assay systems to screen for TERT promoter binding agents (trans-acting TERT transcriptional regulatory factors) using the nucleic acids of the invention.

### c-Myc is a potent activator of TERT gene transcription

Use of recombinant constructs comprising TERT promoter sequences of the invention has, for the first time, demonstrated that c-Myc acts as a potent activator of telomerase activity by direct interaction with cis-acting regulatory sequences in the TERT promoter. c-Myc acts through the rapid up-regulation of hTERT gene expression (Example 8). Significantly, the studies demonstrate that transcriptional activation of the hTERT promoter by c-Myc can be abrogated by deletion or mutation of a single cis-acting regulatory sequence, the "Myc/Max binding site," within the hTERT promoter. Furthermore. the ability of an inducible c-Myc to enhance expression of hTERT is resistant to inhibition of protein synthesis.

### TERT promoter used to drive heterologous gene sequences

The invention also provides constructs in which the TERT promoter sequences of the invention are operably linked to a heterologous gene (in a preferred embodiment, a structural gene). In this way the heterologous gene is transcribed in the same cells at the same time the natural TERT transcript would be expressed. Thus, when the construct is expressed in a transformed cell or transgenic (non-human) animal, the heterologous gene (and protein, if the gene is a coding sequence) is expressed in the same temporal pattern over the same cell range as the wild type, TERT promoter-driven TERT gene.

These constructs are useful for TERT promoter-based assays, for example, to identify biological modulators of TERT and telomerase activity. In alternative embodiments, the heterologous coding sequence operably linked to a TERT promoter of the invention is a marker gene (e.g., alkaline phosphatase, SEAP; β-galactosidase), a modified TERT structural gene or a TERT antisense, a therapeutic gene (e.g., a cytotoxic gene such as thymidine kinase).

In a further embodiment, cytopathic viruses are provided, in particular human cytopathic viruses, such as modified adenovirus or Herpes virus. Viruses, such as adenovirus or Herpes virus require essential virally encoded genes to proliferate and lyse specific cells. If any one of these essential viral genes were modified such that expression of the essential element would be driven by the TERT promoter, proliferation of the virus, and its cytopathic effects, would be restricted to telomerase-expressing cells, in particular tumor cells.

### Definitions

The following terms are defined infra to provide additional guidance to one of skill in the practice of the invention.

The term "amplifying" as used herein incorporates its common usage and refers to the use of any suitable amplification methodology for generating or detecting recombinant or naturally expressed nucleic acid. For example, the invention provides methods and reagents (including specific oligonucleotide PCR primer pairs) for amplifying naturally expressed or recombinant nucleic acids of the invention in vivo or in vitro. An indication that two polynucleotides are "substantially identical" can be obtained by amplifying one of the polynucleotides with a pair of oligonucleotide primers or pool of degenerate primers (e.g., fragments of an TERT promoter sequence) and then using the product as a probe under stringent hybridization conditions to isolate the second sequence (e.g., the TERT promoter sequence) from a genomic library or to identify the second sequence in a Northern or Southern blot.

As used herein, the term "TERT promoter" includes any TERT genomic sequences capable of driving transcription in telomerase activity positive cells. Thus, TERT promoters of the invention include without limitation cis-acting transcriptional control elements and regulatory sequences that are involved in regulating or modulating the timing and/or rate of transcription of a TERT gene. For example, the TERT promoter of the invention comprises cis-acting transcriptional control elements, including enhancers, promoters, transcription terminators, origins of replication, chromosomal integration sequences, 5' and 3' untranslated regions, exons and introns, which are involved in transcriptional regulation. These cis-acting sequences typically interact with proteins or other biomolecules to carry out (turn on/off, regulate, modulate, etc.) transcription.

One of skill in the art will appreciate that the hTERT and mTERT promoter sequences provided herein are exemplary only, and that they may be used as a basis to produce numerous versions of TERT promoters. i.e., promoters that are capable of driving transcription in telomerase activity positive cells. For example, while it is shown herein that a sequence comprising 2447 nucleotides of the disclosed hTERT promoter can drive expression in this manner (pGRN350), one of skill in the art will appreciate that such activity may be obtained using longer or shorter promoter sequences. Furthermore, one of skill in the art will appreciate that promoter sequences that vary from those sequences provided herein by, for example, nucleotide additions, deletions or substitutions may also be used to obtain expression in telomerase activity positive cells. Such variants will share a specified minimum level of structural (sequence) similarity to the disclosed TERT promoter sequences, which similarity may be defined in terms of either sequence identity to the disclosed TERT promoter sequences, or the ability to hybridize to the disclosed sequences at specified levels of hybridization stringency. For example, variant TERT promoters include promoters that hybridize to the TERT promoters disclosed herein (at 37°C in a buffer of 40% formamide, 1 M NaCl, and 1% SDS, followed by a wash in 1X SSC at 45°C), and which are capable of driving transcription in telomerase activity positive cells. Other variant TERT promoters include promoters that share at leasst about 80%, 90%, 95%, 98% or 100% sequence identity with the disclosed TERT promoters. Sequence identity is calculated by first aligning the polynucleotide being examined with the reference counterpart, and then counting the number of residues shared between the sequences being compared as a percentage of the region under examination. No penalty is imposed for the presence of insertions or deletions. but insertions or deletions are permitted only where clearly required to readjust the alignment. The percentage is given in terms of residues in the sequence being examined that are identical to residues in the comparison or reference sequence.

The determination that a promoter is capable of driving transcription in telomerase activity positive cells can be routinely performed as described in Examples 2 and 5. Briefly, the promoter to be tested is operably linked to a coding region that encodes a detectable protein such as alkaline phosphatase or green fluorescent protein. This construct is then introduced into telomerase activity positive (TAP) and tetomerase activity negative (TAN) cells. Detection of the detectable protein in the TAP cells but not in the TAN cells, or of an elevated level of the detectable protein in the TAP compared to the TAN cells (preferably at least a threefold difference) indicates that the promoter is a TERT promoter.

A promoter is said to "preferentially promote transcription" in a cell having a particular phenotype if the level of transcription is at least about 3-fold higher in cells of that phenotype than cells that lack the phenotype. Promoters of this invention preferentially promote transcription in cells expressing TERT, including diseased cells where the disease is associated with overexpression of TERT, such as cancer. There is preferential transcription if the relative increase in cells expressing the stated phenotype is at least about 3-fold, 10-fold, 30-fold or 100-fold higher compared with cells that don't have the phenotype, in order of increasing preference. Promoters that show tower levels of specificity in an assay where just two types of cells are compared may be tested using a larger panel. One skilled in the art will know that TERT positive cells include various types of cancer cells, various types of progenitor cells and stem cells, and under certain conditions, B and T lymphocytes. Suitable negative controls include primary cultures and established cell lines of mature differentiated cells of most tissue types.

In alternative embodiments, the TERT promoter sequence comprises TERT sequences upstream of the translation start site (ATG), for example, in one embodiment, the hTERT promoter comprises residues 44 to 13545 of SEQ ID NO:1. Other embodiments include sequences starting within about one to 5 nucleotides of a translation start codon (for example in SEQ ID NO:1 or SEQ ID NO:2) and ending at about 50, 100, 150, 200, 250, 500, 1000, 2500 or 13500 nucleotides upstream of the translation start codon. Such embodiments can optionally include other regulatory sequences, such as, exon and/or intron sequences. Another embodiment includes TERT intron sequences with regulatory activity, as described in Example 2. hTERT promoters of the invention also include sequences substantially identical (as defined herein) to an exemplary hTERT promoter sequence of the invention, having the sequence set forth by SEQ ID NO:1. Similarly, mTERT promoters of the invention also include sequences substantially identical to an exemplary mTERT promoter sequence of the invention. having the sequence set forth by SEQ ID NO:2.

The term "heterologous" when used with reference to portions of a nucleic acid, indicates that the nucleic acid comprises two or more subsequences which are not found in the same relationship to each other in nature. For instance, the nucleic acid is typically recombinantly produced, having two or more sequences from unrelated genes arranged in a manner not found in nature; such as a promoter sequence of the invention operably linked to a polypeptide coding sequence that, when operably linked, does not reform the naturally occurring TERT gene. For example, the invention provides recombinant constructs (expression cassettes, vectors, viruses, and the like) comprising various combinations of promoters of the invention, or subsequences thereof, and heterologous coding sequences.

As used herein, "isolated," when referring to a molecule or composition, such as an hTERT promoter sequence, means that the molecule or composition is separated from at least one other compound, such as a protein. DNA, RNA, or other contaminants with which it is associated in vivo or in its naturally occurring state. Thus, a nucleic acid sequence is considered isolated when it has been isolated from any other component with which it is naturally associated. An isolated composition can, however, also be substantially pure. An isolated composition can be in a homogeneous state. It can be in a dry or an aqueous solution. Purity and homogeneity can be determined by analytical chemistry techniques such as polyacrylamide gel electrophoresis (PAGE), agarose gel electrophoresis or high pressure liquid chromatography (HPLC).

As used herein, the terms "nucleic acid" and "polynucleotide" are used interchangeably, and include oligonucleotides. They also refer to synthetic and/or non-naturally occurring nucleic acids (including nucleic acid analogues or modified backbone residues or linkages). The terms also refer to deoxyribonucleotide or ribonucleotide oligonucleotides in either single-or double-stranded form. The terms encompass nucleic acids containing known analogues of natural nucleotides. The term also encompasses nucleic acid-like structures with synthetic backbones. DNA backbone analogues provided by the invention include phosphodiester, phosphorothioate, phosphorodithioate, methyl-phosphonate, phosphoramidate, alkyl phosphotriester, sulfamate, 3'-thioacetal, methylene (methylimino), 3'-N-carbamate, morpholino carbamate, and peptide nucleic acids (PNAs); see Oligonucleotides and Analogues, a Practical Approach, edited by F. Eckstein, IRL Press at Oxford University Press (1991); Antisense Strategies, Annals of the New York Academy of Sciences, Volume 600, Eds. Baserga and Denhardt (NYAS 1992); Milligan (1993) J. Med. Chem. 36:1923-1937; Antisense Research and Applications (1993, CRC Press). PNAs contain non-ionic backbones, such as N-(2-aminoethyl) glycine units. Phosphorothioate linkages are described in WO 97/03211; WO 96/39154; Mata (1997) Toxicol. Appl. Pharmacol. 144:189-197. Other synthetic backbones encompassed by the term include methylphosphonate linkages or alternating methylphosphonate and phosphodiester linkages (Strauss-Soukup (1997) Biochemistry 36:8692-8698), and benzyl-phosphonate linkages (Samstag (1996) Antisense Nucleic Acid Drug Dev 6:153-156).

As used herein, the term "operably linked" refers to a functional relationship between two or more nucleic acid segments. Typically, it refers to the functional relationship of a transcriptional regulatory sequence to a transcribed sequence. For example, a TERT promoter sequence of the invention, including any combination of cis-acting transcriptional control elements, is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance.

As used herein, "recombinant" refers to a polynucleotide synthesized or otherwise manipulated in vitro, to methods of using recombinant polynucleotides to produce gene products in cells or other biological systems, or to a polypeptide ("recombinant protein") encoded by a recombinant polynucleotide. "Recombinant means" also encompass the ligation of nucleic acids having coding or promoter sequences from different sources into an expression cassette or vector for expression of a fusion protein; or, inducible, constitutive expression of a protein (for example, a TERT promoter of the invention operably linked to a heterologous nucleotide, such as a polypeptide coding sequence).

As used herein, the "sequence" of a gene (unless specifically stated otherwise) or nucleic acid refers to the order of nucleotides in the polynucleotide, including either or both strands of a double-stranded DNA molecule ― the sequence of both the coding strand and its complement, or of a single-stranded nucleic acid molecule. For example, in alternative embodiments, the promoter of the invention comprises untranscribed, untranslated, and intron TERT sequences, as set forth in the exemplary SEQ ID NO:1 and SEQ ID NO:2.

As used herein, the term "transcribable sequence" refers to any sequence which, when operably linked to a cis-acting transcriptional control element, such as the TERT promoters of the invention, and when placed in the appropriate conditions, is capable of being transcribed to generate RNA.

The terms "identical" or percent "identity," in the context of two or more nucleic acids or polypeptide sequences, refer to two or more sequences or subsequences that are the same or have a specified percentage of nucleotides (or amino acid residues) that are the same, when compared and aligned for maximum correspondence over a comparison window, as measured using one of the following sequence comparison algorithms or by manual alignment and visual inspection. This definition also refers to the complement of a sequence. For example, in alternative embodiments, nucleic acids within the scope of the invention include those with a nucleotide sequence identity that is at least about 60%, at least about 75-80%, about 90%, and about 95% of the exemplary TERT promoter sequence set forth in SEQ ID NO:1 (including residues 44 to 13544 of SEQ ID NO:1) or SEO ID NO:2, and the intron TERT sequences capable of driving a reporter gene in telomerase positive cells. Two sequences with these levels of identity are "substantially identical." Thus, if a sequence has the requisite sequence identity to a TERT promoter sequence or subsequence of the invention, it also is a TERT promoter sequence within the scope of the invention. Preferably, the percent identity exists over a region of the sequence that is at least about 25 nucleotides in length, more preferably over a region that is at least about 50-100 nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identity for the test sequence(s) relative to the reference sequence, based on the designated or default program parameters. A "comparison window", as used herein, includes reference to a segment of any one of the number of contiguous positions selected from the group consisting of from 25 to 600, usually about 50 to about 200, more usually about 100 to about 150 in which a sequence may be compared to a reference sequence of the same number of contiguous positions after the two sequences are optimally aligned. Alignment of sequences can be conducted by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection.

One example of a useful algorithm is PILEUP. PILEUP creates a multiple sequence alignment from a group of related sequences using progressive, pair-wise alignments to show relationship and percent sequence identity. It also plots a tree or dendrogram, showing the clustering relationships used to create the alignment. PILEUP uses a simplification of the progressive alignment method of Feng & Doolittle, J. Mol. Evol. 35:351-360 (1987). The method used is similar to the method described by Higgins & Sharp, CABIOS 5:151-153 (1989). The program can align up to 300 sequences, each of a maximum length of 5,000 nucleotides or amino acids. The multiple alignment procedure begins with the pair-wise alignment of the two most similar sequences. producing a cluster of two aligned sequences. This cluster is then aligned to the next most related sequence or cluster of aligned sequences. Two clusters of sequences are aligned by a simple extension of the pair-wise alignment of two individual sequences. The final alignment is achieved by a series of progressive, pair-wise alignments. The program is run by designating specific sequences and their amino acid or nucleotide coordinates for regions of sequence comparison and by designating the program parameters. Using PILEUP, a reference sequence is compared to another sequence to determine the percent sequence identity relationship (whether the second sequence is substantially identical and within the scope of the invention) using the following parameters: default gap weight (3.00), default gap length weight (0.10), and weighted end gaps. PILEUP can be obtained from the GCG sequence analysis software package (Devereaux (1984) Nuc. Acids Res. 12:387-395).

Another example of algorithm that is suitable for determining percent sequence identity is the BLAST algorithm, which is described in Altschul (1990) J. Mol. Biol. 215:403-410. Software for performing BLAST analyses is publicly available through the National Center for Biotechnology Information: (http://www.ncbi.nim.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul (1990) supra). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues, always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. In one embodiment, to determine if a nudeic acid sequence is within the scope of the invention, the BLASTN program (for nucleotide sequences) is used incorporating as defaults a word-tength (W) of 11, an expectation (E) of 10, M=5, N=4, and a comparison of both strands. For amino acid sequences, the BLASTP program uses as default parameters a word-length (W) of 3, an expectation (E) of 10, and the BLOSUM62 scoring matrix (Henikoff (1989) Proc. Natl. Acad. Sci. USA 89:10915).

The BLAST algorithm also performs a statistical analysis of the similarity between two sequences (Karlin (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

The phrase "selectively (or specifically) hybridizes to" refers to the binding, duplexing, or hybridizing of a molecule to a particular nucleotide sequence under stringent hybridization conditions when that sequence is present in a complex mixture (such as total cellular or library DNA or RNA), wherein the particular nucteotide sequence is detected at least twice background, preferably 10 times background. In one embodiment, a nucleic acid can be determined to be within the scope of the invention according to its ability to hybridize under stringent conditions to another nucleic acid (such as the exemplary sequences described herein).

The phrase "stringent hybridization conditions" refers to conditions under which a probe will primarily hybridize to its target subsequence, typically in a complex mixture of nucleic acid, but to no other sequences. Stringent conditions are sequence-dependent and will be different in different circumstances, depending on the length of the probe. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tm) for the specific sequence at a defined ionic strength and pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (~10 to about 50 nucleotides) and at least about 60°C for long probes (greater than about 50 nucleotides). Stringent conditions may also be achieved with the addition of destabilizing agents such as formamide. For selective or specific hybridization, a positive signal (identification of a nucleic acid of the invention) is about 5-10 times background hybridization. "Stringent" hybridization conditions that are used to identify substantially identical nucleic acids within the scope of the invention include hybridization in a buffer comprising 50% formamide, 5x SSC, and 1% SDS at 42°C, or hybridization in a buffer comprising 5x SSC and 1% SDS at 65°C, both with a wash of 0.2x SSC and 0.1% SDS at 65°C, for long probes. For short probes, stringent hybridization conditions include hybridization in a buffer comprising 50% formamide, 5xSSC and 1% SDS at room temperature or hybridization in a buffer comprising 5 x SSC and 1% SDS at 37°C - 42°C, both with a wash of 0.2 x SSC and 0.1% SDS at 37°C - 42°C. However, as is apparent to one of ordinary skill in the art, hybridization conditions can be modified depending on sequence composition. Moderately stringent hybridization conditions include a hybridization in a buffer of 40% formamide, 1 M NaCl, and 1% SDS at 37°C, and a wash in 1X SSC at 45°C. A positive hybridization is at least twice background. Those of ordinary skill will readily recognize that alternative hybridization and wash conditions can be utilized to provide conditions of similar stringency.

### General Techniques

The TERT promoter sequences of the invention and nucleic acids used to practice this invention, whether RNA, cDNA, genomic DNA, or hybrids thereof, may be isolated from a variety of sources, genetically engineered, amplified, and/or expressed recombinantly. Any recombinant expression system can be used, including, bacterial, yeast, insect or mammalian systems. Alternatively, these nucleic acids can be chemically synthesized in vitro. Techniques for the manipulation of nucleic acids, such as subcloning into expression vectors, labeling probes, sequencing, and hybridization are well described in the scientific and patent literature. Molecular Cloning: A Laboratory Manual (2nd Ed.), Vols. 1-3, Cold Spring Harbor Laboratory, (1989) ("Sambrook"); Current Protocols In Molecular Biology, Ausubel, Ed. John Wiley & Sons, Inc., New York (1997) ("Ausubel"); Laboratory Techniques In Biochemistry And Molecular Biology: Hybridization With Nucleic Acid Probes, Part I. Theory and Nucleic Acid Preparation, Tijssen, ed. Elsevier, N.Y. (1993). Nucleic acids can be analyzed and quantified by any of a number of techniques, including NMR, spectrophotometry, radiography, electrophoresis, capillary electrophoresis, high pressure liquid chromatography (HPLC), thin layer chromatography (TLC), and hyperdiffusion chromatography, fluid or gel precipitin reactions, immunodiffusion (single or double), immunoelectrophoresis, radioimmunoassays (RIAs), enzyme-linked immunosorbent assays (ELISAs), immuno-fluorescent assays, Southern analysis, Northern analysis, dot-blot analysis, gel electrophoresis, RT-PCR, quantitative PCR, other nucleic acid or target or signal amplification methods, radiolabeling, scintillation counting, and affinity chromatography.

### Preparing hTERT promoter sequences

Certain embodiments of the invention are TERT promoters comprising genomic sequences5' (upstream) of an hTERT or mTERT transcriptional start site, and intron sequences. TERT promoters contain cis-acting transcriptional regulatory elements involved in TERT message expression. It will be apparent that, in addition to the nucleic acid sequences provided in hTERT SEQ ID NO:1 or mTERT SEQ ID NO:2, additional TERT promoter sequences can be readily obtained using routine molecular biological techniques. For example, additional hTERT genomic (and promoter) sequence can be obtained by screening a human genomic library using an hTERT nudeic acid probe having a sequence or subsequence as set forth in SEQ ID NO:1 (a nucleic acid sequence is within the scope of the invention if it hybridizes under stringent conditions to an hTERT promoter sequence of the invention). Additional hTERT or mTERT genomic sequence can be readily identified by "chromosome walking" techniques, as described by Hauser (1998) Plant J 16:117-125; Min (1998) Biotechniques 24:398-400. Other useful methods for further characterization of TERT promoter sequences include those general methods described by Pang (1997) Biotechniques 22:1046-1048; Gobinda (1993) PCR Meth. Applic. 2:318; Triglia (1988) Nucleic Acids Res. 16:8186; Lagerstrom (1991) PCR Methods Applic. 1:111; Parker (1991) Nucleic Acids Res. 19:3055.

In some embodiments, the promoter sequence comprises at least about 15, 50, 100, 150, 200, 250, 500, 1000, 2500 or 13,000 bases in SEQ ID NO:1 or SEQ ID NO:2. Included is a nucleic acid molecule comprising a TERT promoter, including but not limited to hTERT or mTERT, optionally linked to a heterologous sequence. The promoter may comprise about 100 to about 200, 200 to about 400, 400 to about 900, or 900 to about 2500, or 2500 to about 5000 nucleotides upstream of a translational start site. In other embodiments, the promoter comprises a sequence that hybridizes with SEQ. ID NO:1 or 2. Exemplary are promoter sequences that preferentially promote transcription in cells expressing telomerase reverse transcriptase. Such sequences can be readily identified using the assays provided elsewhere in this disclosure and in the Examples, in which candidate promoter sequences are operably linked to the encoding region for a reporter protein, and then transfected into cells with known TERT activity to determine the specificity.

The invention provides oligonucleotide primers that can amplify all or any specific region within the TERT promoter sequence of the invention, including specific promoter and enhancer subsequences. The nucleic acids of the invention can also be generated or measured quantitatively using amplification techniques. Using the TERT promoter sequences of the invention (as in the exemplary hTERT SEQ ID NO:1 or mTERT SEQ ID NO:2), the skilled artisan can select and design suitable oligonucleotide amplification primers. Amplification methods include polymerase chain reaction (PCR Protocols, A Guide To Methods And Applications, ed. Innis, Academic Press, N.Y. (1990) and PCR Strategies (1995), ed. Innis, Academic Press, Inc., N.Y., ligase chain reaction (LCR) (Wu (1989) Genomics 4:560; Landegren (1988) Science 241:1077; Barringer (1990) Gene 89:117); transcription amplification (Kwoh (1989) Proc. Natl. Acad. Sci. USA, 86:1173); and, self-sustained sequence replication (Guatelli (1990) Proc. Natl. Acad. Sci. USA, 87:1874); Q β-replicase amplification (Smith (1997) J. Clin. Microbial. 35:1477-1491, automated Q-β replicase amplification assay; Burg (1996) Mol. Cell. Probes 10:257-271) and other RNA polymerase mediated techniques (NASBA. Cangene, Mississauga, Ontario); Berger (1987) Methods Enzymol. 152:307-316, Sambrook, Ausubel, Mullis (1987) U.S. Patent Nos. 4,683,195, and 4,683,202; Arnheim (1990) C&EN 36-47; Lomell J. Clin. Chem., 35:1826 (1989); Van Brunt (1990) Biotechnology, 8:291-294; Wu (1989) Gene 4:560; Sooknanan (1995) Biotechnology 13:563-564. Once amplified, TERT genomic DNA, TERT promoter sequences, and the like, can be cloned, if desired. into any of a variety of vectors using routine molecular biological methods; methods for cloning in vitro amplified nucleic acids are described in Wallace, U.S. Pat. No. 5,426,039.

The invention includes TERT promoter sequences that have been modified in a site-specific manner to alter, add to, or delete some or all of the promoter's functions. For example, specific base pairs can be modified to alter, increase or decrease the binding affinity to trans-acting transcriptional regulatory factors, thus modifying the relative level of transcriptional activation or repression. Modifications can also change secondary structures of specific subsequences, such as those associated with many cis-acting transcriptional elements. Site-specific mutations can be introduced into nucleic acids by a variety of conventional techniques, well described in the scientific and patent literature. Illustrative examples include site-directed mutagenesis by overlap extension polymerase chain reaction (OE-PCR), as in Urban (1997) Nucleic Acids Res. 25:2227-2228; Ke (1997) Nucleic Acids Res 25:3371-3372, and Chattopadhyay (1997) Biotechniques 22:1054-1056, describing PCR-based site-directed mutagenesis "megaprimer" method; Bohnsack (1997) Mol, Biotechnol. 7:181-188; Aitenberg (1997) Biotechniques 22:624-626, describing site-directed mutagenesis using a PCR-based staggered re-annealing method without restriction enzymes; Nicolas (1997) Biotechniques 22:430-434, site-directed mutagenesis using long primer-unique site elimination and exonuclease III. Modified TERT promoter sequences of the invention can be further produced by chemical modification methods. Belousov (1997) Nucleic Acids Res. 25:3440-3444; Frenkel (1995) Free Radic. Biol. Med. 19:373-380; Blommers (1994) Biochemistry 33:7886-7896.

The invention also provides antisense oligonucleotides capable of binding TERT promoter regions which, at least in part, modulate TERT transcription and telomerase activity. For example, antisense oligonucleotides that form triplexes with promoter regions inhibit the activity of that promoter. Joseph (1997) Nucleic Acids Res. 25:2182-2188; Alunni-Fabbroni (1996) Biochemistry 35:16361-16369; Olivas (1996) Nucleic Acids Res 24:1758-1764. Alternatively, antisense oligonucleotides that hybridize to the promoter sequence can be used to inhibit promoter activity.

For example, antisense polynucleotides of the invention can comprise an antisense sequence of at least 7 to 10 to about 20 or more nucleotides that specifically hybridize to a sequence complementary to the TERT promoter sequences of the invention. Alternatively, the antisense polynucleotide of the invention can be from about 10 to about 50 nucleotides in length or from about 14 to about 35 nucleotides in length. In other embodiments, they are less than about 100 nucleotides or less than about 200 nucleotides. In general, the antisense polynucleotide should be long enough to form a stable duplex (or triplex) but, if desired, short enough, depending on the mode of delivery, to be administered in vivo. The minimum length of a polynucleotide required for specific hybridization to a target sequence depends on several factors, such as G/C content, positioning of mismatched bases (if any), degree of uniqueness of the sequence as compared to the population of target polynucleotides, and chemical nature of the nucleotides used in the antisense reagent (methylphosphonate backbone, peptide nucleic acid, phosphorothioate), among other factors. Methods relating to antisense polynucleotides, are also described in Antisense RNA And DNA, (1988), D.A. Melton, Ed., Cold Spring Harbor Laboratory, Cold Spring Harbor, NY); Dagle (1991) Nucleic Acids Research 19:1805; Kim (1998) J. Controlled Release 53:175-182; for antisense therapy. Uhlmann (1990) Chem. Reviews 90:543-584; Poston (1998) J. Thorac. Cardiovasc. Surg. 116:386-396 (ex vivo gene therapy); Haller (1998) Kidney Int. 53:1550-1558; Nguyen (1998) Cancer Res 58:5673-7.

### Identifying TERT promoter subsequences bound by transcriptional regulatory factors

The invention provides means to identify and isolate trans-acting transcriptional regulatory factors that are involved in modulating the activity of the TERT promoter. Identification of cis-acting motifs by sequence identity comparison can be a useful initial means to identify promoter sequences bound by trans-acting factors. The hTERT promoter contains the motif known to bind to c-Myc (the "E-box" or "Myc/Max binding site"). Two SP1 binding sites are located starting at residue -168 and starting at residue -134. Other identified motifs include the sex determining region Y gene product (SRY), hepatic nuclear factor 3-beta (HNF-3β) and hepatic nuclear factor 5 (HNF-5), TFIID-MBP, E2F and c-Myb cis-acting transcriptional regulatory elements. To identify these motifs, a variety of comparison algorithms can be used. Karas (1996) Comput. Appl. Biosci. 12:441-6; Frech (1997) Pac Symp Biocomput. 7:151-62; Brzma (1998) Genome Res 8:1202-1215; Tsunoda (1998) Pac Symp Biocomput :1998:252-83.

In addition to sequence identity analysis, TERT cis-acting transcriptional regulatory elements can be identified by functional assays, including promoter activity assays, DNase assays, binding assays (mobility shift assays), and oligonucleotide affinity column chromatography. After positive or tentative identification of a cis-acting binding site in a TERT promoter, these sequences are used to isolate the trans-acting transcriptional regulatory factor(s). In a preferred embodiment, the trans-acting factors are isolated using sequence-specific oligonucleotide affinity chromatography, the oligonucleotides comprising TERT sequences of the invention.

Another embodiment for identifying transcriptional regulatory motifs involves modifying putative cis-acting regulatory subsequences and assessing the change, if any, of the resultant TERT promoter to modulate transcription. The modification can be one or more residue deletions, residue substitutions, and chemical alterations of nucleotides. The (modified) promoter can be operably linked to TERT, a reporter gene, or any other transcribable sequence.. The relative increase or decrease the modification has on transcriptional rates can be determined by measuring the ability of the unaltered TERT promoter to transcriptionally activate the reporter coding sequence under the same conditions as used to test the modified promoter. An increase or decrease in the ability of the modified TERT promoter to induce transcription as compared to the unmodified promoter construct identifies a cis-acting transcriptional regulatory sequence that is involved in the modulation of TERT promoter activity.

The reporter gene can encode a fluorescent or phosphorescent protein, or a protein possessing enzymatic activity. In alternative embodiments, the detectable protein is firefly luciferase, α-glucuronidase, α-galactosidase, chloramphenicol acetyl transferase, green fluorescent protein, enhanced green fluorescent protein, and the human secreted alkaline phosphatase. Another embodiment tests the ability of these cis-acting elements to bind soluble polypeptide trans-acting factors isolated from different cellular compartments, particularly trans-acting factors expressed in nuclei. For identification and isolation of factors that stimulate transcription, nuclear extracts from cells that express TERT are used.

Furthermore, once a cis-acting motif, or element, is identified, it can be used to identify and isolate trans-acting factors in a variety of cells and under different conditions (such as cell proliferation versus cell senescence). Accordingly, the invention provides a method for screening for trans-acting factors that modulate TERT promoter activity under a variety of conditions, developmental states, and cell types (including normal versus immortal versus malignant phenotypes). The cis-acting transcriptional regulatory sequences of the invention that modulate TERT promoter activity can also be used as oligonucleotides which, upon introduction into a cell, can bind trans-acting regulatory factors to modulate TERT transcription in vivo. This results in increased or decreased cell proliferative capacity for the treatment of various diseases and conditions.

### High throughput screening of small molecule modulators of TERT transcription

The invention provides constructs and methods for screening modulators, in a preferred embodiment, small molecule modulators, of TERT promoter activity in vitro and in vivo. The invention incorporates all assays available to screen for small molecule modulators of TERT transcription. In a preferred embodiment, high throughput assays are adapted and used with the novel TERT promoter sequences and constructs provided by the invention. Schultz (1998) Bioorg Med Chem Lett 8:2409-2414; Weller (1997) Mol Divers. 3:61-70; Femandes (1998) Curr Opin Chem Biol 2:597-603; Sittampalam (1997) Curr Opin Chem Biol 1:384-91.

In alternative embodiments, recombinant constructs contain hTERT promoter sequences driving a marker, such as an alkaline phosphatase marker gene (SEAP) or a §-galactosidase gene. Using a SEAP expressing construct of the invention, it was demonstrated that a TERT promoter fragment of approximately 2.5 kb is sufficient to activate and repress TERT transcription in response to proliferation and/or growth arrest stimuli in a model cell line, IDH4. Two cell clones, ID245-1 and ID245-16 whose SEAP profiles closely matched telomerase activity after TERT up-regulation by dexamethasone were selected and expanded for high throughput screening of small molecule activators of telomerase.

### Treatment of diseases associated with altered telomerase expression

The present invention provides TERT promoter sequences useful for the treatment of diseases and disease conditions. The recombinant and synthetic nucleic acids comprising TERT promoter, or TERT antisense complementary sequences, can be used to create or elevate telomerase activity in a cell, as well as to inhibit telomerase activity in cells in which it is not desired. In a preferred embodiment, human TERT promoter sequences or antisense sequences are used for the treatment of human diseases and disease conditions.

Identification of cis-acting transcriptional regulatory sequences by the invention further provides for the design of targeted sequences that, as oligonucleotides, can modify TERT promoter activity. In one embodiment, telomerase activity is created or elevated by binding significant amounts of a trans-acting transcriptional repressor or down-regulator with a nucleic acid that binds specifically to the repressor. In another embodiment, telomerase activity is down-regulated by antisense oligonucleotides binding to promoter sequences. Similarly, telomerase activity can be inhibited by binding significant amounts of a trans-acting transcriptional activator or up-regulator with a nucleic acid that binds specifically to the activator; or telomerase activity is up-regulated by antisense oligonucleotides binding to promoter sequences involved in telomerase repression. Thus, inhibiting, activating or otherwise altering a telomerase activity (telomerase catalytic activity, fidelity, processivity, telomere binding, etc.) in a cell can be used to change the proliferative capacity of the cell.

For example, reduction of telomerase activity in an immortal cell, such as a malignant tumor cell, can render the cell mortal. Conversely, increasing the telomerase activity in a cell line or a mortal cell (most human somatic cells) can increase the proliferative capacity of the cell. For example, expression of hTERT protein in dermal fibroblasts, thereby increasing telomere length, will result in increased fibroblast proliferative capacity. Such expression can slow or reverse age-related degenerative processes, such as the age-dependent slowing of wound closure (West (1994) Arch. Derm. 130:87). Thus, in one aspect, the present invention provides reagents and methods useful for treating diseases and conditions characterized by the presence, absence, or altered amount of human telomerase activity in a cell (where the diseases and conditions are susceptible to treatment using the compositions and methods disclosed herein). These diseases include, e.g. cancers, other diseases of cell proliferation (particularly, degenerative and aging processes and diseases of aging). immunological disorders, infertility (or fertility).

### TERT promoter operably linked to cellular toxins

In one embodiment, the TERT promoter of the invention is operably linked to a transcribable sequence that encodes a cellular toxin. Polypeptide toxins that can be recombinantly generated include ricin, abrin (Hughes (1996) Hum. Exp. Toxicol. 15:443-451), diphtheria, gelonin (Rosenblum (1996) Cancer Immunol. Immunother. 42:115-121), Pseudomonas exotoxin A, tumor necrosis factor alpha (TNF-α), Crotalus durissus terrificus toxin, Crotalus adamenteus toxin, Naja naja toxin, and Naja mocambique toxin. Rodriguez (1998) Prostate 34:259-269; Mauceri (1996) Cancer Res. 56:4311-4314. The cellular toxin can also be capable of inducing apoptosis, such as the ICE-family of cysteine proteases, the Bcl-2 family of proteins, bax, bcIXs and caspases. Favrot (1998) Gene Ther. 5:728-739; McGill (1997) Front. Biosci. 2:D353-D379; McDonnell (1995) Semin. Cancer Biol. 6:53-60.

Alternatively, the sequence under the control of the TERT promoter can code for polypeptides having activity that is not itself toxic to a cell, but which renders the cell sensitive to an otherwise nontoxic drug, such as Herpes virus thymidine kinase (HSV-TK). The HSV-TK is innocuous but converts the anti-herpetic agent ganciclovir (GCV) to a toxic product that interferes with DNA replication in proliferating cells. Delaney (1996) J. Neurosci. 16:6908-6918; Heyman (1989) Proc. Natl. Acad. Sci. USA 86:2698-2702. The art describes numerous other suitable toxic or potentially toxic proteins and systems that may be applied in this embodiment.

The methods of the invention, in addition to enabling the specific killing of telomerase-positive cells, can also be used to prevent transformation of telomerase negative cells to a telomerase positive state. As shown in the examples below, an hTERT promoter sequence can be operably linked to a reporter gene such that activation of the promoter results in expression of the protein encoded by the reporter gene. If, instead of a reporter protein, the encoded protein is toxic to the cell, activation of the promoter leads to cell morbidity or death.

### Oncolytic viruses and toxins for treating cancer

The present invention provides methods and compositions for reducing TERT promoter activity (and hence telomerase activity) in immortal cells and tumor cells for treating cancer. Cancer cells (malignant tumor cells) that express telomerase activity (telomerase-positive cells) can be mortalized by decreasing or inhibiting TERT promoter activity. Moreover, because measurable telomerase activity levels correlate with disease characteristics such as metastatic potential (U.S. Patent Nos. 5,639,613; 5,648,215; 5,489,508; and Pandita (1996) Proc. Am. Ass. Cancer Res. 37:559), any reduction in TERT promoter activity could reduce the aggressive nature of a cancer to a more manageable disease state.

Taking advantage of this characteristic, in one embodiment of the invention, a TERT promoter sequence is operably linked to a gene encoding a toxin and introduced into a cell to kill the cell (such as ricin, diphtheria, gelonin, Pseudomonas toxin, abrin). If or when TERT transcriptional activators are expressed or activated in the cell, the toxin will be expressed, resulting in specific cell killing.

Alternatively, the TERT promoter-linked gene can encode a protein having activity that is not itself toxic to a cell, but which renders the cell sensitive to an otherwise nontoxic drug (such as Herpes virus thymidine kinase).

In another embodiment, the invention takes advantage of the fact that normal cytopathic viruses, in particular human cytopathic viruses, such as adenovirus or Herpes virus, require essential virally encoded genes to proliferate thereby lysing specific cells. Based on the description that follows, those skilled in the art will recognize that a number of different cytopathic viruses can be adapted according to this invention. Cytopathic viruses are well known in the art, and are described *inter alia* in publications by Coffey, Toda, Chase, and Kramm, *infra.* Genes essential for replication have been characterized in many such viruses. If an essential replication gene of any of these viruses is driven by the TERT promoter, proliferation of the virus and its cytopathic effects would be restricted to tumor cells and other telomerase expressing cells. For example, some essential genetic elements for replication of adenovirus are the E4. E1a, E1b, and E2 regions, or any of the late gene products. Essential genetic elements for replication of HSV-1 include ICP6 and ICP4.

Accordingly, the invention provides constructs and methods for killing telomerase positive cells (such as cancer cells) wherein TERT promoter sequences of the invention are operably linked to such essential replication genetic elements. For use in human cells, human cytopathic viruses modified with hTERT promoter sequences are preferred. Any one or more of the genes required for the replication and packaging of the virus could be modified to be driven by the TERT promoter. For instance, in one embodiment, expression of the E1a gene of adenovirus, which is required for the activation of expression of a cascade of adenoviral genes, is placed under the control of the hTERT promoter.

Thus, expression of E1a, and hence downstream replication of the virus, occurs only in those cells that express telomerase (such as tumor cells). Likewise, a recombinant adenovirus of the invention is designed so the adenoviral capsid genes are under the control of a TERT promoter. While this construct replicates its DNA in most cell types, it packages itself into active, infectious (and cytotoxic) virus only in those cells that express telomerase. Thus, when these constructs are used as cancer therapeutics, the conditionally replicative virus only infects and yields a productive infection in tumor cells (with no effect in "normal" cells that do not express telomerase). infection of normal cells that do not express telomerase is expected to produce either no virus or abortive production of the virus, depending on which gene is driven by the TERT promoter. Thus, these recombinant viruses of the invention allow the natural, yet tumor specific, amplification of an oncolytic virus.

In alternative embodiments, many other elements are incorporated into a TERT promoter restricted oncolytic virus or a TERT promoter restricted replicative virus that is not tytic. Genes encoding suicide genes, marker genes, apoptotic genes or cell cycle regulators are incorporated in the TERT promoter restricted conditionally replicative recombinant virus. Expression of these elements in such a virus would assist the arrest of tumor growth. In one embodiment, elements to be included within these conditionally replicative viruses of the invention are structures that inhibit telomerase activity. These tetomerase inhibitors could incorporate inhibitory oligonucleotides, dominant-negative inhibitors of TERT, or the gene for any agent that would disrupt or prevent TR/TERT assembly, interactions, or activity.

Other elements can also be included in the TERT promoter restricted vectors of the invention. For example, small inhibitory RNA molecules, preferably targeting cancer cells, such as RNA targeting telomerase activity can be synthesized in vivo using a recombinant adenovirus vector. Exemplary sequences are provided in US Patent No. 5,858,777 and GB 20890.4. RNA production from the adenovirus can be achieved by a variety of expression cassettes. For cell growth inhibition purposes. RNA polymerase III expression cassettes based on the structure of tRNA genes and other RNA polymerase III transcripts, including the U6 snRNA gene, as well as RNA polymerase II snRNP (U1, U2) transcripts are preferred due to their ability to produce high levels of transcripts.

The hTERT promoter restricted viruses of the invention can be designed to express inhibitory RNAs, as antisense molecules complementary to several regions of the hTR molecule, including the template region. The inhibitory RNAs can also mimic sequences and/or structures present in the RNA component of telomerase (e.g., hTR), including potential binding site(s) for TERT or other telomerase-associated proteins that might interact with the RNA component. Other elements can also be designed to generate inhibitory RNAs to target TERT mRNA by preventing its normal processing, folding, modification, transport and/or translation.

Other cytopathic viral vectors of the invention can be designed to generate RNA molecules with sequences necessary for cytoplasmic export and translation into peptides. The resulting polypeptides or peptides can be designed to target telomerase components or other molecules that are associated with telomerase thereby influencing telomerase catalytic activity. The peptides that inhibit telomerase will be produced at high level, paralleling the amount of RNA. For example, peptides could be designed to mimic the stretch of amino acids in hTERT involved in its binding to hTR, thereby acting as competitors in the assembly of a functional telomerase.

The TERT promoter restricted viral vectors of the invention can also be designed to generate peptides or polypeptides for any domain of TERT involved in interactions with other proteins and disrupt contacts that are essential for telomerase function. Other TERT promoter restricted viruses of the invention can be designed to generate polypeptides to bind to telomere complexes and prevent access and/or docking of telomerase or to generate immunogenic peptides. in part TERT peptides.

Other TERT promoter restricted viral vectors of the invention can be designed to generate polypeptides to mimic a variety of apoptosis inducing agents observed during programmed cell death and could result in the onset of apoptosis. TERT promoter restricted viruses do not necessarily need to be cytopathic. The TERT promoter conditionally restricted virus could be used to amplify any sequences or any element in any TERT expressing cell, such as a tumor cell.

Any of these embodiments can be provided with the conditionally replicative viruses of the invention. The TERT promoter constructs of the invention can also be used in gene therapy vectors to prevent telomerase activation and result in specific mortalization or death of telomerase-positive cells. Similarly, these gene therapy methods may be used for treating a genetic predilection for cancers.

### Treatment of other conditions

The present invention also provides compositions and methods useful for treatment of diseases and disease conditions (in addition to cancers) characterized by under- or over-expression of telomerase or TERT gene products. Examples include diseases of cell proliferation, diseases resulting from cell senescence (particularly processes and diseases of aging), immunological disorders, infertility, and diseases of immune dysfunction. Certain diseases of aging are characterized by cell senescence-associated changes due to: reduced telomere length (compared to younger cells), resulting from the absence (or much lower levels) of telomerase activity in the cell. Decreased telomere length and decreased replicative capacity contribute to these diseases. Tetomerase activity (resulting in increased telomere length) can be up-regulated by increasing TERT promoter activity in the cell.

The present invention, by providing methods and compositions for modulating TERT promoter activity, also provides methods to treat infertility. Human germline cells (spermatogonia cells, their progenitors or descendants) are capable of indefinite proliferation and characterized by high telomerase activity. Abnormal or diminished levels of TERT gene products can result, in inadequate or abnormal production of spermatozoa, leading to infertility or disorders of reproduction. Accordingly, infertility associated with altered telomerase activity can be treated using the methods and compositions described herein to increase TERT promoter activity levels. Similarly, because inhibition of telomerase may negatively impact spermatogenesis, oogenesis, and sperm and egg viability, the compositions of the invention capable of inhibiting hTERT promoter activity can have contraceptive effects when used to reduce hTERT levels in germline cells.

In a further embodiment, the invention provides methods and composition useful for decreasing the proliferative potential of telomerase-positive cells such as activated lymphocytes and hematopoietic stem cells by reducing TERT promoter activity. Thus, the invention provides means for effecting immunosuppression. Conversely, the methods and reagents of the invention are useful in immunostimulation by increasing TERT promoter activity (resulting in increased proliferative potential) in immune cells, including hematopoietic stem cells (that express a low level of telomerase or no telomerase prior to therapeutic intervention).

### Modulating TERT promoter activity

As is clear from the foregoing discussion, modulation of the level of TERT promoter transcriptional activity (and thus, the levels of tetomerase or telomerase activity of a cell) can have a profound effect on the proliferative potential of the cell, and so has great utility in treatment of disease. This modulation can either be a decrease or an increase in TERT promoter activity. The promoter activity-modulatory nucleic acid molecules of the invention can act through a number of mechanisms. However, the invention is not limited to any particular mechanism of action.

For example, TERT promoter activity may be decreased or increased by single stranded antisense sequences that directly bind to TERT promoter sequences. This will result in decrease in affinity or inhibition of trans-acting transcriptional regulatory factors binding to critical TERT promoter sequences (TATA boxes, CAAT boxes, and the like). When the cis-acting element bound by a trans-acting factor has inhibitory activity, the binding of the oligonucleotide would result in up-regulation of TERT transcription. Conversely, if the promoter subsequence, when bound by a trans-acting factor, has up-regulating activity, the binding of the oligonucleotide would result in down-regulation of TERT transcription. In another embodiment, double-stranded oligonucleotides representing TERT promoter subsequences directly bind trans-acting transcriptional modulatory elements, thus preventing them from binding their corresponding cis-acting elements. In summary, TERT promoter activity may be increased or decreased through any of several mechanisms, or a combination of mechanisms. These include any means apparent to those of skill upon review of this disclosure.

The cis-acting transcriptional regulatory sequences of the invention can also be used as oligonucleotides which, upon introduction into a cell, can bind trans-acting regulatory factors to modulate TERT transcription in vivo. These oligonucleotides can be delivered to target cells through an appropriate delivery scheme or they can be synthesized in vivo by recombinant expression systems (vectors, viruses, and the like).

### Oligonucleotides and other pharmaceutical compositions

Antisense oligonucleotides which hybridize to TERT promoter sequences will inhibit the binding of trans-acting transcriptional regulatory agents to critical TERT promoter sequences. Furthermore, the result will be activation or repression of TERT transcriptional activity, depending on whether the promoter subsequence is down-regulatory or up-regulatory, respectively. Thus, the invention provides antisense oligonucleotides directed to the TERT promoter (cis-acting) binding sites for c-Myc (the "E-box" or "Myc/Max binding sites"), SP1, Y gene product (SRY), HNF-3β, HNF-5, TFIID-MBP, E2F, c-Myb, TATA boxes, CAAT boxes, and other regulatory elements.

TERT polynucleotides can be produced by direct chemical synthesis. Chemical synthesis will typically be used to produce oligonucleotides and polynucleotides containing nonstandard nucleotides (probes, primers and antisense oligonucleotides) although nucleic acids containing only standard nucleotides can also be prepared. Direct chemical synthesis of nucleic adds can be accomplished for example by the phosphotriester method of Narang (1979) Meth. Enzymol. 68:90; the phosphodiester method of Brown (1979) Meth. Enzymol. 68:109; the diethyl-phosphoramidite method of Beaucage (1981) Tetra. Lett. 22:1859; and the solid support method of U.S. Patent No. 4,458,066. Chemical synthesis typically produces a single stranded oligonucleotide, which may be converted into double stranded DNA by hybridization with a complementary sequence, or by polymerization with a DNA polymerase and an oligonucleotide primer using the single strand as a template. One of skill will recognize that while chemical synthesis of DNA is often limited to sequences of less than about 100 or 150 bases, longer sequences may be obtained by the ligation of shorter sequences or by more elaborate synthetic methods. It will be appreciated that the polynucleotides and oligonucleotides of the invention can be made using nonstandard bases (other than adenine, cytidine, guanine, thymine, and uridine) or nonstandard backbone structures to provide desirable properties (increased nuclease-resistance, tighter binding, stability or a desired Tm). Techniques for rendering oligonucleotides nuclease-resistant include those described in PCT publication WO 94/12633. A wide variety of useful modified oligonucleotides may be produced, including oligonucleotides having a peptide nucleic acid (PNA) backbone (Nielsen (1991) Science 254:1497) or incorporating 2'-O-methyl ribonucleotides, phosphorothioate nucleotides, methyl phosphonate nucleotides, phosphotriester nucleotides, phosphorothioate nucleotides, and phosphoramidates. Still other useful oligonucleotides may contain alkyl and halogen-substituted sugar moieties comprising one of the following at the 2' position: OH, SH, SCH₃, F, OCN, OCH₃OCH₃, OCH₃O(CH₂)nCH₃, O(CH₂)nNH₂ or O(CH₂)nCH₃ where n is from 1 to about 10; C1 to C10 lower alkyl, substituted lower alkyl, alkaryl or aralkyl; Cl; Br; CN; CF₃; OCF₃: O-, S-, or N-alkyl; O-, S-, or N-alkereyl; SOCH₃ ; SO₂CH₃; ONO₂; NO₂; N₃; NH₂; heterocycloalkyl; heterocycloalkaryl; amino-alkylamino; polyalkylamino; substituted silyl; an RNA cleaving group; a cholesteryl group; a folate group; a reporter group; an intercalator; a group for improving the pharmacokinetic properties of an oligonucleotide; or a group for improving the pharmacodynamic properties of an oligonucleotide and other substituents having similar properties. Folate, cholesterol or other groups which facilitate oligonucleotide uptake, such as lipid analogs, may be conjugated directly or via a linker at the 2' position of any nucleoside or at the 3' or 5' position of the 3'-terminal or 5'-terminal nucleoside, respectively. One or more such conjugates may be used. Oligonucleotides may also have sugar mimetics such as cyclobutyls in place of the pentofuranosyl group. Other embodiments may include at least one modified base form or "universal base" such as inosine, or inclusion of other nonstandard bases such as queosine and wybutosine as well as acetyl-, methyl-, thio- and similarly modified forms of adenine, cytidine, guanine, thymine, and uridine which are not as easily recognized by endogenous endonucleases. The invention further provides oligonucleotides having backbone analogues such as phosphodiester, phosphorothioate, phosphorodithioate, methylphosphonate, phosphor-amidate, alkyl phosphotriester, sulfamate, 3'-thioacetal, methylene(methylimino), 3'-N-carbamate, morpholino carbamate, chiral-methyl phosphonates, nucleotides with short chain alkyl or cycloalkyl intersugar linkages, short chain heteroatomic or heterocyclic intersugar ("backbone") linkages, or CH₂-NH-O-CH₂, CH₂-N(CH₃)-OCH₂, CH₂-O-N(CH₃)-CH₂, CH₂-N(CH₃)-N(CH₃)-CH₂ and O-N(CH₃)-CH-₂-CH₂ backbones (where phosphodiester is O-P-O-CH₂), or mixtures of the same. Also useful are oligonucleotides having morpholino backbone structures (U.S. Patent No. 5,034,506).

While the invention is not limited by any particular mechanism, oligonucleotides of the invention can also bind to double-stranded or duplex TERT promoter sequences. They can bind in a folded region, forming a triple helix, or "triplex" nucleic acid. Triple helix formation results in inhibition of TERT promoter activity by, disrupting the secondary structure of the promoter sequence, resulting in a new conformation which the trans-acting factor cannot bind with sufficient affinity to have a transcriptional-modifying effect. Alternatively, triple helix formation (induced by the binding of the antisense oligonucleotide of the invention) compromises the ability of the double helix to open sufficiently for the binding of polymerases, transcription factors, or regulatory trans-acting molecules to occur. Triplex oligonucleotide and polynucleotide construction is described in Cheng (1988) J. Biol. Chem. 263:15110; Ferrin (1991) Science 354:1494; Ramdas (1989) J. Biol. Chem. 264:17395; Strobel (1991) Science 254:1639; Rigas (1986) Proc. Natl. Acad. Sci. U.S.A. 83: 9591) Carr, 1994, Molecular and Immunological Approaches, Futura Publishing Co, Mt Kisco NY; Rininsland (1997) Proc. Natl. Acad. Sci. USA 94:5854; Perkins (1998) Biochemistry 37:11315-11322.

The therapeutic nucleic acids and methods of the invention involve the administration of oligonucleotides or polynucleotides that function to inhibit or stimulate TERT promoter activity under in vivo physiological conditions. In one embodiment, these nucleic acids are single stranded antisense sequences capable of binding to promoter sequences. In an alternative embodiment, they are double stranded nucleic acids capable of binding trans-acting transcriptional regulatory factors. They should be sufficiently stable under physiological conditions for a period of time to obtain a therapeutic effect. Modified nucleic acids can be useful in imparting such stability, as well as for targeting delivery of the oligonucleotide to the desired tissue, organ, or cell. Oligo- and poly-nucleotides can be delivered directly as a drug in a suitable pharmaceutical formulation, or indirectly by means of introducing a nucleic acid expression system that can recombinantly generate the hTERT promoter modulating oligonucleotides into a cell. In one embodiment, oligonucleotides directly bind to cis-acting sequences or, alternatively, bind to trans-acting regulatory factors. One embodiment exploits the fact that the TERT promoter is only relatively active in a very limited range of cell types, including, significantly, cancer cells.

Oligonucleotides or expression vectors can be administered by liposomes, immunoliposomes, ballistics, direct uptake into cells, and the like. For treatment of disease the oligonucleotides of the invention are administered to a patient in a therapeutically effective amount, which is an amount sufficient to ameliorate the symptoms of the disease or modulate hTERT promoter activity (thereby affecting telomerase activity) in the target cell. Methods useful for delivery of oligonucleotides for therapeutic purposes are described in U.S. Patent 5,272,065. Telomerase activity can be measured by TRAP assay or other suitable assay of telomerase biological function, as discussed in detail in other publications.

The invention provides pharmaceutical compositions that comprise TERT promoter-containing nucleic acids (polynucleotides, expression vectors, gene therapy constructs) alone or in combination with at least one other agent, such as a stabilizing compound, diluent, carrier, cell targeting agent, or another active ingredient or agent. The therapeutic agents of the invention may be administered in any sterile, biocompatible pharmaceutical carrier, including, but not limited to, saline, buffered saline, dextrose, and water. Any of these molecules can be administered to a patient alone, or in combination with other agents, drugs or hormones, in pharmaceutical compositions where it is mixed with suitable excipients, adjuvants, and/or pharmaceutically acceptable carriers.

The pharmaceutical compositions of the invention can be administered by any means. Methods of parenteral delivery include topical, intra-arterial, intramuscular (IM), subcutaneous (SC), intramedullary, intrathecal, intraventricular, intravenous (IV), intraperitoneal (IP), or intranasal administration. Further details on techniques for formulation and administration may be found in the latest edition of Remington's Pharmaceutical Sciences (Maack Publishing Co, Easton PA); PCT publication WO 93/23572.

Pharmaceutical compositions of the invention include TERT-containing nucleic acids in an effective amount to achieve the intended purpose. "Therapeutically effective amount" or "pharmacologically effective amount" are well recognized phrases and refer to that amount of an agent effective to produce the intended pharmacological result. For example, a therapeutically effective amount is an amount sufficient to treat a disease or condition or ameliorate the symptoms of the disease being treated. Useful assays to ascertain an effective amount for a given application includes measuring the effect on endogenous TERT promoter activity and telomerase activity in a target cell. The amount actually administered will be dependent upon the individual to which treatment is to be applied, and will preferably be an optimized amount such that the desired effect is achieved without significant side effects. The therapeutically effective dose can be estimated initially either in cell culture assays or in any appropriate animal model. The animal model is also used to estimate appropriate dosage ranges and routes of administration in humans. Thus, the determination of a therapeutically effective dose is well within the capability of those skilled in the art.

### Cells lines and animals with modified promoter sequences

Most vertebrate cells senesce after a finite number of divisions in culture (∼50 to 100 divisions). Certain variant cells, however, are able to divide indefinitely in culture (e.g., HeLa cells, 293 cells) and, for this reason, are useful for research and industrial applications. Usually these immortal cell lines are derived from spontaneously arising tumors, or by transformation by exposure to an oncogene, radiation or a tumor-inducing virus or chemical. Unfortunately, a limited selection of cell lines, especially human cell lines representing differentiated cell function, is available. Moreover, many immortal cell lines presently available are characterized by chromosomal abnormalities (aneuploidy, gene rearrangements, or mutations). Further, many long-established cell lines are relatively undifferentiated. Thus, there is a need for the TERT promoter activating compositions and methods of the invention to generate new immortal cell lines, especially using cells of human origin, where hTERT promoter activating compositions and methods are preferred.

The "immortalized cells" of the invention are not limited to those that proliferate indefinitely, but also include cells with increased proliferative capacity compared to similar cells whose TERT promoter has not been up-regulated. Depending on the cell type, increased proliferative capacity may mean proliferation for at least about 50, about 100, about 150, about 200, or about 400 or more generations, or for at least about 3, about 6, about 12, about 18, about 24 or about 36 or more months in culture.

Uses for cells with increased proliferative capacity include the production of natural proteins and recombinant proteins (therapeutic polypeptides such as erythropoietin, human growth hormone, insulin, and the like). or antibodies, for which a stable, genetically normal cell line is preferred. Another use is for replacement of diseased or damaged cells or tissue. For example, autologous immune cells immortalized using an TERT promoter sequence of the invention can be used for cell replacement in a patient after aggressive cancer therapy, such as whole body irradiation. Another use for immortalized cells is for ex vivo production of "artificial" tissues or organs for therapeutic use. Another use for such cells is for screening or validation of drugs, such as telomerase-inhibiting drugs, or for use in production of vaccines or biological reagents. Additional uses of the cells of the invention will be apparent to those of skill.

The invention also provides non-human transgenic animals comprising heterologous TERT or recombinant constructs comprising endogenous TERT promoter. In a preferred embodiment, the transgenic animals of the invention comprise a TERT promoter driving a heterologous gene, such as a reporter gene coding sequence. in a preferred embodiment, an hTERT promoter of the invention is operably linked to a reporter gene in a transgenic mouse. Alternatively, an mTERT promoter is operably linked to a reporter gene in a transgenic mouse. These transgenic animals are very useful as in vivo animal models to screen for modulators of TERT transcriptional activity. The introduction of hTERT, mTERT or other TERT promoters into animals to generate transgenic models is also used to assess the consequences of mutations or deletions to the transcriptional regulatory regions.

In one embodiment, the endogenous TERT gene in these mice is still functional and wild-type (native) telomerase activity can still exist. A TERT promoter of the invention is used to drive a high level expression of an exogenous TERT construct, the endogenously produced mTERT protein can be competitively replaced with the introduced, exogenous TERT protein. This transgenic animal (retaining a functional endogenous telomerase activity) is preferred in situations where it is desirable to retain "normal," endogenous telomerase function and telomere structure. In other situations, where it is desirable that all telomerase activity is by the introduced exogenous TERT protein, a mTERT knockout line can be used

Promoter function, and in a preferred embodiment. hTERT promoter function, can be assessed with these transgenic animals. Alterations of TERT promoters can be constructed that drive TERT or a reporter gene to assess their function and expression pattern and characteristics (the invention also provides constructs and animals and methods for gene expression driven by a TERT promoter by transient transfection).

In one embodiment, the TERT promoters and reagents of the invention are used to create mouse cells and transgenic animals in which the endogenous TERT promoter is deleted, modified, supplemented or inhibited. For example, TERT promoter sequences can be deleted, modified or inhibited on either one or both alleles. The cells or animals can be reconstituted with a wild-type or modified TERT promoter, or, in a preferred embodiment, an exogenous TERT in the form of hTERT.

Construction of a "knockout" cell and animal is based on the premise that the level of expression of a particular gene in a mammalian cell can be decreased or completely abrogated by introducing into the genome a new DNA sequence that serves to interrupt some portion of the DNA sequence of the gene/promoter to be suppressed. To prevent expression of endogenous promoter, simple mutations that alter or disrupt the promoter can be suitable. To up-regulate expression, a native TERT promoter can be substituted with a heterologous or mutated TERT promoter that induces higher levels of transcription, or with multiple copies of transgene TERT promoters. Also, "gene trap insertion" can be used to disrupt a host gene, and mouse embryonic stem (ES) cells can be used to produce knockout transgenic animals, as described herein and in Holzschu (1997) Transgenic Res 6: 97-106.

Vectors specifically designed for integration by homologous recombination comprising TERT promoter sequences are also provided by the invention, important factors for optimizing homologous. recombination indude the degree of sequence identity and length of homology to chromosomal sequences. The specific sequence mediating homologous recombination is also important, because integration occurs much more easily in transcriptionally active DNA. Methods and materials for constructing homologous targeting constructs are described by Mansour (1988) Nature 336: 348; Bradley (1992) Bio/Technotogy 10:534; U.S. Patent Nos. 5,627,059; 5,487,992; 5.631,153; and 5,464,764.

In a preferred embodiment, cell and transgenic animal models express TERT promoter (particularly, hTERT promoter) operably linked to a reporter gene. The cell or animal can be a TERT promoter "knockout" or it can retain endogenous TERT promoter activity. The insertion of the TERT promoter-containing exogenous sequence is typically by homologous recombination between complementary nucleic acid sequences. Thus, the exogenous sequence, which is typically an hTERT or mTERT promoter of this invention, is some portion of the target gene to be modified, such as exon, intron or transcriptional regulatory sequences, or any genomic sequence which is able to affect the level of the target gene's expression; or a combination thereof. The construct can also be introduced into other locations in the genome. Gene targeting via homologous recombination in pluripotential embryonic stem cells allows one to modify precisely the genomic sequence of interest.

In another embodiment, the introduced TERT promoter sequence (modified or wild type) can replace or disrupt an endogenous TERT promoter sequence. A newly introduced TERT promoter sequence can be engineered to have greater or lesser transcriptional activity, be responsive to new trans-acting transcriptional modulating agents, and the like.

Disruption of an endogenous TERT promoter sequence typically will decrease or abrogate ("knockout") the transcription of TERT. In one embodiment, the TERT promoter "knockout" is prepared by deletion or disruption by homologous recombination of the endogenous hTERT promoter. Homologous recombination and other means to alter (and "knockout") expression of endogenous sequences is described in Moynahan (1996) Hum. Mol. Genet. 5:875; Moynahan (1996) Hum. Mol. Genet. 5:875; Baudin (1993) Nucl. Acids Res. 21:3329: Wach (1994) Yeast 10:1793; Rothstein (1991) Methods Enzymol. 194:281; Anderson (1995) Methods Cell Biol. 48:31: Pettitt (1996) Development 122:4149-4157; Ramirez-Solis (1993) Methods Enzymol. 225:855; Thomas (1987) Cell 51:503; Couldrey (1998) Dev. Dyn. 212:284-292). Holzschu (1997) Transgenic Res 6:97-106; U.S. patents 5,464,764; 5,631,153; 5,487,992; 5,627,059, and 5,272,071; WO 91/09955; WO 93/09222; WO 96/29411; WO 95/31560; WO 91/12650. Vectors useful in TERT gene therapy can be viral or nonviral. They may comprise other regulatory or processing sequences. Lyddiatt (1998) Curr Opin Biotechnol 9:177-85.

The invention provides for delivery of the expression systems into cells or tissues in vitro or ex vivo. For ex vivo therapy, vectors may be introduced into cells taken from the patient and clonally propagated for autologous transplant back into the same patient (U.S. Patent Nos. 5,399,493 and 5;437,994. Cells that can be targeted for TERT promoter gene therapy aimed at increasing the telomerase activity of a target cell include, but are not limited to, embryonic stem or germ cells, particularly primate or human cells, hematopoietic stem cells (AIDS and post-chemotherapy), vascular endothelial cells (cardiac and cerebral vascular disease), skin fibroblasts and basal skin keratinocytes (wound healing and bums), chondrocytes (arthritis); brain astrocytes and microglial cells (Alzheimer's Disease), osteoblasts (osteoporosis), retinal cells (eye diseases), and pancreatic islet cells (Type I diabetes).

The exogenous sequence is typically inserted in a construct, usually also with a marker gene to aid in the detection of the knockout construct and/or a selection gene. The knockout construct is inserted in a cell, typically an embryonic stem (ES) cell, usually by homologous recombination. The resultant transformed cell can be a single gene knockout (one haplotype) or a double gene (homozygous) knockout. The knockout construct can be integrated into one or several locations in the cell's genome due to the random nature of homologous recombination events; however, the recombination does occur between regions of sequence complementarity. Typically, less than one to five percent of the ES cells that take up the knockout construct will actually integrate exogenous DNA in these regions of complementarity; thus, identification and selection of cells with the desired phenotype is usually necessary and a selection or marker sequence is usually incorporated into the construct for this purpose. Cells which have incorporated the construct are selected for prior to inserting the genetically manipulated cell into a developing embryo; for example, the cells are subjected to positive selection (using G418, for example, to select for neomycin-resistance) and negative selection (using, for example, FIAU to exclude cells lacking thymidine kinase). Selection and marker techniques include antibiotic resistance selection or β-galactosidase marker expression as described elsewhere in this disclosure.

After selection of manipulated cells with the desired phenotype, such as complete or partial inability to express endogenous TERT promoter, or, expression of the exogenous TERT promoter (as hTERT promoter activity) the cells are inserted into a mouse embryo. Insertion can be accomplished by a variety of techniques, such as microinjection, in which about 10 to 30 cells are collected into a micropipet and injected into embryos that are at the proper stage of development to integrate the ES cell into the developing embryonic blastocyst, at about the eight cell stage, which for mice is about 3.5 days after fertilization. The embryos are obtained by perfusing the uterus of pregnant females. After the ES cell has been introduced into the embryo, it is implanted into the uterus of a pseudopregnant foster mother, which is typically prepared by mating with vascectomized males of the same species. In mice, the optimal time to implant is about two to three days pseudopregnant. Offspring are screened for integration of the TERT nucleic acid sequences and the modified promoter activity phenotype. Offspring that have the desired phenotype are crossed to each other to generate a homozygous knockout. If it is unclear whether germline cells of the offspring have modified promoter, they can be crossed with a parental or other strain and the offspring screened for heterozygosity of the desired trait. The heterozygotes can be crossed with each other to produce mice homozygous for modified TERT genomic sequence. Bijvoet (1998) Hum. Mol. Genet. 7:53-62; Moreadith (1997) J. Mol. Med. 75:208-216; Tojo (1995) Cytotechnology 19:161-165; Mudgett (1995) Methods Mol. Biol. 48:167-184; Longo (1997) Transgenic Res. 6:321-328; U.S. Patents Nos. 5,616,491 (Mak, et al.); 5,464,764; 5,631,153; 5,487,992; 5,627,059; 5,272,071; and, WO 91/09955, WO 93/09222, WO 96/29411, WO 95/31560, and WO 91/12650. Thus, the invention provides for the use of the TERT promoter sequence-containing reagents of the invention to produce "knockout" mouse cells and animals, transgenic animals, and their progeny. These cells and animals can be further reconstituted with wild type or modified endogenous mTERT promoter or exogenous TERT promoter, such as hTERT.

The present invention further provides methods and reagents for karyotype analysis, gene amplification detection, or other chromosomal analysis using probes comprising the TERT promoter sequences of the invention. In various embodiments, amplifications (change in copy number), deletions, insertions, substitutions, or changes in the chromosomal location (translocations) of TERT promoter containing genes are detected. These can be correlated with the presence of a pathological condition or a predisposition to developing a pathological condition (such as cancer). Thus, this information can be used in a diagnostic or prognostic manner. For instance, a translocation event could indicate that activation of TERT expression occurs in some cases by replacing all or part of the TERT promoter with another promoter element that directs TERT transcription in an inappropriate manner. Furthermore, the methods and reagents of the invention can be used to inhibit this inappropriate TERT activation.

Determining the chromosomal location of TERT promoter sequence may also be useful for analysis of TERT gene repression in normal somatic cells, for instance, whether the location is part of non-expressing heterochromatin. Nuclease hypersensitivity assays for distinguishing heterochromatin and euchromatin are described in Wu (1979) Cell 16:797; Groudine (1982) Cell 30:131; Gross (1988) Ann. Rev. Biochem. 57:159. Methods for analyzing karyotype are discussed in Pinkel (1988) Proc. Natl. Acad. Sci. USA 85:9138; EPO Pub. No. 430,402: Choo. ed., Methods In Molecular Biology Vol. 33: In Situ Hybridization Protocols, Humana Press, Totowa, New Jersey, 1994; Kallioniemi (1992) Science 258:818).

### TERT promoter binding proteins and transcriptional regulatory factors

In addition to the novel TERT promoter sequences and identification of the cis-acting transcriptional regulatory sequences contained therein, the invention provides for novel in vitro and cell-based in vivo assay systems to screen for TERT promoter binding proteins (trans-acting transcriptional regulatory factors) using the nucleic acids of the invention. Many assays are available that screen for nucleic acid binding proteins and all can be adapted and used with the novel TERT sequences provided by the invention.

One embodiment of the invention provides a method of screening and isolating a TERT promoter binding compound by contacting a TERT promoter sequence of the invention (particularly, an identified cis-acting regulatory sequence) with a test compound and measuring the ability of the test compound to bind the selected nucleic acid. The test compound, can be any agent capable of specifically binding to a TERT promoter activity, including compounds available in combinatorial libraries, a cell extract, a nuclear extract, a protein or peptide. If a TERT transcriptional activating protein is the goal of the search, a cell with telomerase activity is typically chosen.

Various techniques can be used to identify polypeptides which specifically bind to TERT promoter; for example, mobility shift DNA-binding assays, methylation and uracil interference assays, DNase and hydroxyl radical footprinting analysis, fluorescence polarization, and UV crosslinking or chemical cross-linkers. For a general overview, see Ausubel (chapter 12, DNA-Protein Interactions). One technique for isolating co-associating proteins, including nucleic acid and DNA/RNA binding proteins, includes use of UV crosslinking or chemical cross-linkers, including cleavable cross-linkers dithiobis (succinimidylpropionate) and 3,3'-dithiobis (sulfosuccinimidyl-propionate). McLaughlin (1996) Am. J. Hum. Genet. 59:561-569: Tang (1996) Biochemistry 35:8216-8225; Lingner (1996) Proc. Natl. Acad. Sci. USA 93:10712; Chodosh (1986) Mol. Cell. Biol 6:4723-4733. In many cases, there is a high likelihood that a specific protein (or a related protein) may bind to an hTERT promoter sequence, such as a Myc, NF-kappa B, EF2, Sp1, AP-1 or CAAT box binding site. In these scenarios, where an antibody may already be available or one can be easily generated, co-immunoprecipitation analysis can be used to identify and isolate TERT promoter-binding, trans-acting factors. The trans-acting factor can be characterized by peptide sequence analysis. Once identified, the function of the protein can be confirmed, for example, by competition experiments, factor depletion experiments using an antibody specific for the factor, or by competition with a mutant factor.

Alternatively, TERT promoter-affinity columns can be generated to screen for potential TERT binding proteins. In a variation of this assay, TERT promoter subsequences are biotinylated, reacted with a solution suspected of containing a binding protein, and then reacted with a strepavidin affinity column to isolate the nucleic acid or binding protein complex (Grabowski (1986) Science 233:1294-1299; Chodosh (1986) supra). The promoter-binding protein can then be conventionally eluted and isolated. Mobility shift DNA-protein binding assay using nondenaturing polyacrylamide gel electrophoresis (PAGE) is an extremely rapid and sensitive method for detecting specific polypeptide binding to DNA (Chodosh (1986) supra, Carthew (1985) Cell 43:439-448; Trejo (1997) J. Biol. Chem. 272:27411-27421; Bayliss (1997) Nucleic Acids Res. 25:3984-3990).

Interference assays and DNase and hydroxyl radical footprinting can be used to identify specific residues in the nucleic acid protein -binding site. Bi (1997) J. Biol. Chem. 272:26562-26572; Karaoglu (1991) Nucleic Acids Res. 19:5293-5300. Fluorescence polarization is a powerful technique for characterizing macromolecular associations and can provide equilibrium determinations of protein-DNA and protein-protein interactions. This technique is particularly useful (and better suited than electrophoretic methods) to study low affinity protein-protein interactions. Lundblad (1996) Mol. Endocrinol. 10:607-612.

Proteins identified by these techniques can be further separated on the basis of their size, net surface charge, hydrophobicity and affinity for ligands. In addition, antibodies raised against such proteins can be conjugated to column matrices and the proteins immunopurified according to well known methods. Scopes, R. K., Protein Purification: Principles and Practice, 2nd ed., Springer Verlag, (1987).

Transcriptional regulatory sequences identified by comparison of hTERT and mTERT sequences include the for trans-acting factors c-Myc, SP1, SRY, HNF-3β, HNF-5, TFIID-MBP, E2F and c-Myb. Table 1 shows other transcriptional regulatory sequences that have been identified upstream from the TERT encoding region by comparison of the hTERT sequence with known regulatory motifs. These elements are of interest in regulating transcription in the cell types where the factors that bind to these elements are present.

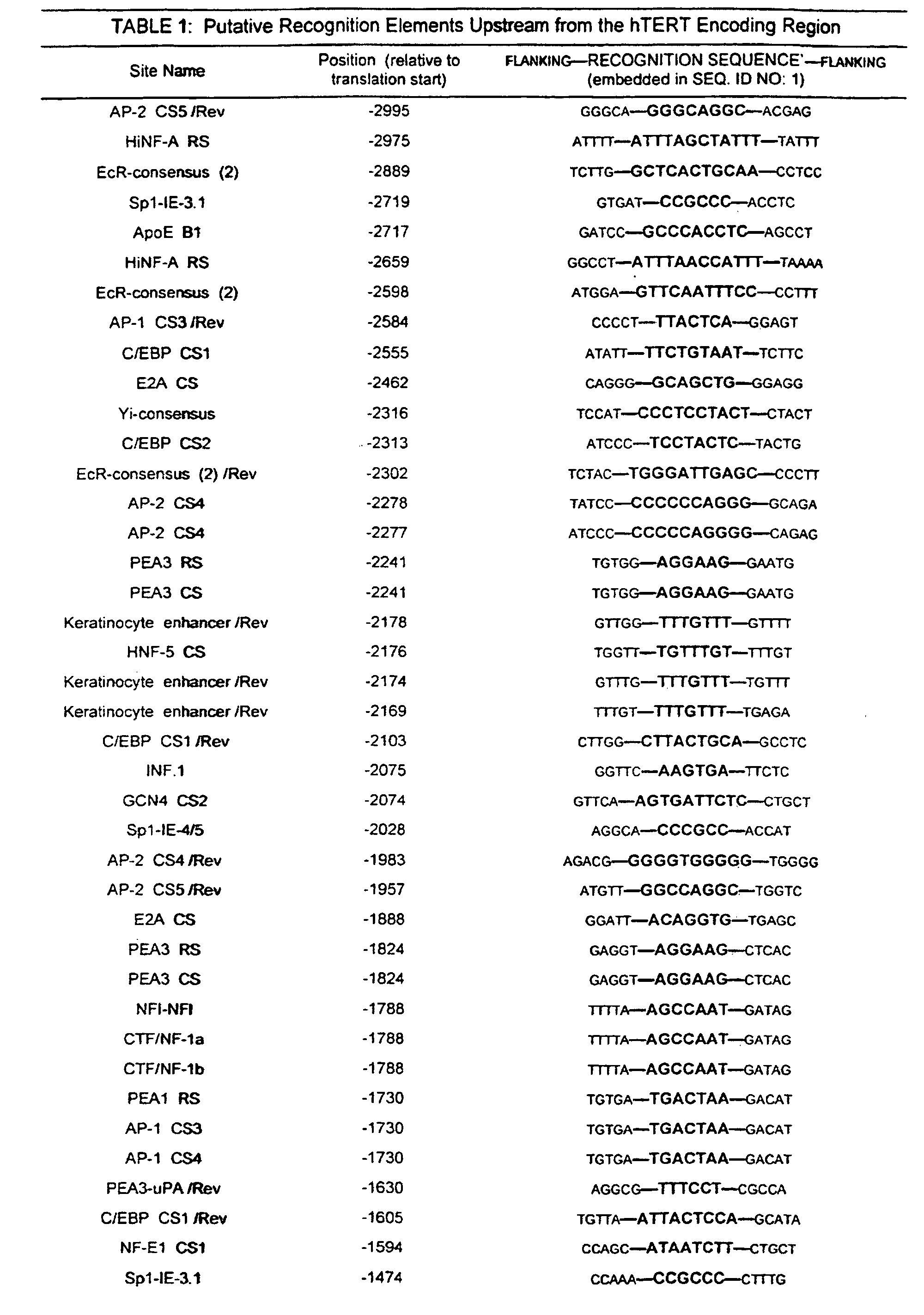

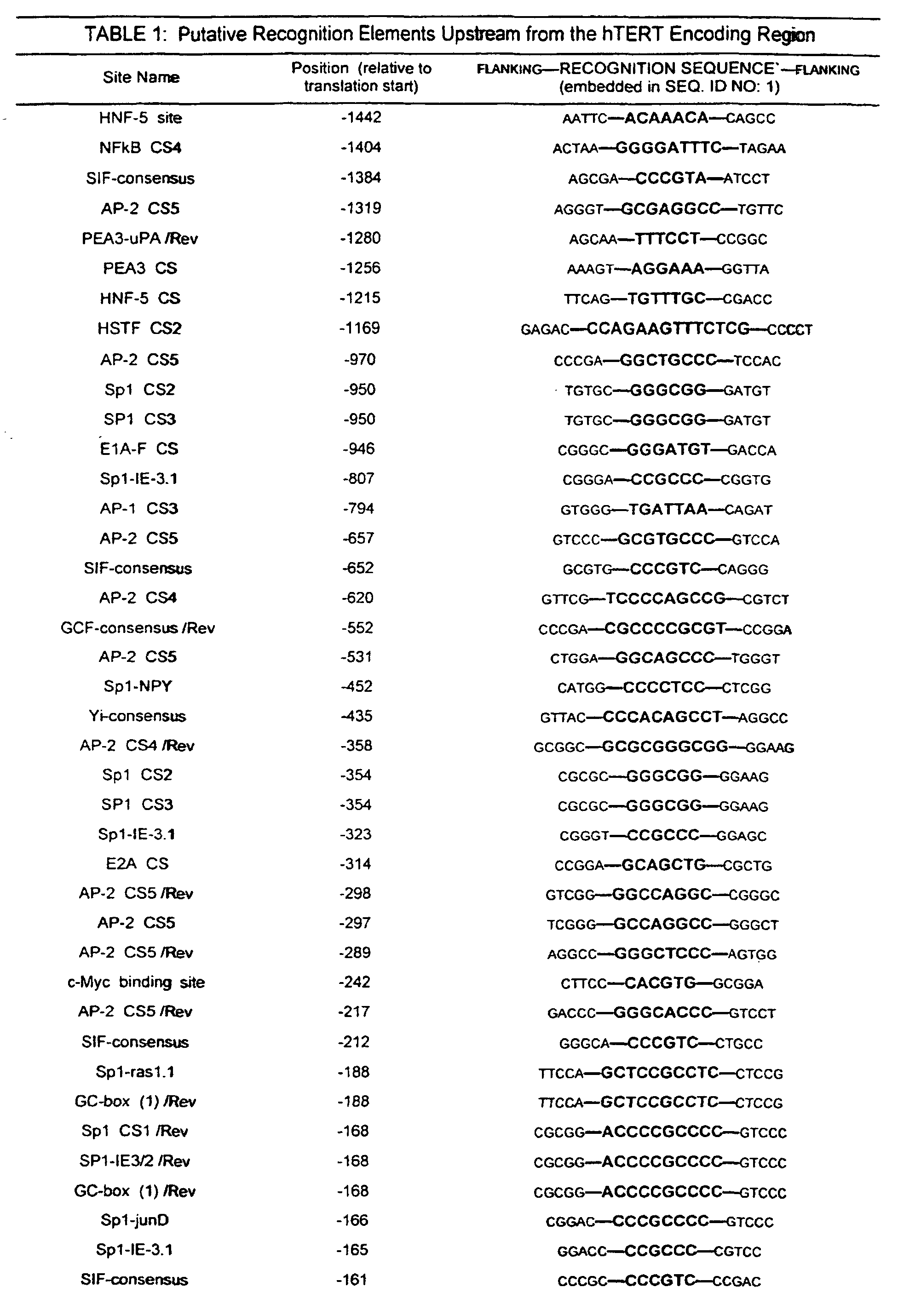

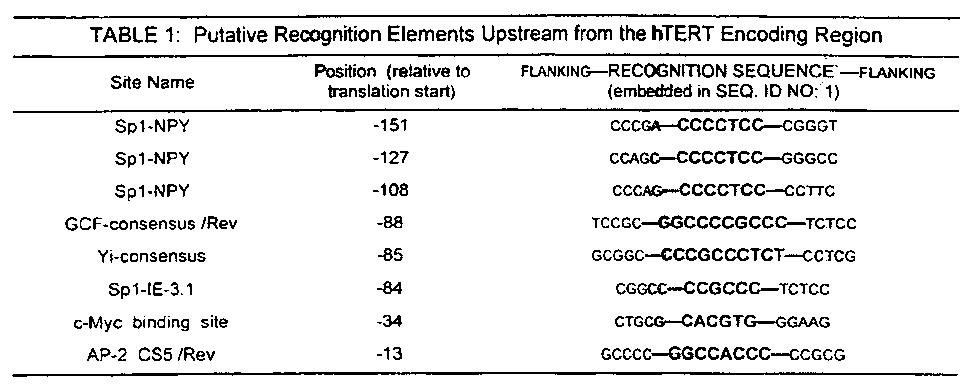

The examples and detailed elaboration provided in this disclosure are for illustrative purposes, and are not intended to limit the invention. Modifications can be made by those skilled in the art that are included within the spirit of this application and scope of the appended claims.

### EXAMPLES

### Example 1: Cloning of λGϕ5 and characterization of hTERT genomic sequences

The following example details the cloning of the human hTERT promoter.

A human genomic DNA library was screened by PCR and hybridization to identify a genomic clone containing hTERT RNA coding sequences. The library was a human fibroblast genomic library made using DNA from WI38 lung fibroblast cells (Stratagene, Cat # 946204). In this fibroblast library, partial Sau3AI fragments were ligated into the Xhol site of a commercial phage cloning vector, Lambda FIX®. Vector (Stratagene, San Diego, CA), with insert sizes ranging from approximately 9 kilobases (kb) to 22 kb.

The genomic library was divided into pools of 150,000 phage each. Each pool screened by nested PCR, with the outer primer pair TCP1.52 & TCP1.57; inner pair TCP1.49 & TCP1.50. These primer pairs span a putative intron in the genomic DNA of hTERT and ensured the PCR product was derived from a genomic source and not from contamination by the hTERT cDNA clone. Positive pools were further subdivided until a pool of 2000 phage was obtained. This pool was plated at low density and screened via hybridization with a DNA fragment encompassing a subset of hTERT cDNA, generated by restriction digest with SphI and EcoRV.

Two positive clones were isolated and rescreened via nested PCR. At rescreening, both clones were positive by PCR. One of the lambda phage clones (designated "Gphi5" or "λGϕ5") was digested with NotI, revealing an insert size of approximately 20 kb. Subsequent mapping indicated the insert size was 15 kb and that phage λGϕ5 contains approximately 13 kb of DNA upstream from the transcriptional start site (upstream from the cDNA sequence).

**Figure 1** shows the structure of Phage λGϕ5, mapped by restriction enzyme digestion and DNA sequencing.

### Isolating, Subcloning and Sequencing the Genomic hTERT Insert

The phage DNA was digested with Ncol. This fragment was cloned into the plasmid pBBS167. The resulting subclones were screened by PCR to identify those containing sequences corresponding to the 5' region of the hTERT cDNA. A subclone (plasmid "pGRN140") containing a 9 kb NcoI fragment (with hTERT gene sequence and about 4 to 5 kb of lambda vector sequence) was partially sequenced to determine the orientation of the insert. pGRN140 was digested using Sall to remove lambda vector sequences, the resulting plasmid (with removed lambda sequences) designated pGRN144. The pGRN144 insert was then sequenced.

A Notl fragment from λGϕ5 (containing the complete approximately 15 kbp genomic insert including the hTERT gene promoter region) was inserted in the NotI site of plasmid pBBS185. Two plasmids were isolated with their respective inserts oriented in opposite directions. One resulted in the insert oriented with the hTERT open reading frame (ORF) in the same orientation as the plasmid's Lac promoter, designated pGRN 142; the second, pGRN 143.

SEQ. ID NO:1 is a listing of the sequence data obtained from plasmid pGRN 142. Nucleotides 1-43 and 15376-15418 are plasmid sequence. Thus, the genomic insert begins at residue 44 and ends at residue 15375. The beginning of the cloned cDNA fragment corresponds to residue 13490. There are Alu sequence elements located ∼1700 base pairs upstream. The sequence of the hTERT insert of pGRN 142 can now be obtained from GenBank (http://www.ncbi.nlm.nih.gov/) under Accession PGRN142.INS AF121948.

Numbering of hTERT residues for plasmids in the following examples begins from the translation initiation codon, according to standard practice in the field. The hTERT ATG codon (the translation initiation site) begins at residue 13545 of SEQ. ID NO:1. Thus, position -1, the first upstream residue, corresponds to nucleotide 13544 in SEQ. ID NO:1.

### Example 2: TERT Promoter-Driven Reporter Constructs

This example describes the construction of plasmids in which reporter genes are operably linked to hTERT promoter sequences of the invention. This also illustrates how the TERT promoter sequence of the invention can analogously be operatively linked to heterologous sequences, such as polypeptide coding sequences, for expression in cells and tissues in vitro and in vivo and transgenic animals. As will be evident to one skilled in the art, techniques such as those illustrated in these examples can be used to test other candidate sequences for ability to specifically promote transcription in cells expressing TERT.

hTERT-linked reporter vectors of the invention have numerous uses, including identification of specific cis-acting sequences and trans-acting transcriptional regulatory factors. Importantly, these hTERT-containing reporter constructs can be used for the screening of agents capable of modulating (i.e., activating or inhibiting) hTERT transcription. These studies can be conducted in vitro and in vivo.

A number of reporter genes, such as firefly luciferase, β-glucuronidase, β-galactosidase, chloramphenicol acetyl transferase, and GFP are known and can be operably linked to hTERT promoter. In this example, the human secreted alkaline phosphatase (SEAP; ClonTech) was used. The SEAP reporter gene encodes a truncated form of the placental enzyme which lacks the membrane anchoring domain, thereby allowing the protein to be secreted efficiently from transfected cells. Levels of SEAP activity detected in the culture medium have been shown to be directly proportional to changes in intracellular concentrations of SEAP mRNA and protein. The chemiluminescence-based SEAP assay is about 10-fold more sensitive than similar assays using firefly luciferase as the reporter enzyme. The SEAP activity can also be assayed with a fluorescent substrate, which provides sensitivity comparable to luciferase. Berger (1988) Gene 66:1; Cullen (1992) Meth. Enzymol. 216:362; Yang (1997) Biotechniques 23:1110-1114.

### hTERT 5' Upstream and Intron Sequences have "Promoter" Activity

Experiments with reporter constructs comprising various hTERT sequences of the invention identified cis-acting regions with "promoter" transcriptional activating activity in both 5' upstream and intron sequences. In brief, four constructs, pGRN148, pGRN150, "pSEAP2 basic" (no promoter sequences = negative control), and "pSEAP2 control" (contains the SV40 early promoter and enhancer) were constructed and transfected in triplicate into mortal and immortal cells.

**Figure 2** shows the plan for construction of plasmid pGRN148. Briefly, a BgI2-Eco47III fragment from pGRN144 (described above) was digested and cloned into the BgIII-NruI site of pSeap2Basic (ClonTech, San Diego, CA). A second reporter-promoter, plasmid pGRN150 was made by inserting the BgIII-FspI fragment from pGRN144 into the BgIII-NruI sites of pSEAP2. Plasmid pGRN173 was constructed by using the EcoRV-Stul fragment from pGRN144. This makes a promoter reporter plasmid that contains the promoter region of hTERT from approximately 2.5 kb upstream from the start of the hTERT ORF to just after the first intron within the coding region. The initiating Met was mutated to Leu, so that the second ATG following the promoter region would be the initiating ATG of the SEAP ORF.

Use of the intron sequence allows identification of regulatory sequences that may be present in the intron (the invention provides transcriptional regulatory sequences from any portion of the hTERT genomic sequence). In addition to the hTERT derived pSEAP reporter constructs, a positive control vector and a negative control vector were used. The negative control (pSEAP2-Basic) is necessary to determine the background signal associated with the DNA backbone of the vector. A positive control is necessary to confirm transfection and expression of exogenous DNA and to verify the presence of active SEAP in the culture media. The positive control is the pSEAP2-Control vector (ClonTech) which contains the SEAP structural gene under transcriptional control of the SV40 promoter and enhancer.

Three constructs, the control, pGRN148 (which include hTERT 5' promoter sequences) and pGRN150, were transfected into a mortal cell line, BJ cells, a human foreskin fibroblast line, Feng (1995) Science 269:1236; and an immortal cell line, the human embryonic kidney line 293; Graham (1977) J. Gen. Virol. 36:59. All transfections were done in parallel with the two control plasmids.

In immortal cells, pGRN148 and pGRN150 constructs appear to drive SEAP expression as efficiently as the pSEAP2 positive control (containing the SV40 early promoter and enhancer). In contrast, in mortal cells only the pSEAP2 control gave detectable activity. Similar results were obtained using another normal cell line (RPE, or retinal pigmental epithelial cells; Aronson (1983) In vitro 19:642-650). In RPE cells transfected with pGRN150, the hTERT promoter region was inactive while the pSEAP2 control plasmid was active. These results indicate that, as expected, hTERT promoter sequences are active in tumor cells but not in mortal cells.

### Identification of the Tissue Specificity Elements of the hTERT Promoter

The hTERT DNA promoter sequences were cloned into the pSEAP2-Basic transcription reporter vector (ClonTech) to generate the plasmids pGRN 148, 150, 175, 176, 181,184, 261, 262, and 319. Summarized below are details of the promoter plasmid construction (nucleotide numbers refer to the number of nucleotides upstream of the translation initiation site at 13545 of SEQ ID NO:1):
pEGFP-1. "Vector from ClonTech containing the Enhanced Green Fluorescent Protein.
pGRN140. *NCO1 fragment containing hTERT upstream sequences and the first intron of hTERT from λGϕ5 into the NCO1 site of a pBBS167 (variant of pUC19 cloning vector with MCS, e.g. ATGACCATGATTACGAATTCGAGCTCGGTACCCGGGGATCCTCTAGAGTCGACCTGCAGGCATGCCCATG GCAGGCCTCGCGCGCGAGATCTCGGGCCCAATCGATGCCGCGGCGATATCGCTCGAGGAAGCTTGGCA CTGGCC (SEQ ID NO:3), and a chloramphenicol sensitive gene between the F 1 ori and the Amp gene in the opposite orientation from the Amp gene). The fragment is oriented so that the hTERT sequences are in the same direction as the Lac promoter.
pGRN144. described above; Sail deletion of pGRN140 to remove phage (lambda) sequences.
pGRN148: *BGL2-ECO47III fragment from pGRN144 containing hTERT upstream sequences (from position -51 to -2482) into the BGL2-NRU1 sites of pSEAP2-Basic to make a hTERT promoter/reporter plasmid.
pGRN150: *BGL2-FSP1 fragment from pGRN144 containing 2447nt of hTERT upstream sequences (from position -36 to -2482) into the BGL2-NRU1 sites of pSEAP2 to make a hTERT promoter/reporter plasmid.
pGRN175: *APA1(Klenow blunt)-SRF1 religation of pGRN150 to delete most of the hTERT upstream sequences. This makes a promoter/reporter plasmid that uses 82 nucleotides of hTERT upstream sequences (from position -36 to -117).
pGRN176: *PML1-SRF1 religation of pGRN150 to delete most of the hTERT upstream sequences. This makes a promoter/reporter plasmid that uses 204 nucleotides of hTERT upstream sequences (from position -36 to -239).
pGRN181: *APA1 digestion and religation of pGRN150 to delete all APA1 sites but one. This makes a promoter/reporter plasmid that comprises from -36 to -114 and -1076 to -2482 of the hTERT upstream sequences.
pGRN184: *XBA1(partial, Klenow fill)-ECOR1 digest and religation of pGRN150 to make a deletion of the hTERT promoter sequences. This makes a promoter/reporter plasmid that expresses a region from -1391 to -2484 of the hTERT upstream sequences.
pGRN213. *FSP1 fragment containing the CatS gene and the F1 ORI plus part of the AmpR gene into the FSP1 sites of pSEAP2-Basic such that the orientation reconstructs the AmpR gene.
pGRN244: *SAL1-NOT1 fragment from pSEAP2-Basic containing the SEAP region into the SAL1-NOT1 sites of pEGFP-1. This modification adds a selectable marker to the vector.
pGRN245: *SAL1-NOT1 fragment from pGRN176 containing the hTERT-promoter/SEAP region into the SAL1-NOT1 sites of pEGFP-1. This modification adds a dominant selectable marker to the vector.
pGRN246: *SAL1-NOT1 fragment from pGRN176 containing the hTERT-promoter/SEAP region into the SAL1-NOT1 sites of pEGFP-1. This modification adds a dominant selectable marker to the vector.
pGRN248 *SAL1-NOT1 fragment from pGRN175 containing the hTERT promoter/SEAP region into the Sall-Notl sites of pEGFP-1. This modification adds a dominant selectable marker to the vector.
pGRN259. *in vitro mutagenesis using RA94 (CCCGGCCACCCCCGCGAattCGCGCGCTCCCCG CTGC) (SEQ ID NO:4) to introduce an EcoRl site at the initiating met of hTERT in pGRN144. This provides hTERT sequences from +1 to -2482 that can be cloned into a vector using EcoRI and BgIII.
pGRN260. *in vitro mutagenesis using RA91 (TTGTACTGAGAGTGCACCATATGCGGTGTGcatgc TACGTAAGAGGTTCCAACTTTCACCATAAT) (SEQ ID NO:5) to delete several sites from the Chloramphenicol region of pGRN213 to create a variant, more useful, MCS. This creates a Mutagenesis version of pSEAP2-Basic with more unique cloning sites in its MCS.
pGRN261: *BGL2-ECOR1 fragment from pGRN259 containing hTERT upstream sequences into the BGL2-ECOR1 sites of pSEAP2-Basic. This makes a promoter/reporter expression plasmid that contains from +1 to -2482 of the hTERT upstream sequences.
pGRN262: *BGL2-ECOR1 fragment from pGRN259 containing hTERT upstream sequences into the BGL2-ECOR1 sites of pGRN260. This makes a promoter/reporter expression and mutagenesis plasmid that contains from +1 to -2482 of the hTERT upstream sequences.
pGRN294. *BbsI-Xhol fragment from pGRN142 containing hTERT upstream sequences from -1667 to - 3278 into the Bbsl-Xhol sites of pGRN259. This makes a vector containing the genomic upstream region for hTERT from +1 to -3278 that can be cloned with EcoRl and Xhol.
pGRN295: *ECOR1-XHO1 fragment from pGRN294 containing from +1 to -3282 of hTERT upstream sequences into the ECOR1-XHO1 sites of pGRN260. This makes a SEAP promoter/reporter/mutagenesis plasmid.
pGRN296: *ECOR1-XHO1 fragment from pGRN294 containing from +1 to -3282 of the hTERT upstream sequences into the ECOR1-XHO1 sites of pSEAP2-Basic. This makes a SEAP promoter/reporter plasmid.
pGRN297. *RA96 (AATTGCGAAGCTTACG) (SEQ ID NO:6) and RA97 (AATTCGTAAGCTTCGC) (SEQ ID NO:7) annealed to make an oligo linker into the ECOR1 sites of pGRN259 replacing the ECOR1 fragment of the intron-exon region of pGRN259.
pGRN299: *XHO1-HIND3 fragment from pGRN298 containing from +1 to -3282 of the hTERT upstream sequences into the XHO1-HIND3 sites of pGL2-Basic. This makes a Luciferase promoter/reporter plasmid with about 3.3Kb of hTERT promoter sequences.
pGRN300: *XHO1-SAC1 fragment from pGRN142 containing hTERT upstream sequences into the XHO1-SAC1 sites of pGRN299 such that the resulting construct contains from +1 to -5124 of the hTERT upstream sequences. This creates an hTERT promoter/reporter construct using Luciferase as a reporter.
pGRN310: *SAC1 fragment from pGRN142 containing hTERT upstream sequences into the SAC1 site of pGRN300 such that the resulting construct contains +1 to -7984 of the hTERT upstream sequences. This creates an hTERT promoter/reporter construct using Luciferase as a reporter.
pGRN311. *SPE1 fragment from pGRN142 containing from -4773 to -13501 of the hTERT upstream sequences into the SPE1 site of pGRN300 such that the orientation reconstructs the genomic region. This makes a Luciferase promoter reporter plasmid that contains the entire pGRN142 upstream genomic region of hTERT plus a 365bp region of genomic DNA from the middle of the 13.5Kb genomic region repeated upstream of the T7 promoter.
pGRN312: *BGL2-FSP1 fragment from pGRN144 into the BGL2-HIND3 (Klenow filled) sites of pGL2-Basic. This makes a Luciferase promoter/reporter version of pGRN150.
pGRN313: *KPN1-NOT1 digested pGRN311 blunted with T4 polymerase and religated. This makes a Luciferase promoter/reporter plasmid using from +1 to -13501 of the hTERT upstream sequences.
pGRN316: *oligo RA101 (5'- TAGGTACCGAGCTCTTACGCGTGC TAGCCCCACGTGGCGGA GGGACTGGGGACCCGGGCA-3') (SEQ ID NO:8) used for in vitro mutagenesis to delete the genomic sequence from pGRN262 between the SRF1 site and the first PML1 site. This makes a promoter- reporter plasmid containing hTERT upstream sequences from +1 to -239.
pGRN317: *oligo RA100 (5'-TAGGTACCGAGCTCTTACGCGTGCTAGCCCCTCGCTGGCGTCCCT GCACCCTGGGAGCGC-3') (SEQ ID NO:9) used for in vitro mutagenesis to delete the genomic sequence from pGRN262 between the SRF1 site and next to the last APA1 site. This makes a promoter -reporter plasmid containing hTERT upstream sequences from +1 to -397.
pGRN319: *RA107 (5'-CGTCCTGCTGCGCACtcaGGAAGCCCTGGCCCC-3') (SEQ ID NO:10) used for in vitro mutagenesis to inactivate the 'B' class E-box just proximal to the hTERT initiating met in pGRN262. This changes the CACGTG (SEQ ID NO:11) to CACTCA (SEQ ID NO:12). Also COD1941 (5'-GATGAATGCTCATGATTCCGTATGGCA-3') (SEQ ID NO:13) was used to switch from CatR to CatS introducing a BSPH1 site and COD2866 (5'-CAGCATCTTTTACTTTCACCAGCGTTTCTGGGTGCGCAAAA ACAGGAAGGCAAAATGCC-3') (SEQ ID NO:14) was used to select from AmpS to AmpR introducing an FSP1 site. In summary, pGRN319 carries a mutation in the E-box.
pGRN350: *RA104 (5'- TAGGTACCGAGCTCTTACGCGTGCTAGCCCCTCCCAGCCCCTC CCCT TCCTTTCCGCGGC-3') (SEQ ID NO:15) used for in vitro mutagenesis to delete the genomic sequence from pGRN262 between the SRF1 site and the last APA1 site before the ATG of the hTERT open reading frame (orf). This makes a promoter- reporter plasmid containing hTERT upstream sequences from +1 to -117.
pGRN351: *SAC2 fragment from pGRN319 into the SAC2 sites of pGRN350 such that the SEAP orf is recreated. This makes a "deactivated E-box" version of pGRN350.
pGRN352: *RA122 (5'- GACCGCGCTTCCCACtcaGCGGAG GGACTGGGG-3') (SEQ ID NO:16) used for in vitro mutagenesis to "deactivate" the penultimate class "B" E-box before the translation start site of hTERT.

The pSEAP2-Basic plasmid lacks eukaryotic promoter and enhancer sequences. This vector contains the SV40 late polyadenylation signal inserted downstream of the SEAP coding sequences to ensure proper and efficient processing of the transcript in eukaryotic cells. It also contains a synthetic transcription blocker (TB), composed of adjacent polyadenylation and transcription pause sites to reduce background transcription. As noted above, the SEAP reporter gene encodes a truncated form of the placental enzyme which lacks the membrane anchoring domain, thereby allowing the protein to be efficiently secreted from transfected cells.

Levels of SEAP activity detected in the culture medium have been shown to be directly proportional to changes in intracellular concentrations of SEAP mRNA. The chemiluminescent SEAP substrate CSPDTM (ClonTech) was used to detect secreted SEAP. Use of this substrate enables monitoring of the expression of the SEAP reporter gene through simple, sensitive, non-radioactive assays of secreted phosphatase activity. This chemiluminescent assay can detect as little as 10-13 g of SEAP protein. The assay is linear over a 104 fold range of enzyme concentrations. This makes the assay (and these vectors) particularly well-suited for comparative analyses.

In addition to the hTERT derived pSEAP reporter constructs, a positive control vector (pSEAP2-Control vector) and a negative control vector (pSEAP2-Basic) were used. The promoter constructs (pGRN 150, 175,176) and the control vectors were transfected into immortal (HEK 293) and mortal (BJ fibroblast, RPE, HUVEC) cells 48-72 hours after transfection. The culture media was collected and assayed for, SEAP activity. The SEAP activity was detected using the chemiluminescent assay from CLONTECH, Great EscAPeTM SEAP Chemiluminescence Kit, according to the manufacturer's protocol. The transfections were performed in triplicate. The culture media from each transfection was collected after 48-72 hours and assayed in triplicate. The background values obtained by transfection of the negative control (pSEAP2-Basic) vector was subtracted from the values obtained with the test constructs. The average of nine measurements was used and plotted for each of the constructs.

### Experimental Results in Immortal and Mortal Cell Lines

The results of the assays show that while the hTERT promoter constructs are capable of driving the expression of the reporter SEAP gene in immortal cells, the same constructs are silent in all mortal cells tested. The pSEAP2-Control vector however is active in all cell types regardless of their mortal or immortal status and the pSEAP2-Basic vector is silent in all cells assayed.

### hTERT Promoter Driving Thymidine Kinase Expression In vitro

The invention provides constructs comprising heterotogous coding sequences operably linked to hTERT promoter sequences. In one embodiment, hTERT coding sequences are operably linked to Herpes simplex virus thymidine kinase ("HSV-TK") coding sequences. HSV-TK is an enzyme that is capable of converting innocuous prodrugs, e.g. ganciclovir, into toxic metabolites that interfere with the cellular replication of proliferating cells (such as cancer cells, which have active hTERT promoter activity). Controlling thymidine kinase (TK) expression by subordinating it to the hTERT promoter restricts TK expression to cells where the hTERT promoter is normally active. This prevents TK expression in "normal" cells, where the hTERT promoter is usually silent.

The ability of the hTERT promoter to specifically drive the expression of the TK gene in tumor cells was tested using a variety of constructs: One construct, designated pGRN266, contains an EcoRI-FseI PCR fragment with the TK gene cloned into the EcoRI-FseI sites of pGRN263. pGRN263, containing approximately 2.5 kb of hTERT promoter sequence, is similar to pGRN150, but contains a neomycin gene as selection marker. pGRN267 contains an EcoRI-FseI PCR fragment with the TK gene cloned into the EcoRI-FseI sites of pGRN264. pGRN264, containing approximately 210 bp of hTERT promoter sequence, is similar to PGRN176, but contains a neomycin gene as selection marker. pGRN268 contains an EcoRI-XbaI PCR fragment with the TK gene cloned into the EcoRI-XbaI (unmethylated) sites of pGRN265. pGRN265, containing approximately 90 bp of hTERT promoter sequence, is similar to pGRN175, but contains a neomycin gene as selection marker.

These hTERT promoter/TK constructs, pGRN266, pGRN267 and pGRN268, were re-introduced into mammalian cells and TK/+ stable clones (and/or mass populations) were selected. Ganciclovir treatment in vitro of the TK/+ cells resulted in selective destruction of all tumor lines tested, including 143B; 293, HT1080, Bxpc-3, DAOY and NIH3T3. Significantly, ganciclovir treatment had no effect on normal BJ cells. This clearly demonstrates the tumor-specificity of all three hTERT promoter fragments used in these experiments.

### Example 3: Direct In vivo hTERT Promoter Suicide Gene Therapy

The invention provides reagents and methods for treating diseases involving unwanted cell proliferation by in vivo gene therapy. To demonstrate the efficacy of this aspect of the invention, the reagents of the invention were used to treat cancer (of human origin) in an art-accepted animal model. A human cancer cell, the osteosarcoma cell line 143B, which normally expresses the telomerase gene, was transfected with a plasmid containing the TK gene driven by the hTERT promoter.

Specifically, sequences -36 to -2482 upstream of the translation start site of SEQ ID NO:1 were used to drive the TK gene. The plasmid also contained the neomycin phosphotransferase gene. After transfection of cells with the plasmid, G418 resistant clones expressing TK were selected. Two hundred thousand of the parental or TK expressing 143B cells were injected subcutaneously in the flank of Balb/c nude (nu/nu) mice to establish tumors. Four to 11 days after tumor implantation the mice were injected IP with 75 mg/kg ganciclovir (GCV) or saline twice daily. Tumor growth was monitored every 3-4 days. When GCV was administered either at 4 or at 11 days post tumor implantation to these tumor bearing animals, TK mediated cell lysis and retarded tumor growth was observed. Such inhibition of tumor cell growth is not observed when saline is administered or if the parental 143B tumor (143BP) is treated with either saline or GCV. Forty-five days after tumor implantation, only the animals implanted with the TK+ 143B clone and treated with GCV showed 100% survival. In the other groups all but one animal died from massive tumor burden.

These data indicate that the hTERT promoter is sufficient to drive TK gene expression both in vivo. It also shows that the reagents and methods of the invention can be used to promote tumor regression in vivo in subjects (including humans) carrying pre-established tumors.

### Example 4: Oncolytic Viruses Under Control of the hTERT Promoter

As discussed earlier the invention provides "conditionally replicating" oncolytic virus constructs in which hTERT promoter sequences of the invention are operably linked to essential virally encoded genes. Use of hTERT promoter sequences of the invention ensures the virus will only be productively expressed in cells with telomerase activity. Thus, constructs can be used therapeutically to lyse only cells that express telomerase, such as immortal or cancer cells. Proliferation of the virus and its cytopathic effects is thus restricted to tumor cells. Details of the construction of an exemplary hTERT promoter driven, conditionally replicating oncolytic virus follows. In this embodiment, the hTERT promoter replaces the normal E1a promoter to create a virus which will only replicate in telomerase expressing cells.

Plasmid pBR/ITR/549-Clal containing nucleotides 1-356 (Ad2 ITR and packaging signals) and 549-920 (a portion of the E1a coding sequence) of Adenovirus 2 (Ad2) linked using a polylinker was built using standard molecular biology procedures in the bacterial plasmid pBR322. In pBR/ITR/TB+phTERT176-E1A and pBR/ITR/TB+phTERT316-E1A, the normal E1a promoter (Ad2 357-548) has been replaced with the hTERT promoter. Ad2 sequences from 916-10680 are added to these plasmids to recreate the expression elements of the 5' end of the virus.

These plasmids (pBR/ITR/TB+phTERT176-10680 and pBR/ITR/TB+phTERT316-10680) are transfected into a telomerase expressing human cell line along with an adenoviral DNA fragment containing Ad2 sequences 10681- 35937. Recombinant plaques are scored and selected 7-21 days post transduction. The hTERT promoter E1a containing Ad2 is propagated and produced for use employing standard schemes for recombinant Ad2 amplification and manufacturing. (Graham and Prevec, 1991, in Methods in Molecular Biology, Chapter 11, Ed E.J. Murray, The Human Press Inc., Clifton, NJ.; Kanegae et al., Jpn J Med Sci Biol, 1994, 47(3):157-66). Because the E1a gene is driven by the hTERT promoter, which is not normally expressed by most somatic cells, recombinant Ad2 genome will only replicate and be packaged into virus particles in cells expressing telomerase.

### Example 5: hTERT Promoter Sequences Driving an Alkaline Phosphatase Reporter Gene for High Throughput Screening.

The invention provides constructs and promoter-based assays to identify small molecule activators and/or repressors of hTERT and telomerase activity. To this end, fragments of the hTERT promoter were cloned into plasmids expressing a secreted form of alkaline phosphatase and a selection marker. The SEAP constructs (pGRN244, pGRN245, pGRN246 and pGRN248) were re-introduced into normal human cells and into immortal cell lines. After selection of stable clones having integrated the hTERT promoter/SEAP constructs, RT-PCR was used to determine the levels of SEAP mRNAs. In 293 cells, the levels of SEAP mRNA were elevated and comparable to the levels of endogenous hTERT, whereas in BJ cells, the levels of SEAP mRNA were virtually undetectable and closely matched the levels of the endogenous hTERT in these cells.

These results indicate that hTERT promoter/SEAP constructs can be used to engineer cells suitable for promoter-based assays and to screen for chemical and/or biological activators and/or repressors of telomerase in normal and tumor cells. pGRN244, pGRN245, pGRN246 and pGRN248 were re-introduced into BJ and 293 cells. SEAP activity and mRNA levels were determined in these cells as criteria for clone selection. Several 293 and BJ lines were selected and two BJ/pGRN245 clones were expanded for high throughput screening. These constructs were also introduced into IDH4 cells, which are immortal lung fibroblasts that express the SV40 large T antigen under the control of the dexamethasone-inducible MMTV promoter. IDH4 cells are telomerase positive and proliferate in the presence of dexamethasone. However, these cells can be induced into a senescent, telomerase negative stage after dexamethasone removal. Upon re-addition of dexamethasone, the cells return to an immortal phenotype and re-activate telomerase.

pGRN244, pGRN245, pGRN246 and pGRN248 were transfected into IDH4 cells. SEAP activity was shown to parallel telomerase activity in the different clones, whereas no significant fluctuation of SEAP activity was observed with the control plasmid. These results indicate that a fragment of approximately 2.5 kb of hTERT promoter sequence (pGRN245) contains sufficient sequence elements to support both activation and repression in response to proliferation and/or growth arrest stimuli that control telomerase activity in IDH4 cells. Two clones, ID245-1 and ID245-16 whose SEAP profile closely matched telomerase activity during drug treatment, were selected and expanded for high throughput screening of small molecule activators of telomerase.

### Example 6: hTERT Promoter Sequences Driving a β-galactosidase Reporter Gene to Identify Biological Regulators of hTERT and Telomerase activity.

The invention also provides constructs and promoter-based assays to identify biological modulators of hTERT and telomerase activity. An exemplary construct of this aspect of the invention is pGRN353 containing a BgIII-HindIII fragment from pGRN297 with approximately 2.5 kb of hTERT promoter sequences cloned into the BgIII-HindIII sites of β-gal-Basic (ClonTech). pGRN353 or similar constructs are re-introduced into BJ cells by co-transfection with a plasmid containing a hygromycin gene as selection marker. Clonal cell lines and/or mass populations are established and used to screen retroviral based cDNA libraries for genes or fragments of genes that can activate the hTERT promoter. pGRN353 or similar constructs are also re-introduced into 143B and 293 cells to screen retroviral libraries to identify sequences that can repress the hTERT promoter.

### Example 7: Identifying Trans-Acting Transcriptional Regulatory Elements

The promoter-reporter (and other) vectors of the invention are also used to identify trans-acting transcriptional regulatory elements. As noted supra, plasmids in which reporter genes are operably linked to hTERT promoter sequences are extremely useful for identification of trans-acting transcriptional modulatory agents and for the screening of potential hTERT promoter-modulating drugs (including biological agents and small molecules). Both transient and stable transfection techniques can be used. In one embodiment, stable transformants of pGRN148 are made in telomerase negative and telomerase positive cells by cotransfection with a eukaryotic selectable marker (such as neo), according to Ausubel, supra.

The resulting cell lines are used for screening of putative telomerase trans-acting transcriptional modulatory agents, for example, by comparing hTERT-promoter-driven expression in the presence and absence of the test compound (the putative trans-acting transcriptional modulating agent). Additional promoter-reporter vectors (including the constructs described herein, as variations thereof) are similarly used to identify and isolate trans-acting factors binding to cis-acting transcriptional regulatory elements, such as, Myc, Sp1, TATA box binding protein, AP-1, CREB, CAAT binding factor and factors binding to hormone response elements (e.g., GRE). The identification and isolation of such trans-acting regulatory sequences provide for further methods and reagents for modulating the transcription and translation of telomerase.

### Example 8: c-Myc acts as a Potent Activator of the TERT Promoter by Direct Interaction with Cis-Acting Regulatory Sequences

Use of recombinant constructs comprising TERT promoter sequences of the invention has, for the first time, demonstrated that c-Myc acts as a potent activator of telomerase activity by direct interaction with cis-acting regulatory sequences in the TERT promoter. Significantly, the studies of the invention also show that transcriptional activation of the hTERT promoter by c-Myc can be abrogated by deletion or mutation of a single cis-acting regulatory sequence, the "Myc/Max binding site."

To determine whether experimental induction of c-Myc can lead to the de novo activation of telomerase in primary human cells, pre-senescent IMR90 cultures engineered to express the mouse ecotropic receptor (Serrano et al. (1997) Cell 88, 593-602) were transduced with either the pBABE retroviral vector or one encoding a hormone inducible c-Myc-Estrogen Receptor (cMycER) fusion protein (Eilers et al., 1989 Nature 340, 66-68; Littlewood (1995) Nuc. Acids Res. 23, 1686-1690). IMR90 cultures do not possess detectable telomerase activity or TERT gene expression (Nakamura et al., 1997; Meyerson et al., 1997).

Retroviral Infection The mouse ecotropic receptor was transduced into IMR90 fibroblasts and all subsequent transductions with ecotropic retrovirus were carried out according to Serrano et al. (1997). pBABE-MycER and pBABE vector control viruses were harvested from stable expressing _2 cell lines.

Cell Culture: IMR90 cells were grown in Dulbecco's Modified Eagle Medium (DMEM) (Gibco/BRL) supplemented with 10% fetal bovine serum (FBS), 0.29 mg/mL L-glutamine, 0.03% penicillin and streptomycin, and 25 µg/mL gentamycin sulfate. For the Myc induction studies in IMR90 cells, MycER transduced cells were exposed to 2 µM 4-OHT for 24, 48 and 72 hours. For the promoter studies NIH 3T3 cells were exposed to 1 µM 4-OHT for 24 and 72 hours. In all cases uninduced controls were treated with an equivalent volume of ethanol, the solvent for 4-OHT.

Telomerase Assays: Telomerase activity was measured by a modified telomerase repeat amplification protocol using the TRAPeze™ telomerase detection kit (Oncor, Gaithersburg, MD) (Kim et al., 1994). Genomic DNA was obtained from vector control or MycER transduced IMR90 fibroblasts. TRAP assays were performed on lysates equivalent to 1000 cells for all samples, with 293T cell lysates serving as a positive control for telomerase activity. PCR internal controls from each experiment were amplified equally. Inactivation of lysate was for 5 minutes at 85°C prior to the TRAP assay.

In the MycER system, the Myc moiety exists in a latent form bound in a complex with HSP-90 through its ER fusion (Eilers et al., 1989; Littlewood et al., 1995). Upon treatment with 4-hydroxy-tamoxifen (4-OHT), the MycER protein is liberated from HSP-90, resulting in a Myc over-expression phenotype (Eilers et al., 1989; Littlewood et al., 1995). Employing this cell culture system, 4-OHT treatment of MycER-transduced IMR90 cultures resulted in the marked and sustained activation of telomerase to a level at or above that detected in lysates derived from an equivalent number of telomerase-positive 293T tumor cells, as assayed by the sensitive TRAP assay. In contrast, untreated MycER-transduced or 4-OHT-treated pBABE-transduced IMR90 cultures remained telomerase negative. Western blot analysis confirmed abundant MycER protein levels in the MycER-transduced cultures in the presence or absence of 4-OHT.

Notably, enforced expression of oncogenes such as H-Ras, and cellular modulators of the Rb and p53 pathways (E7, cyclin D1, Mdm2, dominant-negative p53) have not been found to be capable of influencing telomerase activity in IMR90 cells (Wang et al., 1998).

### c-Myc Enhancement of hTERT Transcription Requires the Presence of a Cis-Acting Promoter Element: the Proximal Myc-Binding E-Box

hTERT Reporter Construction: The pGRN150 (E box deleted), pGRN261 (2.5 kbp hTERT reporter) are described above. NIH 3T3 cells were grown in Dulbecco's Modified Eagle Medium (DMEM) (Gibco/BRL) supplemented with 10% fetal bovine serum (FBS), 0.29 mg/mL L-glutamine, 0.03% penicillin and streptomycin, and 25 µg/mL gentamycin sulfate. NIH 3T3 cells were transfected using LipoFectamine reagent (Life Sciences) with 100 ng of a promoter reporter, and 200 ng of pCMX-β-Galactosidase which served as an internal control for transfection efficiency. Transfected cells were allowed to recover for 6 hours in complete DMEM and then treated with 1 µM 4-OHT or ethanol for 36 hours prior to analysis of secreted alkaline phosphatase activity using the Great EscAPe™ assay (ClonTech). β-galactosidase activity was assayed by incubation of whole cell extracts with 400 µg/ml ONPG in buffer containing 60 mM Na2HPO4, 40 mM NaH2PO4, 10 mM KCl and 1 mM MgSO4 and relative transfection efficiencies determined by reading absorbance at 415 nm.

Expression of endogenous hTERT following exposure to 4-OHT (or solvent alone) was measured at various times in the presence of 1 µM cyclohexamide in IMR90 fibroblasts transduced with MycER. Reverse transcription of RNA derived from each sample followed by PCR and Southern blotting of the amplified products was carried out as described above. Glyceraldehyde-6-phosphate dehydrogenase (GAPDH) was amplified from the same reverse transcription products as an internal semi-quantitative control and visualized by ethidium bromide staining. Low level expression of hTERT mRNA was detected in uninduced samples after very long exposures; however, the level of hTERT mRNA did not change over time in the uninduced samples.

The activity of the hTERT promoter was dramatically enhanced by c-Myc-ER in NIH 3T3 cells. The ability of c-Myc-ER to enhance hTERT promoter activity was dependent upon sequences in the hTERT promoter that included an evolutionarily conserved Myc binding site (E-box).

To determine whether the increased telomerase activity induced by activation of c-Myc-ER was a result of increased transcription of the hTERT gene we initially examined the effect of 4-OHT induction of c-Myc-ER activity upon hTERT promoter sequences placed upstream of the secreted alkaline phosphatase reporter gene. The hTERT promoter contains two putative Myc-binding sites positioned at -242 and -34 relative to the ATG initiation codon.

NIH 3T3 cells engineered to express c-Myc-ER stably were transfected with constructs containing a secreted alkaline phosphatase reporter under the control of a 2.5 kb fragment of the hTERT promoter, a 2.5 kb fragment of the hTERT promoter lacking the proximal E-box, or a promoterless reporter construct. The basal activity of the wild-type hTERT promoter and that of the hTERT promoter lacking the proximal E-box were equivalent and approximately 3 fold higher than the activity of the promoterless reporter. Induction of c-Myc-ER activity with 1 µM 4-OHT enhanced the activity of the 2.5 kb hTERT promoter approximately 10 fold. By contrast, the activity of the promoter lacking the proximal E-box was not significantly affected by induction of c-Myc-ER. Similarly, the promoterless reporter was not affected by induction of c-Myc-ER. Clearly, this shows that transcription of a heterologous encoding region can be regulated by modulating a transcriptional regulatory element such as c-Myc within the promoter region, which in turn is modulated by a ligand for the estrogen receptor.

To further confirm the role of the proximal E-box in regulating the hTERT promoter we tested the effect of changing the E-box from CACGTG to CACTCA. The mutation in the E-box reduced the promoter activity due to 4-OHT stimulation to the equivalent of the E-box deletion and 10-fold below the wild-type promoter. This demonstrates that c-Myc-ER is not able to significantly activate an hTERT promoter with an attenuated E-box at - 34 and that the E-box at -242 is not able to significantly mediate c-Myc activation. These results suggest that the ability of c-Myc to stimulate the hTERT promoter is mediated via the -34 E-box.

### hTERT is a Direct Target of c-Myc Regulated Transcription

To confirm the ability of c-Myc to stimulate transcription of the hTERT gene directly, we assayed for hTERT gene expression in MycER-transduced cultures of IMR90 cells 0, 1, 3 and 9 hours following the addition of 4-OHT. The cultures were treated with cyclohexamide for 30 minutes prior to addition of 4-OHT to prevent de novo protein synthesis. hTERT expression was undetectable at the zero hour time point for the Myc transduced cultures. Pretreatment of these cells with cyclohexamide alone had no effect on expression of hTERT mRNA. Induction of the c-Myc-ER activity by treatment with 2 M 4-OHT in the presence of 1 cyclohexamide led to a rapid increase in expression of hTERT message.

hTERT expression was detected by 1 hour post-induction, and increased 3 and 9 hours post induction. By contrast, cells treated with solvent alone were not induced to express hTERT. Furthermore, the expression level of GAPDH was similar at all time points in cells treated with 4-OHT or solvent atone. These observations strongly suggest that Myc acts directly upon the hTERT promoter to enhance transcription of the hTERT gene.

### Lack of Equivalence of Myc and TERT in Cellular Transformation.

To further explore the functional implications of Myc induction of telomerase activity in primary cells, we examined whether TERT could substitute for c-Myc as an immortalizing agent in the rat embryonic fibroblast (REF) cooperation assay. In this assay, co-transfection of Myc and activated RAS (H-RASG12V) effects the malignant transformation of early passage REFs. This cooperative activity can be quantified by monitoring the number of transformed foci appearing in the monolayer 7 to 10 days post-transfection. In two separate experiments, various combinations of the expression constructs encoding c-Myc, H-RASG12V, TERT, or vector control were introduced into early passage REFs. Strong cooperative activity was observed in the RAS and Myc co-transfections as evidenced by an average of 34 foci per 10 cm plate; while Ras alone generated between 0 and 3 foci per plate; consistent with previous findings that an immortalizing agent and activated RAS are required for efficient transformation of primary rodent cells (Land et al., 1983). By contrast, co-transfection of TERT and RAS did not generate transformed foci counts above that scored for the RAS alone controls. These results indicate that expression of hTERT is insufficient to account for the immortalizing function of Myc in a rat embryonic fibroblast (REF) cooperation assay.

Effect of c-Myc-ER on the activity of the hTERT promoter in NIH3T3 cells was determined by detection of secreted alkaline phosphatase activity. Cells were treated with 4-OHT for 36 hours. Uninduced cells were treated with solvent alone for 36 hours. The detected secreted alkaline phosphatase activity was corrected for transfection efficiency in each case using β-galactosidase.

### Example 9: Cloning of mouse TERT promoter

The following example details the cloning of the mouse mTERT promoter.

mTERT Construction : A hybridization probe (nucleotides 1586-1970) of the mTERT cDNA (pGRN188) was used to identify a recombinant phage (mTERT1) from a 129SV mouse genomic phage library (Stratagene). An 8 kb HindIII fragment of mTERT1 that hybridized to the 1586-1970 probe was subcloned into pBluescript™ II KS + (Stratagene) to generate clone B2.18. The regions encompassing the initiator and promoter were sequenced.

The mTERT upstream sequence is listed in SEQ. ID NO:2 The sequence can be obtained on GenBank under Accession B2.18 AF121949.

**Figure 3** shows the alignment of homologous portions of the human and mouse promoter sequences. The sequences were aligned using the GAP program from the Wisconsin GCG package, using a value of 48 for gap creation and a value of 3 for gap extension. Using a small portion of the coding region (~ 450 bases) was found to improve the initial alignment.

### Conservation of Human and Mouse TERT Promoters

To determine whether the ability of c-Myc to enhance telomerase activity was mediated through increased transcription of the hTERT gene, we compared the sequences of the human and mouse TERT promoters. Alignment of the first 300 bases of the human and mouse promoters indicates a number of conserved regions. In particular, the Myc/Max binding site (E-box) located at -34 of the human promoter and at - 32 of the mouse promoter, are highly conserved. A second E-box was identified at -242 of the human promoter; however, this site was not conserved in the mouse promoter. These observations raised the possibility that the conserved Myc binding site in particular might play a role in the regulation of hTERT expression by c-Myc

### Example 10: Exemplary oncolytic virus

Based on the principles illustrated in Example 4, the following experiment was done as a model for an oncolytic virus based on the Ad2 type adenovirus. A construct was made in which the adenovirus E1a replication gene was placed under control of the hTERT promoter, which should activate transcription in telomerase-expressing cancer cells. As a positive control, a similar construct was made in which E1a was placed under control of the CMV promoter, which should activate transcription in any cell.

Reagents were obtained as follows. pBR322, restriction enzymes: NEB, Beverly, MA. Adenovirus Type 2 (Ad2), tissue culture reagents: Gibco/BRL, Grand Island, NY. Protection Mammalian Transfection Systems: Promega, Madison, WI. Tumor and Normal Cell lines: ATCC, Manassas, VA, except BJ line, which was obtained from J. Smith, U. of Texas Southwestern Medical Center.

Briefly, a pBR322-based plasmid was constructed which contains the Adenovirus Type 2 genome with deletions from 356-548nt (E1a promoter region) and 27971-30937nt (E3). A multiple cloning region was inserted at the point of deletion of the E1a promoter, and hTERT promoter (-239 to -36nt) or CMV promoter (-524 to -9nt) was subsequently cloned. Numbering of the CMV sequence is in accodance with Akrigg et al., Virus Res 2:107, 1985. Numbering of the Ad2 sequence is in accordance with "DNA Tumor Viruses: Molecular Biology of Tumor Viruses", J. Tooze ed., Cold Spring Harbor Laboratory, NY.

These plasmid DNAs were digested with SnaBI to liberate ITRs, then phenol-chloroform extracted, precipitated and transfected into 293A cells for propagation of the virus. Several rounds of plaque purifications were performed using A549 cells, and a final isolate was expanded on these same cells. Viruses were titered by plaque assay on 293A cells, and tested for the presence of 5' WT Ad sequences by PCR. DNA was isolated from viruses by HIRT extraction.

The hTERT promoter construct was designated *AdphTERT-E1dIE3*. The CMV promoter construct was designated *AdCMV-E1dIE3*.

**Figure 4** shows the effect of these viruses on normal and cancer-derived cell lines. Each cell line was plated at 5x10 in a 48-well format and infected at an MOI=20, ~24h post plating. The cells were then cultured over a period of 17-48 days, and fed every fourth day. The pictures shown in the Figure were taken 7 days after infection. The top row shows the results of cells that were not virally infected (negative control). The middle row shows the results of cells infected with oncolytic adenovirus, in which replication gene E1a is operably linked to the hTERT promoter. The bottom row shows the results of cells infected with adenovirus in which E1a is operably linked to the CMV promoter (positive control). Results are summarized in Table 2:

**TABLE 2:**

| Effect of Oncolytic Virus on Cancerous and Non-cancerous Cells | | | | | | |
|---|---|---|---|---|---|---|
| Cell Line | Origin | Culture Conditions | | Uninfected cell Lysis | Lysis by Ad-phTERT-E1dIE3 | Lysis by Ad-CMV-E1dIE3 |
| BJ | foreskin fibroblast | 90% DMEM/M199 + 10% FBS | Fig. 4 (A) | NO | NO | YES |
| IMR | lung fibroblast | 90% DMEM/M199+ 10% FBS | Fig.4(A) | NO | NO | YES |
| WI-38 | lung fibroblast | 90% DMEM/M199 + 10% FBS + 5 µg mL gentamicin | Fig. 4 (A) | NO | NO | YES |
| A549 | lung carcinoma | 90% RPMI + 10% FBS | ― | NO | YES | YES |
| AsPC-1 | adenocarcinoma, pancreas adenocarcinoma, pancreas | 90% RPMI + 10% FBS | ― | NO | YES | YES |
| BxPC-3 | | 90% EMEM + 10% FBS | ― | NO | YES | YES |
| DAOY | medulloblastoma | 90% EMEM + 10% FBS | Fig.4(B) | NO | YES | YES |
| HeLa: | cervical carcinoma | 90% EMEM + 10% FBS | Fig.4(B) | NO | YES | YES |
| HT1080 | fibrosarcoma | 90% EMEM + 10% FBS | Fig.4(B) | NO | YES | YES |

All cell lines tested were efficiently lysed by AdCMV-E1dlE3 by day 17 post-infection. All tumor lines were lysed by AdphTERT-E1dlE3 in a similar, but slightly delayed time-frame, while normal lines showed no signs of cytopathic effect and remained healthy out to 6 weeks post-infection.

In a parallel experiment, each cell line was infected with an adenovirus containing the gene encoding the green fluorescent protein as a visual marker (MOI=100), to determine relative transduction efficiency of these cells by adenovirus vectors. The cell lines exhibited a wide range of transduction efficiencies (∼1-2% to 100%). Even cells that are transduced poorly can be efficiently eradicated with the hTERT controlled adenovirus.

Together, the results confirm that a oncolytic virus can be constructed by placing a genetic element essential for replication of the virus under control of an hTERT promoter. Replication and lysis occurs in cancer cells, but not in differentiated non-malignant cells.

Figure 5 is a map of the oncolytic adenovirus used in the infection experiment shown in Figure 4. It comprises the Inverted Terminal Repeat (ITR) from the adenovirus (Ad2); followed by the hTERT medium-length promoter (phTERT176) operably linked to the adenovirus E1a region; followed by the rest of the adenovirus deleted for the E3 region (ΔE3). Shown underneath are some modified constructs. The middle construct comprises an additional sequence in between the hTERT promoter and the E1a region. The HI sequence is an artificial intron engineered from adenovirus and immunoglobulin intron splice donor and acceptor sequences. It is thought that placing an intron in the hTERT promoter adenovirus replication gene cassette will promote processing and transport of heteronuclear RNA, thereby facilitating formation of the replicated viral particles. The third adenovirus construct is similar, except that the E1a region used is longer at the 5' end by 51 nucleotides. It is thought that this may also promote more efficient conditional replication of the oncolytic virus.

### REFERENCES

1. Bello-Femandez. (1993).. Proc Natl Acad Sci U S A. 90, 7804-8.
2. Bishop (1991). C.S.H. Symp. Quant. Biol. 56, 99-107.
3. Bodnar (1996). Expt. Cell Res. 228, 58-64.
4. Bodnar (1998). Science 279,349-52.
5. Chase (1998) Nature Biotechnol. 16, 444-448.
6. Coffey (1998) Science 282:1332-1334
7. Counter (1992).EMBO J. 11, 1921-1929.
8. Eilers (1989). Nature 340, 66-68.
9. Eilers (1991). EMBO J. 10, 133-41.
10. Fujimoto.(1997). Biochem. & Biophys. Res. Comm. 241, 775-781.
11. Galaktionov (1996). Nature 382, 511-7
12. Grandori (1996). EMBOJ. 15,4344-57
13. Grandori (1997). TIBS 22, 177-181.
14. Greenberg, (1998) Oncogene 16, 1723-30.
15. Harley (1990). Nature 345, 458-460.
16. Harrington. (1997). Genes Dev. 11, 3109-3115.
17. Hastie (1990) Nature 346, 866-868.
18. Hiyama (1995). Nature Med. 1, 249-255.
19. Kilian (1997). Hum. Mol. Genet. 6, 2011-2019.
20. Kim, (1994) Science 266, 2011-2015.
21. Kiyono (1998). Nature 396, 84-88.
22. Klingelhutz (1996) Nature 380, 79-82.
23. Kramm (1997) Hum. Gene Ther.8, 2057-2068.
24. Land (1983) Nature 304, 596-602.
25. Lee (1997) Proc Natl Acad Sci U S A 94,12886-91.
26. Marhin (1997) Oncogene 14, 2825-34.
27. Meyerson (1997) Cell 90, 785-795.
28. Nakamura (1997) Science 277, 955-959.
29. Nakayama (1998) Nature Genet. 18, 65-68.
30. Reed (1986) Proc. Natl. Acad. Sci USA 83, 3982-3986.
31. Schreiber-Agus (1995) Cell 80, 777-786.
32. Smith (1998) Science 282, 1484-7.
33. Toda (1998) Human Gene Therapy 9, 2177-2185.
34. van Steensel (1997) Nature 385,740-3.
35. Vaziri (1998) Curr. Biol. 8, 279-282.
36. Wagner. (1993) Cell Growth Differ. 4,879-83.
37. Wang. (1998) Genes Dev. 12, 1769-74.
38. Wright. (1995) Trends Cell Biol. 5, 293-297.
39. Xu (1997) Oncogene 15, 2589-2596.

### BIOLOGICAL DEPOSIT

The lambda clone designated λGϕ5 (from which SEQ. ID NO:1 was determined) was deposited under terms of the Budapest Treaty with the American Type Culture Collection (ATCC), 10801 University Blvd., Manassas, Virginia 20110-2209 U.S.A., on August 14, 1997, under Accession No. 98505.

### SEQUENCE LISTING

SEQ. ID NO:1 (hTERT gene sequence in GenBank Accession AF121948)

GCGGCCGCGA GCTCTAATAC GACTCACTAT AGGGCGTCGA CTCGATCAAT GGAAGATGAG 60
GCATTGCCGA AGAAAAGATT AATGGATTTG AACACACAGC AACAGAAACT ACATGAAGTG 120
AAACACAGGA AAAAAAAGAT AAAGAAACGA AAGAAAAAGG GCATCAGTGA GCTTCAGCAG 180
AAGTTCCATC GGCCTTACAT ATGTGTAAGC AGAGGCCCTG TAGGAGCAGA GGCAGGGGGA 240
AAATACTTTA AGAAATAATG TCTAAAAGTT TTTCAAATAT GAGGAAAAAC ATAAAACCAC 300
AGATCCAAGA AGCTCAACAA AACAAAGCAC AAGAAACAGG AAGAAATTAA AAGTTATATC 360
ACAGTCAAAT TGCTGAAAAC CAGCAACAAA GAGAATATCT TAAGAGTATC AGAGGAAAAG 420
AGATTAATGA CAGGCCAAGA AACAATGAAA ACAATACAGA TTTCTTGTAG GAAACACAAG 480
ACAAAAGACA TTTTTTAAAA CCAAAAGGAA AAAAAATGCT ACATTAAAAT GTTTTTTACC 540
CACTGAAAGT ATATTTCAAA ACATATTTTA GGCCAGGCTT GGTGGCTCAC ACCTGTAATC 600
CCAGCACTTT GGGAGGCCAA GGTGGGTGGA TCGCTTAAGG TCAGGAGTTC GAGACCAGCC 660
TGGCCAATAT AGCGAAACCC CATCTGTACT AAAAACACAA AAATTAGCTG GGTGTGGTGA 720
CACATGCCTG TAATCCCAGG TACTCAGGAG GCTAAGGCAG GAGAATTGCT TGAACTGGGA 780
GGCAGAGGTG GTGAGCCAAG ATTGCACCAG TGCACTCCAG CCTTGGTGAC AGAGTGAAAC 840
TCCATCTCAA AAACAAACAA ACAAAATACA TATACATAAA TATATATGCA CATATATATA 900
CATATATAAA TATATATACA CATATATAAA TCTATATACA TATATACATA TATACACATA 960
TATAAATCTA TATACATATA TATACATATA TAATATATTT ACATATATAA ATATATACAT 1020
ATATAAATAT ACATATATAA ATACATATAT AAATATACAT ATATAAATAT ACATATATAA 1080
ATATACATAT ATAAATATAT ACATATATAA ATATACATAT ATAAATATAT ATACATATAT 1140
AAATATATAA ATATACAAGT ATATACAAAT ATATACATAT ATAAATGTAT ATACGTATAT 1200
ACATATATAT ATAAATATAT AAAAAAACTT TTGGCTGGGC ACCTTTCCAA ATCTCATGGC 1260
ACATATAAGT CTCATGGTAA CCTCAAATAA AAAAACATAT AACAGATACA CCAAAAATAA 1320
AAACCAATAA ATTAAATCAT GCCACCAGAA GAAATTACCT TCACTAAAAG GAACACAGGA 1380
AGGAAAGAAA GAAGGAAGAG AAGACCATGA AACAACCAGA AAACAAACAA CAAAACAGCA 1440
GGAGTAATTC CTGACTTATC AATAATAATG CTGGGTGTAA ATGGACTAAA CTCTCCAATC 1500
AAAAGACATA GAGTGGCTGA ATGGACGAAA AAAACAAGAC TCAATAATCT GTTGCCTACA 1560
AGAATATACT TCACCTATAA AGGGACACAT AGACTGAAAA TAAAAGGAAG GAAAAATATT 1620
CTATGCAAAT GGAAACCAAA AAAAGAACAG AACTAGCTAC ACTTATATCA GACAAAATAG 1680
ATTTCAAGAC AAAAAGTACA AAAAGAGACA AAGTAATTAT ATAATAATAA AGCAAAAAGA 1740
TATAACAATT GTGAATTTAT ATGCGCCCAA CACTGGGACA CCCAGATATA TACAGCAAAT 1800
ATTATTAGAA CTAAGGAGAG AGAGAGATCC CCATACAATA ATAGCTGGAG ACTTCACCCC 1860
GCTTTTAGCA TTGGACAGAT CATCCAGACA GAAAATCAAC CAAAAAATTG GACTTAATCT 1920
ATAATATAGA ACAAATGTAC CTAATTGATG TTTACAAGAC ATTTCATCCA GTAGTTGCAG 1980
AATATGCATT TTTTCCTCAG CATATGGATC ATTCTCAAGG ATAGACCATA TATTAGGCCA 2040
CAGAACAAGC CATTAAAAAT TCAAAAAAAT TGAGCCAGGC ATGATGGCTT ATGCTTGTAA 2100
TTACAGCACT TTGGGGAGGG TGAGGTGGGA GGATGTCTTG AGTACAGGAG TTTGAGACCA 2160
GCCTGGGCAA AATAGTGAGA CCCTGTCTCT ACAAACTTTT TTTTTAATT AGCCAGGCAT 2220
AGTGGTGTGT GCCTGTAGTC CCAGCTACTT AGGAGGCTGA AGTGGGAGGA TCACTTGAGC 2280
CCAAGAGTTC AAGGCTACGG TGAGCCATGA TTGCAACACC ACACACCAGC CTTGGTGACA 2340
GAATGAGACC CTGTCTCAAA AAAAAAAAAA AAAATTGAAA TAATATAAAG CATCTTCTCT 2400
GGCCACAGTG GAACAAAACC AGAAATCAAC AACAAGAGGA ATTTTGAAAA CTATACAAAC 2460
ACATGAAAAT TAAACAATAT ACTTCTGAAT AACCAGTGAG TCAATGAAGA AATTAAAAAG 2520
GAAATTGAAA AATTTATTTA AGCAAATGAT AACGGAAACA TAACCTCTCA AAACCCACGG 2580
TATACAGCAA AAGCAGTGCT AAGAAGGAAG TTTATAGCTA TAAGCAGCTA CATCAAAAAA 2640
GTAGAAAAGC CAGGCGCAGT GGCTCATGCC TGTAATCCCA GCACTTTGGG AGGCCAAGGC 2700
GGGCAGATCG CCTGAGGTCA GGAGTTCGAG ACCAGCCTGA CCAACACAGA GAAACCTTGT 2760
CGCTACTAAA AATACAAAAT TAGCTGGGCA TGGTGGCACA TGCCTGTAAT CCCAGCTACT 2820
CGGGAGGCTG AGGCAGGATA ACCGCTTGAA CCCAGGAGGT GGAGGTTGCG GTGAGCCGGG 2880
ATTGCGCCAT TGGACTCCAG CCTGGGTAAC AAGAGTGAAA CCCTGTCTCA AGAAAAAAAA 2940
AAAAGTAGAA AAACTTAAAA ATACAACCTA ATGATGCACC TTAAAGAACT AGAAAAGCAA 3000
GAGCAAACTA AACCTAAAAT TGGTAAAAGA AAAGAAATAA TAAAGATCAG AGCAGAAATA 3060
AATGAAACTG AAAGATAACA ATACAAAAGA TCAACAAAAT TAAAAGTTGG TTTTTTGAAA 3120
AGATAAACAA AATTGACAAA CCTTTGCCCA GACTAAGAAA AAAGGAAAGA AGACCTAAAT 3180
AAATAAAGTC AGAGATGAAA AAAGAGACAT TACAACTGAT ACCACAGAAA TTCAAAGGAT 3240
CACTAGAGGC TACTATGAGC AACTGTACAC TAATAAATTG AAAAACCTAG AAAAAATAGA 3300
TAAATTCCTA GATGCATACA ACCTACCAAG ATTGAACCAT GAAGAAATCC AAAGCCCAAA 3360
CAGACCAATA ACAATAATGG GATTAAAGCC ATAATAAAAA GTCTCCTAGC AAAGAGAAGC 3420
CCAGGACCCA ATGGCTTCCC TGCTGGATTT TACCAATCAT TTAAAGAAGA ATGAATTCCA 3480
ATCCTACTCA AACTATTCTG AAAAATAGAG GAAAGAATAC TTCCAAACTC ATTCTACATG 3540
GCCAGTATTA CCCTGATTCC AAAACCAGAC AAAAACACAT CAAAAACAAA CAAACAAAAA 3600
AACAGAAAGA AAGAAAACTA CAGGCCAATA TCCCTGATGA ATACTGATAC AAAAATCCTC 3660
AACAAAACAC TAGCAAACCA AATTAAACAA CACCTTCGAA AGATCATTCA TTGTGATCAA 3720
GTGGGATTTA TTCCAGGGAT GGAAGGATGG TTCAACATAT GCAAATCAAT CAATGTGATA 3780
CATCATCCCA ACAAAATGAA GTACAAAAAC TATATGATTA TTTCACTTTA TGCAGAAAAA 3840
GCATTTGATA AAATTCTGCA CCCTTCATGA TAAAAACCCT CAAAAAACCA GGTATACAAG 3900
AAACATACAG GCCAGGCACA GTGGCTCACA CCTGCGATCC CAGCACTCTG GGAGGCCAAG 3960
GTGGGATGAT TGCTTGGGCC CAGGAGTTTG AGACTAGCCT GGGCAACAAA ATGAGACCTG 4020
GTCTACAAAA AACTTTTTTA AAAAATTAGC CAGGCATGAT GGCATATGCC TGTAGTCCCA 4080
GCTAGTCTGG AGGCTGAGGT GGGAGAATCA CTTAAGCCTA GGAGGTCGAG GCTGCAGTGA 4140
GCCATGAACA TGTCACTGTA CTCCAGCCTA GACAACAGAA CAAGACCCCA CTGAATAAGA 4200
AGAAGGAGAA GGAGAAGGGA GAAAGGAGGG AGAAGGGAGG AGGAGGAGAA GGAGGAGGTG 4260

GAGGAGAAGT GGAAGGGGAA GGGGAAGGGA AAGAGGAAGA AGAAGAAACA TATTTCAACA 4320
TAATAAAAGC CCTATATGAC AGACCGAGGT AGTATTATGA GGAAAAACTG AAAGCCTTTC 4380
CTCTAAGATC TGGAAAATGA CAAGGGCCCA CTTTCACCAC TGTGATTCAA CATAGTACTA 4440
GAAGTCCTAG CTAGAGCAAT CAGATAAGAG AAAGAAATAA AAGGCATCCA AACTGGAAAG 4500
GAAGAAGTCA AATTATCCTG TTTGCAGATG ATATGATCTT ATATCTGGAA AAGACTTAAG 4560
ACACCACTAA AAAACTATTA GAGCTGAAAT TTGGTACAGC AGGATACAAA ATCAATGTAC 4620
AAAAATCAGT AGTATTTCTA TATTCCAACA GCAAACAATC TGAAAAAGAA ACCAAAAAAG 4680
CAGCTACAAA TAAAATTAAA CAGCTAGGAA TTAACCAAAG AAGTGAAAGA TCTCTACAAT 4740
GAAAACTATA AAATATTGAT AAAAGAAATT GAAGAGGGCA CAAAAAAAGA AAAGATATTC 4800
CATGTTCATA GATTGGAAGA ATAAATACTG TTAAAATGTC CATACTACCC AAAGCAATTT 4860
ACAAATTCAA TGCAATCCCT ATTAAAATAC TAATGACGTT CTTCACAGAA ATAGAAGAAA 4920
CAATTCTAAG ATTTGTACAG AACCACAAAA GACCCAGAAT AGCCAAAGCT ATCCTGACCA 4980
AAAAGAACAA AACTGGAAGC ATCACATTAC CTGACTTCAA ATTATACTAC AAAGCTATAG 5040
TAACCCAAAC TACATGGTAC TGGCATAAAA ACAGATGAGA CATGGACCAG AGGAACAGAA 5100
TAGAGAATCC AGAAACAAAT CCATGCATCT ACAGTGAACT CATTTTTGAC AAAGGTGCCA 5160
AGAACATACT TTGGGGAAAA GATAATCTCT TCAATAAATG GTGCTGGAGG AACTGGATAT 5220
CCATATGCAA AATAACAATA CTAGAACTCT GTCTCTCACC ATATACAAAA GCAAATCAAA 5280
ATGGATGAAA GGCTTAAATC TAAAACCTCA AACTTTGCAA CTACTAAAAG AAAACACCGG 5340
AGAAACTCTC CAGGACATTG GAGTGGGCAA AGACTTCTTG AGTAATTCCC TGCAGGCACA 5400
GGCAACCAAA GCAAAAACAG ACAAATGGGA TCATATCAAG TTAAAAAGCT TCTGCCCAGC 5460
AAAGGAAACA ATCAACAAAG AGAAGAGACA ACCCACAGAA TGGGAGAATA TATTTGCAAA 5520
CTATTCATCT AACAAGGAAT TAATAACCAG TATATATAAG GAGCTCAAAC TACTCTATAA 5580
GAAAAACACC TAATAAGCTG ATTTTCAAAA ATAAGCAAAA GATCTGGGTA GACATTTCTC 5640
AAAATAAGTC ATACAAATGG CAAACAGGCA TCTGAAAATG TGCTCAACAC CACTGATCAT 5700
CAGAGAAATG CAAATCAAAA CTACTATGAG AGATCATCTC ACCCCAGTTA AAATGGCTTT 5760
TATTCAAAAG ACAGGCAATA ACAAATGCCA GTGAGGATGT GGATAAAAGG AAACCCTTGG 5820
ACACTGTTGG TGGGAATGGA AATTGCTACC ACTATGGAGA ACAGTTTGAA AGTTCCTCAA 5880
AAAACTAAAA ATAAAGCTAC CATACAGCAA TCCCATTGCT AGGTATATAC TCCAAAAAAG 5940
GGAATCAGTG TATCAACAAG CTATCTCCAC TCCCACATTT ACTGCAGCAC TGTTCATAGC 6000
AGCCAAGGTT TGGAAGCAAC CTCAGTGTCC ATCAACAGAC GAATGGAAAA AGAAAATGTG 6060
GTGCACATAC ACAATGGAGT ACTACGCAGC CATAAAAAAG AATGAGATCC TGTCAGTTGC 6120
AACAGCATGG GGGGCACTGG TCAGTATGTT AAGTGAAATA AGCCAGGCAC AGAAAGACAA 6180
ACTTTTCATG TTCTCCCTTA CTTGTGGGAG CAAAAATTAA AACAATTGAC ATAGAAATAG 6240
AGGAGAATGG TGGTTCTAGA GGGGTGGGGG ACAGGGTGAC TAGAGTCAAC AATAATTTAT 6300
TGTATGTTTT AAAATAACTA AAAGAGTATA ATTGGGTTGT TTGTAACACA AAGAAAGGAT 6360
AAATGCTTGA AGGTGACAGA TACCCCATTT ACCCTGATGT GATTATTACA CATTGTATGC 6420
CTGTATCAAA ATATCTCATG TATGCTATAG ATATAAACCC TACTATATTA AAAATTAAAA 6480
TTTTAATGGC CAGGCACGGT GGCTCATGTC CATAATCCCA GCACTTTGGG AGGCCGAGGC 6540
GGTGGATCAC CTGAGGTCAG GAGTTTGAAA CCAGTCTGGC CACCATGATG AAACCCTGTC 6600
TCTACTAAAG ATACAAAAAT TAGCCAGGCG TGGTGGCACA TACCTGTAGT CCCAACTACT 6660
CAGGAGGCTG AGACAGGAGA ATTGCTTGAA CCTGGGAGGC GGAGGTTGCA GTGAGCCGAG 6720
ATCATGCCAC TGCACTGCAG CCTGGGTGAC AGAGCAAGAC TCCATCTCAA AACAAAAACA 6780
AAAAAAAGAA GATTAAAATT GTAATTTTTA TGTACCGTAT AAATATATAC TCTACTATAT 6840
TAGAAGTTAA AAATTAAAAC AATTATAAAA GGTAATTAAC CACTTAATCT AAAATAAGAA 6900
CAATGTATGT GGGGTTTCTA GCTTCTGAAG AAGTAAAAGT TATGGCCACG ATGGCAGAAA 6960
TGTGAGGAGG GAACAGTGGA AGTTACTGTT GTTAGACGCT CATACTCTCT GTAAGTGACT 7020
TAATTTTAAC CAAAGACAGG CTGGGAGAAG TTAAAGAGGC ATTCTATAAG CCCTAAAACA 7080
ACTGCTAATA ATGGTGAAAG GTAATCTCTA TTAATTACCA ATAATTACAG ATATCTCTAA 7140
AATCGAGCTG CAGAATTGGC ACGTCTGATC ACACCGTCCT CTCATTCACG GTGCTTTTTT 7200
TCTTGTGTGC TTGGAGATTT TCGATTGTGT GTTCGTGTTT GGTTAAACTT AATCTGTATG 7260
AATCCTGAAA CGAAAAATGG TGGTGATTTC CTCCAGAAGA ATTAGAGTAC CTGGCAGGAA 7320
GCAGGTGGCT CTGTGGACCT GAGCCACTTC AATCTTCAAG GGTCTCTGGC CAAGACCCAG 7380
GTGCAAGGCA GAGGCCTGAT GACCCGAGGA CAGGAAAGCT CGGATGGGAA GGGGCGATGA 7440
GAAGCCTGCC TCGTTGGTGA GCAGCGCATG AAGTGCCCTT ATTTACGCTT TGCAAAGATT 7500
GCTCTGGATA CCATCTGGAA AAGGCGGCCA GCGGGAATGC AAGGAGTCAG AAGCCTCCTG 7560
CTCAAACCCA GGCCAGCAGC TATGGCGCCC ACCCGGGCGT GTGCCAGAGG GAGAGGAGTC 7620
AAGGCACCTC GAAGTATGGC TTAAATCTTT TTTTCACCTG AAGCAGTGAC CAAGGTGTAT 7680
TCTGAGGGAA GCTTGAGTTA GGTGCCTTCT TTAAAACAGA AAGTCATGGA AGCACCCTTC 7740
TCAAGGGAAA ACCAGACGCC CGCTCTGCGG TCATTTACCT CTTTCCTCTC TCCCTCTCTT 7800
GCCCTCGCGG TTTCTGATCG GGACAGAGTG ACCCCCGTGG AGCTTCTCCG AGCCCGTGCT 7860
GAGGACCCTC TTGCAAAGGG CTCCACAGAC CCCCGCCCTG GAGAGAGGAG TCTGAGCCTG 7920
GCTTAATAAC AAACTGGGAT GTGGCTGGGG GCGGACAGCG ACGGCGGGAT TCAAAGACTT 7980
AATTCCATGA GTAAATTCAA CCTTTCCACA TCCGAATGGA TTTGGATTTT ATCTTAATAT 8040
TTTCTTAAAT TTCATCAAAT AACATTCAGG AGTGCAGAAA TCCAAAGGCG TAAAACAGGA 8100
ACTGAGCTAT GTTTGCCAAG GTCCAAGGAC TTAATAACCA TGTTCAGAGG GATTTTTCGC 8160
CCTAAGTACT TTTTATTGGT TTTCATAAGG TGGCTTAGGG TGCAAGGGAA AGTACACGAG 8220
GAGAGGACTG GGCGGCAGGG CTATGAGCAC GGCAAGGCCA CCGGGGAGAG AGTCCCCGGC 8280
CTGGGAGGCT GACAGCAGGA CCACTGACCG TCCTCCCTGG GAGCTGCCAC ATTGGGCAAC 8340
GCGAAGGCGG CCACGCTGCG TGTGACTCAG GACCCCATAC CGGCTTCCTG GGCCCACCCA 8400
CACTAACCCA GGAAGTCACG GAGCTCTGAA CCCGTGGAAA CGAACATGAC CCTTGCCTGC 8460
CTGCTTCCCT GGGTGGGTCA AGGGTAATGA AGTGGTGTGC AGGAAATGGC CATGTAAATT 8520
ACACGACTCT GCTGATGGGG ACCGTTCCTT CCATCATTAT TCATCTTCAC CCCCAAGGAC 8580
TGAATGATTC CAGCAACTTC TTCGGGTGTG ACAAGCCATG ACAACACTCA GTACAAACAC 8640
CACTCTTTTA CTAGGCCCAC AGAGCACGGC CCACACCCCT GATATATTAA GAGTCCAGGA 8700
GAGATGAGGC TGCTTTCAGC CACCAGGCTG GGGTGACAAC AGCGGCTGAA CAGTCTGTTC 8760
CTCTAGACTA GTAGACCCTG GCAGGCACTC CCCCAGATTC TAGGGCCTGG TTGCTGCTTC 8820
CCGAGGGCGC CATCTGCCCT GGAGACTCAG CCTGGGGTGC CACACTGAGG CCAGCCCTGT 8880

CTCCACACCC TCCGCCTCCA GGCCTCAGCT TCTCCAGCAG CTTCCTAAAC CCTGGGTGGG 8940
CCGTGTTCCA GCGCTACTGT CTCACCTGTC CCACTGTGTC TTGTCTCAGC GACGTAGCTC 9000
GCACGGTTCC TCCTCACATG GGGTGTCTGT CTCCTTCCCC AACACTCACA TGCGTTGAAG 9060
GGAGGAGATT CTGCGCCTCC CAGACTGGCT CCTCTGAGCC TGAACCTGGC TCGTGGCCCC 9120
CGATGCAGGT TCCTGGCGTC CGGCTGCACG CTGACCTCCA TTTCCAGGCG CTCCCCGTCT 9180
CCTGTCATCT GCCGGGGCCT GCCGGTGTGT TCTTCTGTTT CTGTGCTCCT TTCCACGTCC 9240
AGCTGCGTGT GTCTCTGTCC GCTAGGGTCT CGGGGTTTTT ATAGGCATAG GACGGGGGCG 9300
TGGTGGGCCA GGGCGCTCTT GGGAAATGCA ACATTTGGGT GTGAAAGTAG GAGTGCCTGT 9360
CCTCACCTAG GTCCACGGGC ACAGGCCTGG GGATGGAGCC CCCGCCAGGG ACCCGCCCTT 9420
CTCTGCCCAG CACTTTTCTG CCCCCCTCCC TCTGGAACAC AGAGTGGCAG TTTCCACAAG 9480
CACTAAGCAT CCTCTTCCCA AAAGACCCAG CATTGGCACC CCTGGACATT TGCCCCACAG 9540
CCCTGGGAAT TCACGTGACT ACGCACATCA TGTACACACT CCCGTCCACG ACCGACCCCC 9600
GCTGTTTTAT TTTAATAGCT ACAAAGCAGG GAAATCCCTG CTAAAATGTC CTTTAACAAA 9660
CTGGTTAAAC AAACGGGTCC ATCCGCACGG TGGACAGTTC CTCACAGTGA AGAGGAACAT 9720
GCCGTTTATA AAGCCTGCAG GCATCTCAAG GGAATTACGC TGAGTCAAAA CTGCCACCTC 9780
CATGGGATAC GTACGCAACA TGCTCAAAAA GAAAGAATTT CACCCCATGG CAGGGGAGTG 9840
GTTGGGGGGT TAAGGACGGT GGGGGCAGCA GCTGGGGGCT ACTGCACGCA CCTTTTACTA 9900
AAGCCAGTTT CCTGGTTCTG ATGGTATTGG CTCAGTTATG GGAGACTAAC CATAGGGGAG 9960
TGGGGATGGG GGAACCCGGA GGCTGTGCCA TCTTTGCCAT GCCCGAGTGT CCTGGGCAGG 10020
ATAATGCTCT AGAGATGCCC ACGTCCTGAT TCCCCCAAAC CTGTGGACAG AACCCGCCCG 10080
GCCCCAGGGC CTTTGCAGGT GTGATCTCCG TGAGGACCCT GAGGTCTGGG ATCCTTCGGG 10140
ACTACCTGCA GGCCCGAAAA GTAATCCAGG GGTTCTGGGA AGAGGCGGGC AGGAGGGTCA 10200
GAGGGGGGCA GCCTCAGGAC GATGGAGGCA GTCAGTCTGA GGCTGAAAAG GGAGGGAGGG 10260
CCTCGAGCCC AGGCCTGCAA GCGCCTCCAG AAGCTGGAAA AAGCGGGGAA GGGACCCTCC 10320
ACGGAGCCTG CAGCAGGAAG GCACGGCTGG CCCTTAGCCC ACCAGGGCCC ATCGTGGACC 10380
TCCGGCCTCC GTGCCATAGG AGGGCACTCG CGCTGCCCTT CTAGCATGAA GTGTGTGGGG 10440
ATTTGCAGAA GCAACAGGAA ACCCATGCAC TGTGAATCTA GGATTATTTC AAAACAAAGG 10500
TTTACAGAAA CATCCAAGGA CAGGGCTGAA GTGCCTCCGG GCAAGGGCAG GGCAGGCACG 10560
AGTGATTTTA TTTAGCTATT TTATTTTATT TACTTACTTT CTGAGACAGA GTTATGCTCT 10620
TGTTGCCCAG GCTGGAGTGC AGCGGCATGA TCTTGGCTCA CTGCAACCTC CGTCTCCTGG 10680
GTTCAAGCAA TTCTCGTGCC TCAGCCTCCC AAGTAGCTGG GATTTCAGGC GTGCACCACC 10740
ACACCCGGCT AATTTTGTAT TTTTAGTAGA GATGGGCTTT CACCATGTTG GTCAGGCTGA 10800
TCTCAAAATC CTGACCTCAG GTGATCCGCC CACCTCAGCC TCCCAAAGTG CTGGGATTAC 10860
AGGCATGAGC CACTGCACCT GGCCTATTTA ACCATTTTAA AACTTCCCTG GGCTCAAGTC 10920
ACACCCACTG GTAAGGAGTT CATGGAGTTC AATTTCCCCT TTACTCAGGA GTTACCCTCC 10980
TTTGATATTT TCTGTAATTC TTCGTAGACT GGGGATACAC CGTCTCTTGA CATATTCACA 11040
GTTTCTGTGA CCACCTGTTA TCCCATGGGA CCCACTGCAG GGGCAGCTGG GAGGCTGCAG 11100
GCTTCAGGTC CCAGTGGGGT TGCCATCTGC CAGTAGAAAC CTGATGTAGA ATCAGGGCGC 11160
GAGTGTGGAC ACTGTCCTGA ATCTCAATGT CTCAGTGTGT GCTGAAACAT GTAGAAATTA 11220
AAGTCCATCC CTCCTACTCT ACTGGGATTG AGCCCCTTCC CTATCCCCCC CCAGGGGCAG 11280
AGGAGTTCCT CTCACTCCTG TGGAGGAAGG AATGATACTT TGTTATTTTT CACTGCTGGT 11340
ACTGAATCCA CTGTTTCATT TGTTGGTTTG TTTGTTTTGT TTTGAGAGGC GGTTTCACTC 11400
TTGTTGCTCA GGCTGGAGGG AGTGCAATGG CGCGATCTTG GCTTACTGCA GCCTCTGCCT 11460
CCCAGGTTCA AGTGATTCTC CTGCTTCCGC CTCCCATTTG GCTGGGATTA CAGGCACCCG 11520
CCACCATGCC CAGCTAATTT TTTGTATTTT TAGTAGAGAC GGGGGTGGGG GTGGGGTTCA 11580
CCATGTTGGC CAGGCTGGTC TCGAACTTCT GACCTCAGAT GATCCACCTG CCTCTGCCTC 11640
CTAAAGTGCT GGGATTACAG GTGTGAGCCA CCATGCCCAG CTCAGAATTT ACTCTGTTTA 11700
GAAACATCTG GGTCTGAGGT AGGAAGCTCA CCCCACTCAA GTGTTGTGGT GTTTTAAGCC 11760
AATGATAGAA TTTTTTTATT GTTGTTAGAA CACTCTTGAT GTTTTACACT GTGATGACTA 11820
AGACATCATC AGCTTTTCAA AGACACACTA ACTGCACCCA TAATACTGGG GTGTCTTCTG 11880
GGTATCAGCG ATCTTCATTG AATGCCGGGA GGCGTTTCCT CGCCATGCAC ATGGTGTTAA 11940
TTACTCCAGC ATAATCTTCT GCTTCCATTT CTTCTCTTCC CTCTTTTAAA ATTGTGTTTT 12000
CTATGTTGGC TTCTCTGCAG AGAACCAGTG TAAGCTACAA CTTAACTTTT GTTGGAACAA 12060
ATTTTCCAAA CCGCCCCTTT GCCCTAGTGG CAGAGACAAT TCACAAACAC AGCCCTTTAA 12120
AAAGGCTTAG GGATCACTAA GGGGATTTCT AGAAGAGCGA CCCGTAATCC TAAGTATTTA 12180
CAAGACGAGG CTAACCTCCA GCGAGCGTGA CAGCCCAGGG AGGGTGCGAG GCCTGTTCAA 12240
ATGCTAGCTC CATAAATAAA GCAATTTCCT CCGGCAGTTT CTGAAAGTAG GAAAGGTTAC 12300
ATTTAAGGTT GCGTTTGTTA GCATTTCAGT GTTTGCCGAC CTCAGCTACA GCATCCCTGC 12360
AAGGCCTCGG GAGACCCAGA AGTTTCTCGC CCCTTAGATC CAAACTTGAG CAACCCGGAG 12420
TCTGGATTCC TGGGAAGTCC TCAGCTGTCC TGCGGTTGTG CCGGGGCCCC AGGTCTGGAG 12480
GGGACCAGTG GCCGTGTGGC TTCTACTGCT GGGCTGGAAG TCGGGCCTCC TAGCTCTGCA 12540
GTCCGAGGCT TGGAGCCAGG TGCCTGGACC CCGAGGCTGC CCTCCACCCT GTGCGGGCGG 12600
GATGTGACCA GATGTTGGCC TCATCTGCCA GACAGAGTGC CGGGGCCCAG GGTCAAGGCC 12660
GTTGTGGCTG GTGTGAGGCG CCCGGTGCGC GGCCAGCAGG AGCGCCTGGC TCCATTTCCC 12720
ACCCTTTCTC GACGGGACCG CCCCGGTGGG TGATTAACAG ATTTGGGGTG GTTTGCTCAT 12780
GGTGGGGACC CCTCGCCGCC TGAGAACCTG CAAAGAGAAA TGACGGGCCT GTGTCAAGGA 12840
GCCCAAGTCG CGGGGAAGTG TTGCAGGGAG GCACTCCGGG AGGTCCCGCG TGCCCGTCCA 12900
GGGAGCAATG CGTCCTCGGG TTCGTCCCCA GCCGCGTCTA CGCGCCTCCG TCCTCCCCTT 12960
CACGTCCGGC ATTCGTGGTG CCCGGAGCCC GACGCCCCGC GTCCGGACCT GGAGGCAGCC 13020
CTGGGTCTCC GGATCAGGCC AGCGGCCAAA GGGTCGCCGC ACGCACCTGT TCCCAGGGCC 13080
TCCACATCAT GGCCCCTCCC TCGGGTTACC CCACAGCCTA GGCCGATTCG ACCTCTCTCC 13140
GCTGGGGCCC TCGCTGGCGT CCCTGCACCC TGGGAGCGCG AGCGGCGCGC GGGCGGGGAA 13200
GCGCGGCCCA GACCCCCGGG TCCGCCCGGA GCAGCTGCGC TGTCGGGGCC AGGCCGGGCT 13260
CCCAGTGGAT TCGCGGGCAC AGACGCCCAG GACCGCGCTT CCCACGTGGC GGAGGGACTG 13320
GGGACCCGGG CACCCGTCCT GCCCCTTCAC CTTCCAGCTC CGCCTCCTCC GCGCGGACCC 13380
CGCCCCGTCC CGACCCCTCC CGGGTCCCCG GCCCAGCCCC CTCCGGGCCC TCCCAGCCCC 13440
TCCCCTTCCT TTCCGCGGCC CCGCCCTCTC CTCGCGGCGC GAGTTTCAGG CAGCGCTGCG 13500

TCCTGCTGCG CACGTGGGAA GCCCTGGCCC CGGCCACCCC CGCGATGCCG CGCGCTCCCC 13560
GCTGCCGAGC CGTGCGCTCC CTGCTGCGCA GCCACTACCG CGAGGTGCTG CCGCTGGCCA 13620
CGTTCGTGCG GCGCCTGGGG CCCCAGGGCT GGCGGCTGGT GCAGCGCGGG GACCCGGCGG 13680
CTTTCCGCGC GCTGGTGGCC CAGTGCCTGG TGTGCGTGCC CTGGGACGCA CGGCCGCCCC 13740
CCGCCGCCCC CTCCTTCCGC CAGGTGGGCC TCCCCGGGGT CGGCGTCCGG CTGGGGTTGA 13800
GGGCGGCCGG GGGGAACCAG CGACATGCGG AGAGCAGCGC AGGCGACTCA GGGCGCTTCC 13860
CCCGCAGGTG TCCTGCCTGA AGGAGCTGGT GGCCCGAGTG CTGCAGAGGC TGTGCGAGCG 13920
CGGCGCGAAG AACGTGCTGG CCTTCGGCTT CGCGCTGCTG GACGGGGCCC GCGGGGGCCC 13980
CCCCGAGGCC TTCACCACCA GCGTGCGCAG CTACCTGCCC AACACGGTGA CCGACGCACT 14040
GCGGGGGAGC GGGGCGTGGG GGCTGCTGCT GCGCCGCGTG GGCGACGACG TGCTGGTTCA 14100
CCTGCTGGCA CGCTGCGCGC TCTTTGTGCT GGTGGCTCCC AGCTGCGCCT ACCAGGTGTG 14160
CGGGCCGCCG CTGTACCAGC TCGGCGCTGC CACTCAGGCC CGGCCCCCGC CACACGCTAG 14220
TGGACCCCGA AGGCGTCTGG GATGCGAACG GGCCTGGAAC CATAGCGTCA GGGAGGCCGG 14280
GGTCCCCCTG GGCCTGCCAG CCCCGGGTGC GAGGAGGCGC GGGGGCAGTG CCAGCCGAAG 14340
TCTGCCGTTG CCCAAGAGGC CCAGGCGTGG CGCTGCCCCT GAGCCGGAGC GGACGCCCGT 14400
TGGGCAGGGG TCCTGGGCCC ACCCGGGCAG GACGCGTGGA CCGAGTGACC GTGGTTTCTG 14460
TGTGGTGTCA CCTGCCAGAC CCGCCGAAGA AGCCACCTCT TTGGAGGGTG CGCTCTCTGG 14520
CACGCGCCAC TCCCACCCAT CCGTGGGCCG CCAGCACCAC GCGGGCCCCC CATCCACATC 14580
GCGGCCACCA CGTCCCTGGG ACACGCCTTG TCCCCCGGTG TACGCCGAGA CCAAGCACTT 14640
CCTCTACTCC TCAGGCGACA AGGAGCAGCT GCGGCCCTCC TTCCTACTCA GCTCTCTGAG 14700
GCCCAGCCTG ACTGGCGCTC GGAGGCTCGT GGAGACCATC TTTCTGGGTT CCAGGCCCTG 14760
GATGCCAGGG ACTCCCCGCA GGTTGCCCCG CCTGCCCCAG CGCTACTGGC AAATGCGGCC 14820
CCTGTTTCTG GAGCTGCTTG GGAACCACGC GCAGTGCCCC TACGGGGTGC TCCTCAAGAC 14880
GCACTGCCCG CTGCGAGCTG CGGTCACCCC AGCAGCCGGT GTCTGTGCCC GGGAGAAGCC 14940
CCAGGGCTCT GTGGCGGCCC CCGAGGAGGA GGACACAGAC CCCCGTCGCC TGGTGCAGCT 15000
GCTCCGCCAG CACAGCAGCC CCTGGCAGGT GTACGGCTTC GTGCGGGCCT GCCTGCGCCG 15060
GCTGGTGCCC CCAGGCCTCT GGGGCTCCAG GCACAACGAA CGCCGCTTCC TCAGGAACAC 15120
CAAGAAGTTC ATCTCCCTGG GGAAGCATGC CAAGCTCTCG CTGCAGGAGC TGACGTGGAA 15180
GATGAGCGTG CGGGACTGCG CTTGGCTGCG CAGGAGCCCA GGTGAGGAGG TGGTGGCCGT 15240
CGAGGGCCCA GGCCCCAGAG CTGAATGCAG TAGGGGCTCA GAAAAGGGGG CAGGCAGAGC 15300
CCTGGTCCTC CTGTCTCCAT CGTCACGTGG GCACACGTGG CTTTTCGCTC AGGACGTCGA 15360
GTGGACACGG TGATCGAGTC GACTCCCTTT AGTGAGGGTT AATTGAGCTC GCGGCCGC 15418

SEQ. ID NO:2 (mTERT sequence, GenBank Accession AF121949)

1 aagcttccag caaaccagtt agagctgagt tgatgctctg aagaagagaa aatgtagaga
61 cggtactgaa caaataatgt ctgggcaaac ctcagacatg aaaatggaag acgtggaaat
121 ccagagaact ctgagggaaa ataaaacaca actccaggtc atcacgggac tcatcaaact
181 gctgaggtgc agccacagag aaaaatctta aaatagccta gaacgatgca tgacacataa
241 agcacagaga agacgaagct gagtctgtct tgtaggaaca acttgagaag acctaaacca
301 ctgcaatgag tgcattctgc taacttagaa tttgctaccc agttcagatc caaaaagggt
361 ttcacaaagt tcaacacaaa acagtagcag gagtggctaa gggggacaca ctgataggaa
421 ttcagagaag tagggaatgc tcatatgggg acattacaaa atgtactttc atgttgctta
481 aatcatttta attgtcaacc acatcaagct aaataatgct ttgaggttca taacatttgg
541 agattatgtc tacactagca gagaaggcac caataacatc ccaattgcta gattctcata
601 gaatcatgag tcacaatggc agagacaggt tctgagagtg tgtccttgtt gtaaacagta
661 tgctctacaa actaagttgg ctgcaatatc actaggcagt gttgtcccat aagacaacta
721 tcacatatgt ggtccagtga tgaccaaagc atcttttagc attttgcaaa tgaagctcaa
781 atcgaatatg actaagctca tgcagtacaa atcaaaggta cactgggata gtttaaaaga
841 tacatacttg tactggttag ttttgtgtca gcttgacaca gctggagtta tcacagagaa
901 aagagcttca gttgaggaaa ttcctccatg agatccagct atagggcatt ttctcaatta
961 gtgatcaagg ggggaaggcc ccttgtgggt gggaccatct ctgggctggt agtcttggtt
1021 ctataagaga gcaggctgag caagccagga gaagcaagcc agtaaagaac atccctccat
1081 ggcttctgca tcagctcctg ctccctgacc tgcttgagtt ccagttctaa cttctttcag
1141 tgatgaacag caatgtggaa atgaaagctg aataaaccct ttcctcccca ttttgcttct
1201 tggtcatgat gtttgtgcag gaatagaaac cctgactaag acaatactat aaaccctaaa
1261 agttgtaaac caaacacatg tgtttccatt aagccatcgt agaacaataa gtactcaacc
1321 ccaagtcaca taactataat cccagccttt gaaaaccggg atcaggaatt caaggctagc
1381 ctcatctata tgtaagatta aagcctgttt gggctgcatg agactttgtt tcaaaaaaaa
1441 aaaaaaaaaa gcaaacaggc aaaaacaaac acaagacaag acagatgtaa aatgaaggag
1501 gggtagatgg gtcaagtaga aaatagcata ggaaacgagt caagtataga agaggtggta
1561 gtaaccagat catgcagaag gactcaaggc catctcctca cagtggctta ggtaggcctt
1621 cctctgctct tgagcagggg cagagttgcc gctttaagga ggggatcagt cacctttaag
1681 aactgaaaag ctgaacagtc ttctcaagtc agaagccagt ggcttcatct tacacctctc
1741 ttccttccct tgctactcat attggatctg atgatttgcc caacttggaa gaaacatctc
1801 ttctgaaggg tttcacagac accccatctt tccgagaaag gaccgcatag gctggccatc
1861 cctgtgctta caaaaggaat aattaagaaa cttaattcca taagcaaata caacctttcc
1921 aagccccaag tggatgattt tatcttactg tttttttata tctcatcaaa taacttccaa
1981 gggctcaaaa atccaaagat gtaaaaaagg aactgagctc tgtttgccaa gccatgagga
2041 ttaaataatg acattcaaag agatttttgt gccctaagta ctttttattg gttttcatag
2101 atggtttaat gtgcaagatg aagcaaacag agatgggagt ggtatcagca tggattaagg
2161 tggcagttgt gagggagggg tactgagaga acaggacaag gtaacctatc taaggagagg
2221 ccaagttggc aagtgccagg gacttctaag cccagaacta gtacacattc cttaggtgct
2281 gtttgggaag tcagggagtc accagccttg ggatctataa aagtgcatgg tggcattcac
2341 tcacatactt cctgagctgt tcgatgttga tgaagtcgtg ggtatgagac tgttgtgtca
2401 gtgacaaact atgtaaatga gaatgattgt ttccatcttg accactaaga cgtaaaccgg
2461 ttccagtgat ctccaaacat ggcaagctac agcagagcag cagccccatc cagagccttg

2521 ccctggttct gaatggggga gaatccagtg ggagtcggtt gctgccagca tgttggggta
2581 gaaggctgga gcatgacagg tccccgagga tttcctgctt cctatatggg tagggatact
2641 tgaggtcctc tcttctacct ccttccctgc agggtttata acctctacca ctgtctgtct
2701 ctgggatagc tcctagggtg cagcccctcc ccaaaaaggc ctctccctgg cctcatgtct
2761 ctaagaacag ctttctaaag caggcctgtt acacaaaggc tcccttttcc tggcttcatc
2821 gttgctggta gacaacttcc actcgttttc cacttcagtt tcttctactc tgttgttatt
2881 tgattctgat gcttgaaccc agggttgtgt agtcagcaag tgctaccccc tccctcctct
2941 tctttgtttt tttgaggcag ggtctcattt tgcccaagtg gacctaaatt tcagcatgta
3001 gctggcctgg ttttgaatgc cttctcatcc tgcctctact tcccaagagt agcttacaag
3061 tgtgcaccac catgccccgc gatattctta tttttgagac tgttttctat gctggtttct
3121 ttggggaact acactaaggt agcttacaag tgtgcaccac catgccccgc gatattctta
3181 tttttgagac tgttttctat gctggtttct ttggggaact acactaaggt agcttcattg
3241 ttggcataaa tttctcagtt caggcccata tctcctaagt agcagaacta agcaaatctc
3301 aaacaaaccc ctcaaaaaga ctgatgtcca ctaaacggac ttctaaaata gctcctgtaa
3361 tcctgagcat ttacaaggcg gcagacctcc tataagggag taaatatgaa aacgcgcctg
3421 ttcaaatgct aggtcggtgg atagaagcaa tttcctcaga aagctgaagg caccaaaggt
3481 tatatttgtt agcatttcag tgtttgccaa actcagctac agtagagatc acagattccc
3541 tatttcccag agattcaaaa ttcagcagcc cctctctaac tatggctcag agtcgtgtca
3601 ttacatatgc cccaacaaca acccccaccc ctatcctacc cccgcctcac acgtgcaagt
3661 actatcacag ttgccaacct agcagagctg ccatcctaag gtcgaggtcg ccgctttggc
3721 tgtgtgcaca ggcaagcgcc ctcacccaat ggccctggcc ttgctatggg tgcgtgagtt
3781 gagatgatgc tctggactct gaggtgaagg ccactggaac agtgaaaaaa gctaacgcag
3841 ggcttttacc tagtcccctt cctttggtgg tgggtgttta cggaacatat ttgggatctg
3901 agtgtatggt cgcaccacaa taaagcctta acctatatag tagaatttca gctgtaatca
3961 ttaagaactg agattgccac cacccacctc actgtctgtg tcaaccacag caggctggag
4021 cagtcagctc aggaacaggc aaaaccttag gtccctccgc ctacctaacc ttcaatacat
4081 caaggatagg cttctttgct tgcccaaacc tcgccccagt ctagaccacc tggggattcc
4141 cagctcaggg cgaaaaggaa gcccgagaag cattctgtag agggaaatcc tgcatgagtg
4201 cgcccccttt cgttactcca acacatccag caaccactga acttggccgg ggaacacacc
4261 tggtcctcat gcaccagcat tgtgaccatc aacggaaaag tactattgct gcgaccccgc
4321 cccttccgct acaacgcttg gtccgcctga atcccgcccc ttcctccgtt cccagcctca
4381 tctttttcgt cgtggactct cagtggcctg ggtcctggct gttttctaag cacacccttg
4441 catcttggtt cccgcacgtg ggaggcccat cccggccttg agcacaatga cccgcgctcc
4501 tcgttgcccc gcggtgcgct ctctgctgcg cagccgatac cgggaggtgt ggccgctggc
4561 aacctttgtg cggcgcctgg ggcccgaggg caggcggctt gtgcaacccg ggacccgaa
4621 gatctaccgc actttggttg cccaatgcct agtgtgcatg cactggggct cacagcctcc
4681 acctgccgac ctttccttcc accaggtggg cctccaggcg ggatccccat gggtcagggg
4741 cggaaagccg ggaggacgtg ggatagtgcg tctagctcat gtgtcaagac cctcttctcc
4801 ttaccaggtg tcatccctga aagagctggt ggccagggtt gtgcagagac tctgcgagcg
4861 caacgagaga aacgtgctgg cttttggctt tgagctgctt aacgaggcca gaggcgggcc
4921 tcccatggcc ttcactagta gcgtgcgtag ctacttgccc aacactgtta ttgagaccct
4981 gcgtgtcagt ggtgcatgga tgctactgtt gagccgagtg ggcgacgacc tgctggtcta
5041 cctgctggca cactgtgctc tttatcttct ggtgcccccc agctgtgcct accaggtgtg
5101 tgggtctccc ctgtaccaaa tttgtgccac cacggatatc tggccctctg tgtccgctag
5161 ttacaggccc acccgacccg tgggcaggaa tttcactaac cttaggttct tacaacagat
5221 caagagcagt agtcgccagg aagcaccgaa acccctggcc ttgccatctc gaggtacaaa
5281 gaggcatctg agtctcacca gtacaagtgt gccttcagct aagaaggcca gatgctatcc
5341 tgtcccgaga gtggaggagg gaccccacag gcaggtgcta ccaaccccat caggcaaatc
5401 atgggtgcca agtcctgctc ggtcccccga ggtgcctact gcagagaaag atttgtcttc
5461 taaaggaaag gtgtctgacc tgagtctctc tgggtcggtg tgctgtaaac acaagcccag
5521 ctccacatct ctgctgtcac caccccgcca aaatgccttt cagctcaggc catttattga
5581 gaccagacat ttcctttact ccaggggaga tggccaagag cgtctaaacc cctcattcct
5641 actcagcaac ctccagccta acttgactgg ggccaggaga ctggtggaga tcatctttct
5701 gggctcaagg cctaggacat caggaccact ctgcaggaca caccgtctat cgcgtcgata
5761 ctggcagatg cggcccctgt tccaacagct gctggtgaac catgcagagt gccaatatgt
5821 cagactcctc aggtcacatt gcaggtttcg aacagcaaac caacaggtga cagatgcctt
5881 gaacaccagc ccaccgcacc tcatggattt gctccgcctg cacagcagtc cctggcaggt
5941 atatggtttt cttcgggcct gtctctgcaa ggtggtgtct gctagtctct ggggtaccag
6001 gcacaatgag cgccgcttct ttaagaactt aaagaagttc atctcgttgg ggaaatacgg
6061 caagctatca ctgcaggaac tgatgtggaa gatgaaagta gaggattgcc actggctccg
6121 cagcagcccg ggtgagcatg gctggtctcc agctgaatgc attaggggcc cagaaaaggg
6181 agacaatggg tggcagtaac ccaggtcccc agtggtgtgg tggctttatg cagtccgtgg
6241 ttggatgagt tccatcttat ggtctctgac tccaagctcc ctccagctcg ccttgcacaa
6301 actaagattc ttgtccaagc cctgggcagg ttctcagggc tggggacatt gtggtgaaca
6361 gataagcaga cggggagcat ggtggatagg agttctggca cagtgcacca gagagagtct
6421 ggaagcgcta gtgagagcta atgtaagggc ccgtggttcg ccaaagaatg ataaccccgg
6481 actcaaatag tatgccaaag caaggagcat ttcattctgc agaaatcaag catgcaggtg
6541 gggggggggg gttgctctca ttccaagatg gagagacaac caagtataga ttttaagggg
6601 atcgggggcc tttatcttac tccatctcta ggggcattcc attactgggg catggggttg
6661 gaggttggaa actgttaatg gggaggtctg gaaacttgct gccccattgt ccttgcttca
6721 ggctaggtag ctgagtagct tctaatggca ggatagtttc tgactagctg tctaaagtct
6781 ggggtgtttg tttttttgtt ttttctagta acttacttgc ctgaacttgc tcagttttta
6841 ggcctggtct cctggactgc caatttgaag cctattaagg agtcagcctg tctcactact
6901 ccaggttatc tataatcccc ctgtagaacg gtacctcact gataacaatg acagaccaac
6961 ataggaaccc actatccttg tggtgcatga gtttcaaagg ttcttctggt cctcccagtg
7021 tgcagatcca tgcttaagct atggtcctcc cagtgtgcag atccgtgctt aagctatggt
7081 cttgcagctg ctcgatctac aaagggtagg gtgaacgaag gaaagataaa tgaaaaaaaa

7141 aaaactgttt cctacagtga agatcgctgc cccatcttag ctatgagaag ggactgggga
7201 gtggagcctg gtgcataaaa gaggattgtg ttacttggaa ggctgcagag cctggactcc
7261 tgtgccctcc ttgcctggtt ttctgggttt aatgttgagg ttggccctct gtagtcacta
7321 cctgacccct tccctttcag ccaaccctcc ggttacaccc tgtgcatgta tggaaggggc
7381 caaacgccct atcctgctct cccttcccca aaattcttag gatattaaca acttatgggg
7441 aaaagatggt agagctatgt ttacccacca tgtacttggg aagctccgaa gtaagctt

## Claims

1. An oncolytic virus having a genome in which a telomerase reverse transcriptase (TERT) promoter is operably linked to a genetic element essential for replication or assembly of the virus, wherein the promoter promotes transcription of the genetic element in cells expressing telomerase reverse transcriptase (TERT), thereby promoting replication of the virus, and wherein replication of the virus in a cell leads to lysis of the cell.

2. The oncolytic virus of claim 1, which is a replication-conditional adenovirus.

3. The oncolytic virus of claim 1, which is a replication-conditional herpesvirus.

4. A polynucleotide for assembling the oncolytic virus of claim 1, in which a telomerase reverse transcriptase (TERT) promoter is operatively linked to a genetic element essential for replication or assembly of a virus.

5. The oncolytic virus or polynucleotide of any preceding claim, wherein the genetic element essential for replication or assembly of a virus is an adenovirus E4, E1a, E1b or E2 gene or a herpes simplex virus ICP6 or ICP4 gene.

6. The oncolytic adenovirus of claim 2, wherein the genetic element essential for replication or assembly is an adenovirus E1a region.

7. The oncolytic virus or polynucleotide of any preceding claim, wherein the promoter polynucleotide is a promoter for human telomerase reverse transcriptase (hTERT).

8. The oncolytic virus or polynucleotide of any preceding claim, wherein the nucleotide sequence of the promoter is contained in phage λGϕ5.

9. The oncolytic virus or polynucleotide of any preceding claim, wherein the promoter has one or more of the following features:
a) it comprises a sequence of at least about 100 consecutive nucleotides in SEQ. ID NO:1;
b) it comprises a sequence of at least about 500 consecutive nucleotides in SEQ. ID NO:1;
c) it comprises a sequence of at least about 100 consecutive nucleotides in SEQ. ID NO:2;
d) it comprises a sequence of at least about 500 consecutive nucleotides in SEQ. ID NO:2; or
e) it hybridizes with a polynucleotide complementary to a sequence having feature a), b), c) or d) under stringent conditions, and has the characteristic of preferentially promoting transcription in cells expressing TERT.

10. The oncolytic virus or polynucleotide of any preceding claim, wherein the promoter polynucleotide has one or more of the following features:
a) it comprises the sequence from position -117 to position -36 relative to the translation initiation site (position 13545) of SEQ. ID NO:1;
b) it comprises the sequence from position -239 to position -36 relative to the translation initiation site (position 13545) of SEQ. ID NO:1;
c) it comprises the sequence from position -117 to position +1 relative to the translation initiation site (position 13545) of SEQ. ID NO:1;
d) it comprises the sequence from position -239 to position +1 relative to the translation initiation site (position 13545) of SEQ. ID NO:1;
e) it consists of no more than 82 consecutive nucleotides; or
f) it hybridizes with a polynucleotide complementary to a sequence having feature a), b), c), or d) under stringent conditions, and has the characteristic of preferentially promoting transcription in cells expressing TERT.

11. The oncolytic virus or polynucleotide of any preceding claim, further comprising an encoding region whose expression is toxic to the cell, or which renders the cell more susceptible to toxic effects of a drug.

12. The oncolytic virus or polynucleotide of the preceding claim, wherein the encoding region encodes thymidine kinase, and the drug is ganciclovir.

13. A method for selecting a virus having characteristics of an oncolytic virus according to any of claims 1-12, comprising providing a oncolytic virus in which said TERT promoter is operably linked to a genetic element required for replication or assembly of the virus, using the virus to infect a cell expressing TERT and a cell not expressing TERT, and selecting the virus if it kills the cell expressing TERT.

14. A method for producing a oncolytic virus according to claims 1-12, comprising operably linking said TERT promoter sequence to said genetic element to form a linked gene; transfecting the linked gene into a telomerase expressing cell; and then propagating virus obtained from the cell.

15. A in vitro method for killing a cell expressing telomerase reverse transcriptase, comprising contacting the cell in vitro with the oncolytic virus according to any of claims 1-12.

16. The method of claim 15, wherein the cell is a cancer cell.

17. The method of claim 16, wherein the cancer is selected from the group consisting of lung cancer, pancreatic cancer, medulloblastoma, cervical carcinoma, and fibrosarcoma.

18. A medicament comprising the oncolytic virus according to any of claims 1-12 for use in medicine.

19. Use of the oncolytic virus according to any of claims 1-10 in the preparation of a medicament for treatment of cancer.

20. The use according to claim 19, wherein the cancer is selected from the group consisting of lung cancer, pancreatic cancer, medulloblastoma, cervical carcinoma, and fibrosarcoma.

## Patentansprüche

1. Onkolytisches Virus mit einem Genom, in dem ein Promotor der Telomerase-Reverse-Transkriptase (TERT) wirkungsmäßig mit einem genetischen Element verbunden ist, das für die Replikation oder das Zusammensetzen des Virus erforderlich ist, wobei der Promotor die Transkription des genetischen Elementes in die Telomerase-Reverse-Transkriptase (TERT) exprimierenden Zellen fördert, damit die Replikation des Virus fördert, und wobei die Replikation des Virus in einer Zelle zur Auflösung der Zelle führt.

2. Onkolytisches Virus nach Anspruch 1, das ein replikationsbedingtes Adenovirus ist.

3. Onkolytisches Virus nach Anspruch 1, das ein replikationsbedingtes Herpesvirus ist.

4. Polynukleotid zum Zusammensetzen des onkolytischen Virus nach Anspruch 1, wobei ein Promotor der Telomerase-Reverse-Transkriptase (TERT) wirkungsmäßig mit einem genetischen Element verbunden ist, das für die Replikation oder das Zusammensetzen eines Virus erforderlich ist.

5. Onkolytisches Virus oder Polynukleotid nach einem der vorhergehenden Ansprüche, wobei das genetische Element, das für die Replikation oder das Zusammensetzen eines Virus erforderlich ist, ein Adenovirus-E4-, E1a-, E1b- oder E2-Gen oder ein Herpes-Simplex-Virus-ICP6- oder ICP4-Gen ist.

6. Onkolytisches Adenovirus nach Anspruch 2, wobei das genetische Element, das für die Replikation oder das Zusammensetzen erforderlich ist, eine Adenovirus-E1a-Region ist.

7. Onkolytisches Virus oder Polynukleotid nach einem der vorhergehenden Ansprüche, wobei das Promotor-Polynukleotid ein Promotor für humane Telomerase-Reverse-Transkriptase (hTERT) ist.

8. Onkolytisches Virus oder Polynukleotid nach einem der vorhergehenden Ansprüche, wobei die Nukleotidsequenz des Promotors in Phage λGϕ5 enthalten ist.

9. Onkolytisches Virus oder Polynukleotid nach einem der vorhergehenden Ansprüche, wobei der Promotor eines oder mehrere der folgenden Merkmale aufweist:
a) er enthält eine Sequenz von mindestens ungefähr 100 aufeinanderfolgenden Nukleotiden in SEQ ID NO: 1;
b) er enthält eine Sequenz von mindestens ungefähr 500 aufeinanderfolgenden Nukleotiden in SEQ ID NO: 1;
c) er enthält eine Sequenz von mindestens ungefähr 100 aufeinanderfolgenden Nukleotiden in SEQ ID NO:2;
d) er enthält eine Sequenz von mindestens ungefähr 500 aufeinanderfolgenden Nukleotiden in SEQ ID NO:2, oder
e) er hybridisiert unter stringenten Bedingungen mit einem Polynukleotid, das zu einer Sequenz mit Merkmal a), b), c) oder d) komplementär ist, und weist die Eigenschaft auf, vorzugsweise Transkription in Zellen zu fördern, welche TERT exprimieren.

10. Onkolytisches Virus oder Polynukleotid nach einem der vorhergehenden Ansprüche, wobei das Promotor-Polynukleotid eines oder mehrere der folgenden Merkmale aufweist:
a) es enthält die Sequenz von Position -117 bis zu Position -36 relativ zur Translationsstartstelle (Position 13545) von SEQ ID NO:1;
b) es enthält die Sequenz von Position -239 bis zu Position -36 relativ zur Translationsstartstelle (Position 13545) von SEQ ID NO:1;
c) es enthält die Sequenz von Position -117 bis zu Position +1 relativ zur Translationsstartstelle (Position 13545) von SEQ ID NO:1;
d) es enthält die Sequenz von Position -239 bis zu Position +1 relativ zur Translationsstartstelle (Position 13545) von SEQ ID NO:1;
e) es besteht aus nicht mehr als 82 aufeinanderfolgenden Nukleotiden, oder
f) es hybridisiert unter stringenten Bedingungen mit einem Polynukleotid, das zu einer Sequenz mit Merkmal a), b), c) oder d) komplementär ist, und weist die Eigenschaft auf, vorzugsweise Transkription in Zellen zu fördern, welche TERT exprimieren.

11. Onkolytisches Virus oder Polynukleotid nach einem der vorhergehenden Ansprüche, das ferner eine Codierregion aufweist, deren Expression für die Zelle toxisch ist, oder welche die Zelle für toxische Wirkungen eines Medikamentes empfänglicher macht.

12. Onkolytisches Virus oder Polynukleotid nach dem vorhergehenden Anspruch, wobei die Codierregion Thymidinkinase codiert und das Medikament Ganciclovir ist.

13. Verfahren zum Auswählen eines Virus mit Eigenschaften eines onkolytischen Virus nach einem der Ansprüche 1 bis 12, welches das Bereitstellen eines onkolytischen Virus, in dem der TERT-Promotor wirkungsmäßig mit einem genetischen Element verbunden ist, das für die Replikation oder das Zusammensetzen des Virus erforderlich ist, das Verwenden des Virus zum Infizieren einer TERT-exprimierenden Zelle und einer Zelle, die nicht TERT exprimiert, und Auswählen des Virus aufweist, falls es die Zelle tötet, welche TERT exprimiert.

14. Verfahren zur Herstellung eines onkolytischen Virus nach Anspruch 1 bis 12, welches wirkungsmäßiges Verbinden der TERT-Promotorsequenz mit dem genetischen Element zum Bilden eines verbundenen Gens, Transfektieren des verbundenen Gens in eine Telomerase-exprimierende Zelle und anschließendes Propagieren des Virus, das aus der Zelle erhalten wurde, aufweist.

15. In vitro - Verfahren zum Töten einer Telomerase-Reverse-Transkriptase exprimierenden Zelle, wobei das Verfahren in vitro Kontaktieren der Zelle mit dem onkolytischen Virus nach einem der Ansprüche 1 bis 12 aufweist.

16. Verfahren nach Anspruch 15, wobei die Zelle eine Krebszelle ist.

17. Verfahren nach Anspruch 16, wobei der Krebs aus der Gruppe ausgewählt ist, die aus Lungenkrebs, Bauchspeicheldrüsenkrebs, Medulloblastoma, Gebärmutterhalskrebs und Fibrosarcoma besteht.

18. Medikament, welches das onkolytische Virus nach einem der Ansprüche 1 bis 12 zur medizinischen Verwendung enthält.

19. Verwendung des onkolytischen Virus nach einem der Ansprüche 1 bis 10 bei der Zubereitung eines Medikaments zur Krebsbehandlung.

20. Verwendung nach Anspruch 19, wobei der Krebs aus der Gruppe ausgewählt ist, die aus Lungenkrebs, Bauchspeicheldrüsenkrebs, Medulloblastoma, Gebärmutterhalskrebs und Fibrosarcoma besteht.

## Revendications

1. Virus oncolytique présentant un génome dans lequel un promoteur de télomérase transcriptase inverse (TERT) est lié de manière fonctionnelle à un élément génétique essentiel pour la réplication ou l'assemblage du virus, dans lequel le promoteur promeut la transcription de l'élément génétique dans des cellules exprimant la télomérase transcriptase inverse (TERT), en promouvant ainsi la réplication du virus, et dans lequel la réplication du virus dans une cellule mène à la lyse de la cellule.

2. Virus oncolytique suivant la revendication 1, qui est un adénovirus à réplication conditionnelle.

3. Virus oncolytique suivant la revendication 1, qui est un virus de l'herpès à réplication conditionnelle.

4. Polynucléotide pour l'assemblage du virus oncolytique suivant la revendication 1, dans lequel un promoteur de télomérase transcriptase inverse (TERT) est lié de manière fonctionnelle à un élément génétique essentiel pour la réplication ou l'assemblage d'un virus.

5. Virus oncolytique ou polynucléotide suivant l'une quelconque des revendications précédentes, dans lequel l'élément génétique essentiel pour la réplication ou l'assemblage d'un virus est le gène E4, E1a, E1b ou E2 de l'adénovirus ou le gène ICP6 ou ICP4 du virus de l'herpès simplex.

6. Adénovirus oncolytique suivant la revendication 2, dans lequel l'élément génétique essentiel pour la réplication ou l'assemblage est la région E1a de l'adénovirus.

7. Virus oncolytique ou polynucléotide suivant l'une quelconque des revendications précédentes, dans lequel le promoteur du polynucléotide est un promoteur de la télomérase transcriptase inverse humaine (hTERT).

8. Virus oncolytique ou polynucléotide suivant l'une quelconque des revendications précédentes, dans lequel la séquence nucléotidique du promoteur est contenue dans le phage λGϕ5.

9. Virus oncolytique ou polynucléotide suivant l'une quelconque des revendications précédentes, dans lequel le promoteur présente une ou plusieurs des particularités suivantes :
a) il comprend une séquence d'au moins environ 100 nucléotides consécutifs de la séquence I.D. NO 1;
b) il comprend une séquence d'au moins environ 500 nucléotides consécutifs de la séquence I.D. NO 1;
c) il comprend une séquence d'au moins environ 100 nucléotides consécutifs de la séquence I.D. NO 2;
d) il comprend une séquence d'au moins environ 500 nucléotides consécutifs de la séquence I.D. NO 2; ou
e) il réalise une hybridation avec un polynucléotide complémentaire d'une séquence présentant la particularité a), b), c) ou d) dans des conditions stringentes, et il présente la caractéristique de promouvoir de manière préférentielle une transcription dans des cellules exprimant TERT.

10. Virus oncolytique ou polynucléotide suivant l'une quelconque des revendications précédentes,
dans lequel le polynucléotide promoteur présente une ou plusieurs des particularités suivantes :
a) il comprend la séquence de la position -117 à la position -36 par rapport au site d'initiation de la traduction (position 13 545) de la séquence I.D. NO 1;
b) il comprend la séquence de la position -239 à la position -36 par rapport au site d'initiation de la traduction (position 13 545) de la séquence I.D. NO 1;
c) il comprend la séquence de la position -117 à la position +1 par rapport au site d'initiation de la traduction (position 13 545) de la séquence I.D. NO 1;
d) il comprend la séquence de la position -239 à la position +1 par rapport au site d'initiation de la traduction (position 13 545) de la séquence I.D. NO 1;
e) il ne comprend pas plus de 82 nucléotides consécutifs; ou
f) il réalise une hybridation avec un polynucléotide complémentaire d'une séquence présentant la particularité a), b), c), ou d) dans des conditions stringentes, et il présente la caractéristique de promouvoir de manière préférentielle une transcription dans des cellules exprimant TERT.

11. Virus oncolytique ou polynucléotide suivant l'une quelconque des revendications précédentes, comprenant en outre une région codante dont l'expression est toxique pour la cellule ou qui rend la cellule plus sensible aux effets toxiques d'un médicament.

12. Virus oncolytique ou polynucléotide suivant l'une quelconque des revendications précédentes, dans lequel la région codante code pour la thymidine kinase et le médicament est le ganciclovir.

13. Procédé de sélection d'un virus présentant les caractéristiques d'un virus oncolytique suivant l'une quelconque des revendications 1-12, comprenant le fait de procurer un virus oncolytique dans lequel ledit promoteur TERT est lié de manière fonctionnelle à un élément génétique requis pour la réplication ou l'assemblage du virus, utilisant le virus pour infecter une cellule exprimant TERT et une cellule n'exprimant pas TERT, et sélectionnant le virus s'il tue la cellule exprimant TERT.

14. Procédé de production d'un virus oncolytique suivant les revendications 1-12, comprenant la liaison de manière fonctionnelle de ladite séquence de promoteur TERT audit élément génétique pour former un gène lié; la transfection du gène lié dans une cellule exprimant la télomérase; puis la propagation du virus obtenu à partir de la cellule.

15. Procédé in vitro pour tuer une cellule exprimant la télomérase transcriptase inverse, comprenant la mise en contact de la cellule in vitro avec le virus oncolytique suivant l'une quelconque des revendications 1-12.

16. Procédé suivant la revendication 15, dans lequel la cellule est une cellule cancéreuse.

17. Procédé suivant la revendication 16, dans lequel le cancer est sélectionné parmi le groupe comprenant le cancer du poumon, le cancer du pancréas, le médulloblastome, le carcinome du col de l'utérus et le fibrosarcome.

18. Médicament comprenant le virus oncolytique suivant l'une quelconque des revendications 1-12 pour une utilisation en médecine.

19. Utilisation du virus oncolytique suivant l'une quelconque des revendications 1-10, dans la préparation d'un médicament pour le traitement du cancer.

20. Utilisation suivant la revendication 19, dans lequel le cancer est sélectionné parmi le groupe comprenant le cancer du poumon, le cancer du pancréas, le médulloblastome, le carcinome du col de l'utérus et le fibrosarcome.
